# EUROPEAN PATENT SPECIFICATION

(11) **EP 1 871 296 B1**
(45) Date of publication and mention of the grant of the patent: **20.07.2011**
(21) Application number: 06750825.9
(22) Date of filing: 20.04.2006
(51) Int. Cl.: A61F 2/06

(54) **INTERNAL CANNULATED JOINT FOR MEDICAL DELIVERY SYSTEMS**
INTERNES KANÜLIERTES VERBINDUNGSSTÜCK FÜR MEDIZINISCHE AUSTRAGEGABESYSTEME
JOINT A CANULE INTERNE POUR SYSTEMES DE MISE EN PLACE DE DISPOSITIFS MEDICAUX

(30) Priority: 20.04.2005 US 673199 P; 23.01.2006 US 761565 P
(43) Date of publication of application: 02.01.2008
(73) Proprietor: Cook Incorporated, Bloomington, IN 47402 (US)
(72) Inventor: PAL, Dharmendra, Wilmington, MA 01887 (US)
(74) Representative: Mathys & Squire LLP
(86) International application number: PCT/US2006/014875
(87) International publication number: WO 2006/113869

(56) References cited:
- EP-A- 1 095 634
- US-A- 6 063 318
- US-A1- 2006 282 041

## Description

### Technical Field

The present invention relates to an internal cannulated joint for medical devices generally used percutaneously or through a delivery apparatus (such as an endoscope or endoscope accessory channel device) for delivering devices inside a patient's body.

### Background of the Invention

This invention relates an internal cannulated joint for medical device delivery systems that employ a catheter. These medical device delivery systems have a host of uses, including, for example, the deployment of a self-expanding implantable prosthesis at selected locations inside a patient's body. The invention may also be used, however, with a balloon expandable and non-expanding implantable prosthesis. In addition to being used with a rapid insertion delivery system, the invention may be used in an "over-the-wire" delivery system, so both systems will be described below.

By way of background, stents are configured to be implanted into body vessels having a passageway in order to reinforce, support, repair, or otherwise enhance the performance of the passageway. The term "passageway" is understood to be any lumen, channel, flow passage, duct, chamber, opening, bore, orifice, or cavity for the conveyance, regulation, flow, or movement of bodily fluids and/or gases of an animal. As an example, stents have been used in the passageways of an aorta, artery, bile duct, blood vessel, bronchiole, capillary, esophagus, fallopian tube, heart, intestine, trachea, ureter, urethra, vein, and other locations in a body (collectively, "vessel") to name a few.

One type of stent is self-expanding. For a self-expanding stent, the stent is resiliently compressed into a collapsed first, smaller diameter, carried by the delivery system, and due to its construction and material properties, the stent expands to its second, larger diameter upon deployment. In its expanded configuration, the stent exhibits sufficient stiffness so that it will remain substantially expanded and exert a radially outward force in the vessel passageway on an interior surface of the vessel. One particularly useful self-expanding stent is the Z-stent, introduced by Cook Incorporated, due to its ease of manufacturing, high radial force, and self-expanding properties. Examples of the Z-stent are found in United States Patent Nos. 4,580,568; 5,035,706; 5,282,824; 5,507,771; and 5,720,776, the disclosures of which are incorporated in their entireties. The Zilver stent, introduced by Cook Incorporated, is another particularly useful self- expanding stent due to its nitinol platform and use of the Z-stent design properties. Examples of the Zilver stent are found in United States Patent Nos. 6,743,252 and 6,299,635.

Many delivery systems employ a tubular catheter, sheath, or other introducer (individually and collectively, "catheter") having first and second ends and comprising a lumen for receiving the wire guide. Optionally, these delivery systems may fit through a working channel within an endoscope or an external accessory channel device used with an endoscope.

Generally stated, these delivery systems may fall within two categories. The first category of delivery systems to have been used, and consequently the first to be discussed below, is commonly referred to as an "over-the-wire" catheter system. The other category of delivery systems is sometimes referred to as a "rapid exchange" catheter system. In either system, a wire guide is used to position the delivery system within a vessel passageway. The typical wire guide has proximal and distal ends. A physician inserts the distal end into the vessel passageway, advances, and maneuvers the wire guide until the distal end reaches its desired position within the vessel passageway.

In the "over-the-wire" catheter delivery system, a physician places the catheter over the wire guide, with the wire guide being received into a lumen that extends substantially through the entire length of the catheter. In this over-the-wire type of delivery system, the wire guide may be back-loaded or front-loaded into the catheter. In front-loading an over- the-wire catheter delivery system, the physician inserts the distal end of the wire guide into the catheter's lumen at or near the catheter's proximal end. In back-loading an over-the-wire catheter delivery system, the physician inserts a distal portion of the catheter over the proximal end of the wire guide. The back-loading technique is more common when the physician has already placed the wire guide into the patient, which is typically the case today. In either case of back-loading or front-loading an over-the-wire catheter delivery system, the proximal and distal portions of the catheter will generally envelop the length of the wire guide that lies between the catheter first and second ends. While the wire guide is held stationary, the physician may maneuver the catheter through the vessel passageway to a target site at which the physician is performing or intends to perform a treatment, diagnostic, or other medical procedure.

Unlike the over-the-wire system where the wire guide lies within the catheter lumen and extends substantially the entire length of the catheter, in a novel "rapid insertion" catheter delivery system described in US 2006-0259117, the wire guide occupies a catheter lumen extending only through a distal segment of the catheter. The so- called rapid insertion system comprises a system proximal end, an elongate flexible middle section and a system distal end that is generally tubular.

The system distal end, in general, comprises an inner guide channel member sized to fit within an outer guide channel member that is substantially axially slideable relative to the inner guide channel member.
The outer guide channel member and inner guide channel member further have entry and exit ports defining channels configured to receive a wire guide. A port includes any structure that functions as an entry or exit aperture, cutout, gap, hole, opening, orifice, passage, passageway, port, or portal, while a guide channel is understood to be any aperture, bore, cavity, chamber, channel, duct, flow passage, lumen, opening, orifice, or passageway that facilitates the conveyance, evacuation, flow, movement, passage, regulation, or ventilation of fluids, gases, or a diagnostic, monitoring, scope, other instrument, or more particularly a catheter or wire guide.

A wire guide may extend from the outer and inner member entry ports, through the outer and inner member guide channels, and exit the distal end at or near a breech position opening located at or near a transition region where the guide channels and exit ports are approximately aligned relatively coaxially to facilitate a smooth transition of the wire guide. Furthermore, the outer guide channel member has a slightly stepped profile, whereby the outer guide channel member comprises a first outer diameter and a second smaller outer diameter proximal to the first outer diameter and located at or near the transition region.

The system distal end also has a self-expanding deployment device mounting region (e.g., a stent mounting region) positioned intermediate the inner guide channel member entry and exit ports for releasably securing a stent. At the stent mounting region, a stent is releasably positioned axially intermediate distal and proximal restraint markers and sandwiched transversely (i.e., compressed) between the outside surface of the inner guide channel member and the inside surface of an outer guide channel member.

Turning to the system proximal end of the rapid insertion delivery system, the proximal end, in general, comprises a handle portion. The handle portion has a handle that the physician grips and a pusher stylet that passes through the handle. The pusher stylet is in communication with-directly or indirectly through intervening parts-the inner guide channel member at the distal end. Meanwhile, the handle is in communication with-directly or indirectly through intervening parts-the outer guide channel member at the distal end. Holding the pusher stylet relatively stationary (while, for example, actuating the handle) keeps the stent mounting region of the inner guide channel member properly positioned at the desired deployment site. At the same time, proximally retracting the handle results in a corresponding proximal movement of the outer guide channel member relative to the inner guide channel member to thereby expose and, ultimately, deploy the self-expanding stent from the stent mounting region. At times, a physician may need to deploy a second self-expanding stent by withdrawing the system from the proximal end of the wire guide. The physician may then reload the catheter with additional stents, and if that is not an option the physician may load another stent delivery system with an additional stent, onto the wire guide. Also, the physician may withdraw the stent delivery system altogether and replace the delivery system with a catheter or different medical device intended to be loaded onto the wire guide.

The delivery system in the rapid insertion delivery system further comprises an elongate flexible middle section delivery device extending intermediate the system proximal end and the system distal end. The middle section delivery device comprises an outer sheath and an inner compression member having first and second ends associated with the system distal end and system proximal end, respectively.

More particularly, the outer sheath first end may be coterminous with or, if separate from, may be associated with (e.g., joined or connected directly or indirectly) the distal end outer guide channel member at or near the transition region, while the outer sheath second end is associated with the handle at the system proximal end. The inner compression member first end is associated with the distal end inner guide channel member at or near the transition region, while the inner compression member second end is associated with the pusher stylet at the proximal end. Therefore, the outer guide channel member of the distal end may move axially (as described above) and independently relative to an approximately stationary inner guide channel member of the system distal end and, thereby, deploy the stent.

Before the earliest priority date claimed in this application, the ways of associating the inner compression member first end to the inner guide channel member has typically been to use a mechanical lap joint. A drawback to a mechanical lap joint connection is the propensity to lose the friction fit between the components. Accordingly, a glued joint is often employed as an alternative to a mechanical lap joint. While glue, adhesives, and the like (collectively, "glue") offer advantages over a mechanical joint, one must choose the right glue to join dissimilar materials. In any event, lap joints and glued joints may vary in strength and integrity depending on the type of materials being joined and whether the materials have incongruous mating surfaces. In addition, the point attachments that are typically formed by these joints could cause joint failure due to inadequate stress distribution, and may detach when a torque-load is applied.

Reference is directed, for example, to EP 1 095 634 which discloses a self-expanding stent delivery system comprising a proximal and distal shaft sections, a tapal distal tip, first and second proximal hubs and a stent. The distal shaft section includes an inner tubular body about which the stent is compressed and which is held compressed in place by a tubular cartridge, the introducer tip and an outer body sheath. The proximal shaft section may include a proximal shaft tube, a stabilising wire 34 and the first and second proximal hubs. An intermediate transition tube couples the proximal shaft tube to the distal outer body.

The present invention solves these and other problems by joining the inner compression member and the inner guide channel member together with an internal cannulated joint.

Therefore, it would be desirable to have an internal joint for a medical device delivery system for self-expanding devices such as stents, prosthetic valve devices, and other implantable articles inside a patient's body as taught herein.

### Summary of the Invention

The present invention is set for in the appended claims. In one embodiment, an elongate inner compression member has a proximal end portion and a distal mating end portion, and an inner guide channel member has a first end portion, a second end portion, and a channel therebetween. An insert body has a distal mating end portion with a first connection operatively coupled to the inner guide channel member second end portion, and a proximal connecting end portion with a second connection operatively coupled to the inner compression member distal mating end portion, wherein one of the first and second connections comprises a melt bond.

In another embodiment, an elongate internal compression member includes a proximal end portion and a distal mating end portion. An inner guide channel member has a first end portion, a second end portion, and a channel therebetween. An insert body has a distal mating end portion implanted into the inner guide channel member second end portion, and a proximal connecting end portion operatively coupled to the inner compression member distal mating end portion.

In yet another embodiment of an internal joint for use in a medical device, an inner guide channel member has a first end portion, a second end portion, and a channel therebetween. An insert body has a mating end portion and a proximal end portion defining a lumen therebetween. An outer sleeve has a first end portion, a mounting end portion, and a lumen therethrough. The outer sleeve mounting end portion is disposed about the insert mating end portion, which is disposed about the inner member second end portion, and at least one junction is configured for operatively coupling the insert mating end portion, the inner guide channel second end portion, and the outer sleeve mounting end portion.
In still another embodiment, the present invention provides a delivery system configured for rapid insertion delivery of self-expanding devices such as stents, prosthetic valve devices, and other implantable articles inside a patient's body. The delivery apparatus includes a system proximal portion, an elongate flexible middle section delivery device having an inner compression member with a mating end portion, and a system distal portion having inner and outer guide channel members. An insert body has a distal mating end portion having an entry port and a proximal connecting end portion having an exit port and defining a lumen therebetween, the proximal connecting end portion being operatively coupled to the inner compression member distal mating end portion, and the distal mating end portion being operatively coupled to the inner guide channel member second end portion. Brief Description of the Drawing

Figure 1 is a schematic view, broken away, of a medical device system.

Figure 2 is an exploded side view, broken away, of a system proximal portion of a medical device.

Figure 2A shows longitudinally sectioned exploded side views of a handle first connector and a handle second connector.

Figure 2B shows a longitudinally sectioned side view of operatively coupled first and second connectors according to Figures 2A.

Figure 2C shows a longitudinally sectioned side view of a handle first connector and a handle second connector according to Figure 2B operatively coupling a strain relief member and/or an outer sheath.

Figure 3 is a longitudinally sectioned view along a partial length of an embodiment of an outer sheath of a middle section delivery device and/or for an outer guide channel member of a system distal portion of a medical device.

Figure 4 is a longitudinally sectioned view, broken away, of a system distal portion of medical device delivery system.

Figures 4A and 4B schematically represent cross sectional views of melt bonding of components; Figure 4A before melt bonding and Figure 4B after melt bonding.

Figure 5 is a longitudinally sectioned view, broken away, of an alternative embodiment of a system distal portion of a medical device delivery system.

Figure 6 is a longitudinally sectioned view of an embodiment of a system distal portion, shown having a portion of a wire guide.

Figure 7 is a longitudinally sectioned view, broken away, of another embodiment of a system distal portion of a medical device delivery system.

Figures 7A, 7B, and 7C show cross sectional views of Figure 7 taken along the lines 7A-7A, 7B-7B, and 7C-7C, respectively.

Figure 8A is a perspective, schematic view of an insert body for joining two components.

Figure 8B is a longitudinally sectioned side view of Figure 8A.

Figures 8C through 8I are perspective, schematic views of alternative embodiments of insert bodies.

Figure 8J shows a schematic perspective view of an optional outer sleeve.

Figure 8K shows a longitudinally sectioned side view, broken away, of the outer sleeve of Figure 8J.

Figure 8L shows longitudinally sectioned and broken away alternative embodiment an optional outer sleeve according to Figure 8J.

Figure 9 is a sectional view, broken away, showing a distal end of a delivery device having an internal joint.

Figures 9A and 9B are longitudinally sectioned, broken away, schematic views showing alternative embodiments of an internal joint.

Figures 9C through 9G Figures 9B are schematic diagrams illustrating a method of implanting an inner compression member into an insert body and of implanting an insert body into an inner guide channel member.

Figure 10 is a sectional view, broken away, showing a distal end of a delivery device having an internal joint according to an alternative.

Figure 10A is a cross sectional view of Figure 10 taken along lines A-A.

Figure 10B is a sectional view, broken away, showing a distal end of a delivery device having an internal joint according to an alternative.

Figure 10C is a cross sectional view of Figure 10B taken along lines A-A.

Figure 10D is a sectional view, broken away, showing a distal end of a delivery device having an internal joint proximal connecting end according to an alternative.

Figure 10E is a sectional view, broken away, showing a distal end of a delivery device having an internal joint proximal connecting end according to an alternative.

### DETAILED DESCRIPTION

The present invention relates to medical devices, and in particular to an internal joint for joining an inner compression member and
an inner guide channel member for use in a delivery system configured for deploying expandable metallic, polymeric, and plastic devices or non-expanding metallic, polymeric, and plastic devices, which devices may include, by way of example and not by way of limitation, stents, prosthetic valve devices, and other implantable articles at selected locations inside a patient's body. For conciseness and ease of description of the embodiments of the invention, the term "stent" and its variations shall refer individually and collectively (without limiting the invention) to all self-expanding, balloon-expandable, or non-expanding devices used with the invention, such as stents, prosthetic valve devices, and other implantable articles inside a patient's body.

For the purposes of promoting an understanding of the principles of the invention, the following provides a detailed description of embodiments of the invention as illustrated by the drawings as well as the language used herein to describe the aspects of the invention. The description is not intended to limit the invention in any manner, but rather serves to enable those skilled in the art to make and use the invention. As used herein the terms comprise(s), include(s), having, has, with, contain(s) and the variants thereof are intended to be open ended transitional phrases, terms, or words that do not preclude the possibility of additional steps or structure.

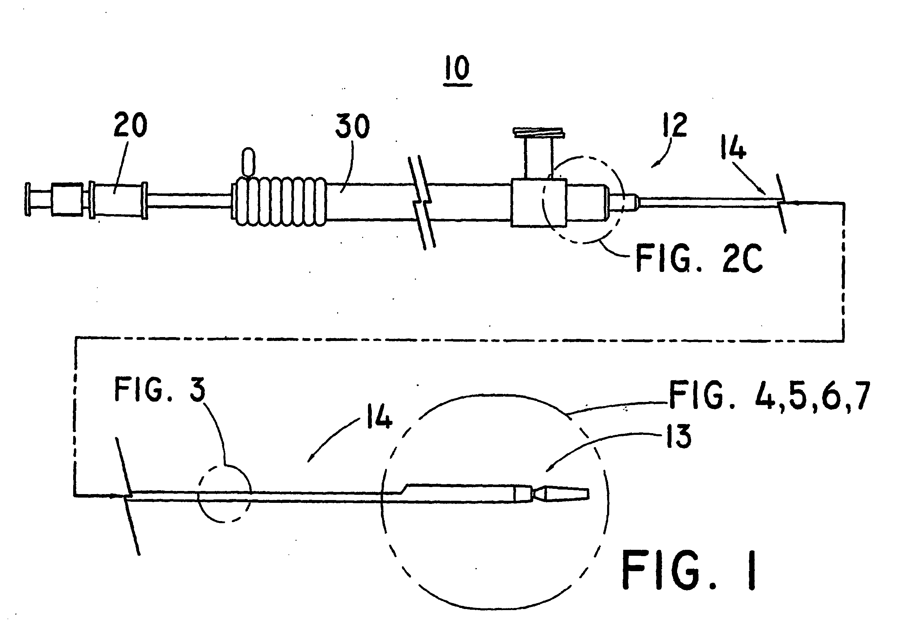

In Figure 1, an illustrative embodiment of a delivery system 10 having a host of uses, including for the rapid insertion of self-expanding stents, is provided. The delivery system 10 comprises a system proximal portion 12, a middle section delivery device 14, and a system distal portion 13 shown in a partially deploying position.

### SYSTEM PROXIMAL PORTION 12

In the embodiment shown in Figure 1, the proximal portion 12 remains outside of the patient's body. The proximal portion 12 comprises a handle 30 and an optional pusher stylet 20.

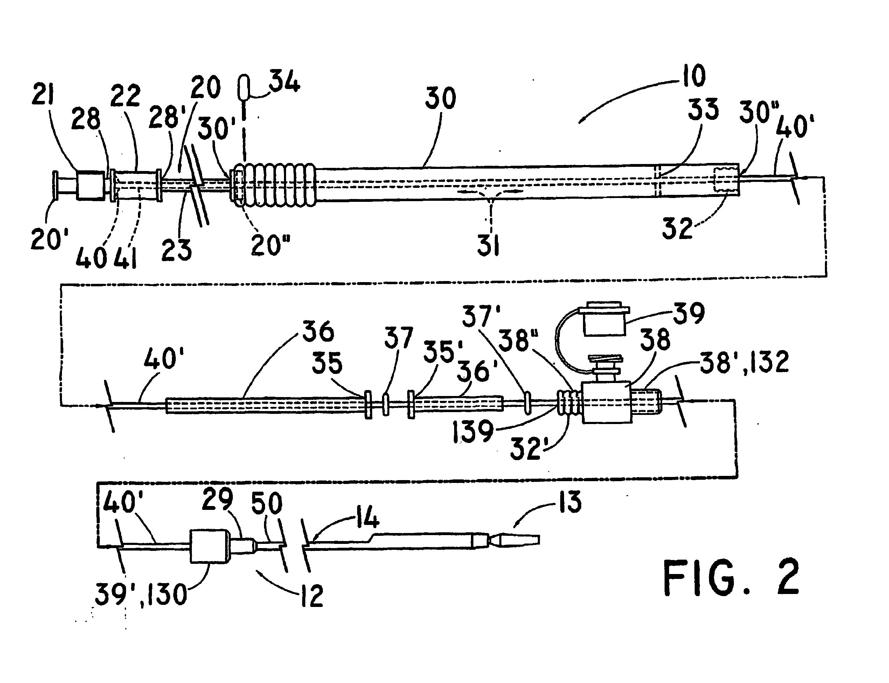

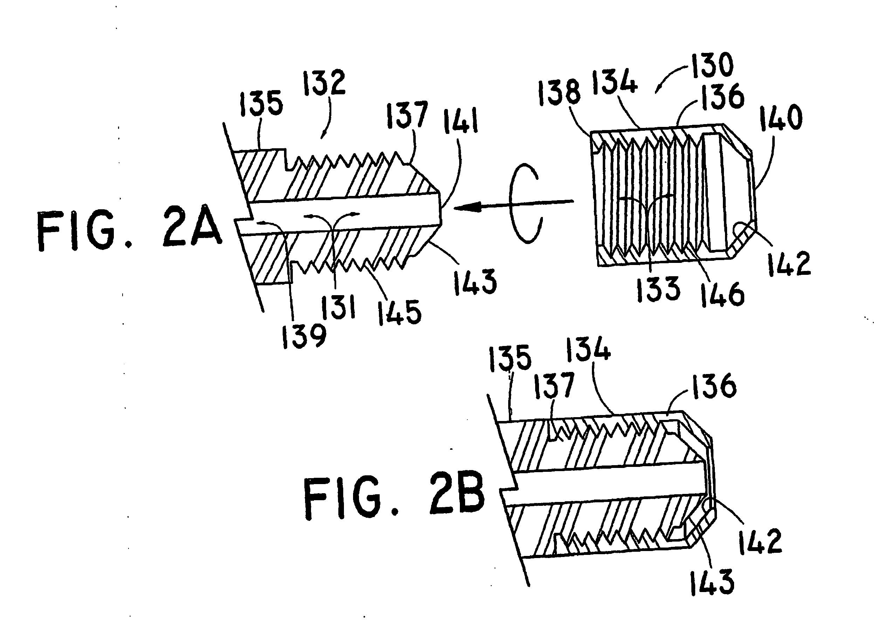

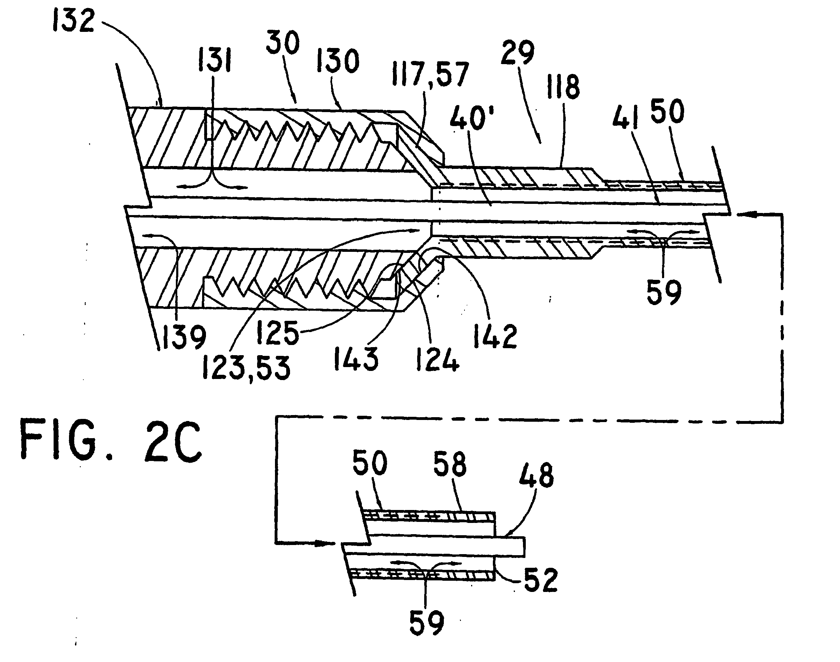

Figure 1 depicts a schematic representation of the handle 30 and the optional pusher stylet 20 shown more particularly in Figure 2. In general, a handle 30 retracts an outer guide channel member (discussed below) of the distal portion 13 of the delivery system 10 to deploy a stent, as will be explained later. The handle 30 may comprise any mechanical, electromechanical, pneumatic, or hydraulic handle configured in communication with-directly or indirectly through intervening parts-the distal portion's outer guide channel member. Communication would include, by way of illustration and not by way of limitation, a handle 30 that uses or is otherwise associated with, directly or indirectly, an elongated mechanical wire, rod, shaft, cable, sheath, pneumatic tube, or hydraulic pistons, cylinders and/or flow paths configured for moving the outer guide channel member proximally in order to deploy a stent.

Figure 2 provides a schematic view, broken away, of a delivery system 10 for rapid insertion of self-expanding stents, but could be used with other implantable prostheses described above. The delivery system 10 shown in Figure 2 is one embodiment of the proximal portion 12, middle section delivery device 14, and distal portion 13 shown in a partially deploying position. The middle section delivery device 14 extends distally from the proximal portion 12, and a distal portion 13 extends to a position that is distal the middle section delivery device 14. More particularly, Figure 2 shows an exploded view of the proximal portion 12 of the delivery system 10 according to one embodiment of the invention, with an emphasis on the handle 30 and the optional stylet 20. Features of one embodiment of a handle 30 and pusher stylet 20 are discussed below.

The handle 30 comprises any tubular structure having a distal aperture 30" and a proximal aperture 30', the apertures defining a chamber 31 therebetween. In general, the handle 30 is a component, instrument, mechanism, tool, device, apparatus, or machine configured for directly or indirectly retracting an outer guide channel member (discussed below) of the distal portion 13 of the device to expose and, ultimately, to deploy a stent self-expanding implantable prostheses such as stents, prosthetic valve devices, and other implantable articles (hereafter, "sent" or "stents") at a selected location inside a patient's body.

The handle 30 is axially slideable relative to an elongate (long) inner compression member 41 that comprises a proximal end 40 and a middle section 40'. As discussed more fully below, the inner compression member 41 helps to keep the stent from moving proximally with proximal movement of the handle 30, which handle movement causes the outer guide channel member to withdraw proximally over the stent in order to expose and thereby to deploy the stent. Thus, the inner compression member helps to "push" the stent or stent carrying inner guide channel member in order to counter the urge for the stent or stent carrying member to prolapse proximally with the withdrawing of the outer guide channel member. As will be understood, "pushing" on the inner compression member will keep the stent carrying inner guide channel member (and therefore the stent) from translating as a result of an outer sheath or outer guide channel member being pulled over the stent; thereby "pushing" holds the stent in place at the desired deployment site within the patient's body. In one embodiment, the handle 30 is a unidirectional handle that is axially slideable relative to the inner compression member 41 and/or the optional pusher stylet 20 in order to deploy a stent. In one embodiment, the inner compression member 41 is secured to a pusher stylet 20.

As shown in Figure 2, one embodiment of a pusher stylet 20 comprises a proximal end 20', a distal end 20", and a cannula 23 intermediate the proximal and distal ends 20', 20", respectively, and a receptacle 22. The cannula 23, as should be understood, comprises any suitable hollow plastic or metal tube. As a hollow tube, the cannula 23 optionally allows the inner compression member 41 to pass proximally through the cannula 23 and to the proximal end 20' so that the inner compression member proximal end 40 (such as a proximal end that is flared) may secure to a plug 21 that fits within the receptacle 22, wherein Figure 2 shows the proximal end 20', plug 21, and an optional securing material 28 are shown in an exploded view relative to the receptacle 22 into which they may be secured. Furthermore, the cannula 23 assists with keeping that portion of the inner compression member substantially straight.

The stylet 20 is optional, because in an alternative embodiment the physician may hold the inner compression member proximal end 40' directly in order to "push" (e.g., hold substantially stationary) the stent carrying inner guide channel member (and therefore the stent). This controls the stent carrying inner guide channel member and stent from translating as a result of an outer sheath or outer guide channel member being pulled over the stent, so that the stent remains at the desired deployment site within the patient's body. Alternatively, the stylet 20 is any stationary handle secured to the inner compression member 41 for achieving the "pushing" (e.g., hold substantially stationary) of the stent or stent carrying inner guide channel member while the outer sheath or outer guide channel member are moved proximally.

The stylet distal end 20" is housed within the handle chamber 31 and is flared or otherwise flanged sufficiently to be larger than the handle proximal aperture 30' so as not to pull out of the chamber 31. In one embodiment, the stylet distal end 20" is secured to the distal portion of the stylet cannula 23, while in another embodiment the stylet distal end 20" is formed integral with the distal portion of the stylet cannula 23. Consequently, the stylet distal end 20" functions as a proximal stop that prevents the stylet cannula 23 from backing all the way out the handle while being axially slideable within the handle chamber 31. Thus, the stylet 20 will not slide off the handle 30, if so desired. The stylet distal end 20" may also, in one embodiment, function as a distal stop against a restraint 33 formed in the handle chamber 31 intermediate the handle proximal and distal apertures 30', 30", respectively, where intermediate should be understood to be any position between, and not necessarily equidistant to, the handle apertures 30', 30". As a result of the stylet distal end 20", the handle 30 may slide axially the distance separating the handle restraint 33 and the stylet distal end 20", which has a maximum distance of when the stylet distal end 20" is abutting the handle proximal aperture 30'.

A threaded tapered plug 21 and threaded tapered receptacle 22 optionally secure the inner compression member proximal end 40. In one embodiment, the inner compression member proximal end 40 is flared. Securing material 28, such as glue, adhesives, resins, welding, soldering, brazing, chemical bonding materials or combinations thereof and the like (collectively and individually, "glue") may be used to keep the threaded tapered plug 21 from backing out of the threaded tapered receptacle 22. A portion of the cannula 23 and stylet distal end 20" are received within the handle chamber 31 distal to the handle proximal aperture 30' as previously explained.

By optionally placing the inner compression member proximal end 40 in mechanical communication with the plug 21 and receptacle 22, the gripping and "pushing" (e.g., hold substantially stationary) on the stylet 20 (e.g., the receptacle 22) thereby helps to keep the inner compression member 41 from moving away from the distal portion 13 and, accordingly, counters the tendency for a stent or stent carrying member to move proximally during withdrawal of the outer guide channel member as will be explained below. Of course, the inner compression member may be secured elsewhere by the stylet 20, such as at or near the stylet distal end 20" or intermediate the stylet proximal and distal ends 20', 20", respectively, and the stylet distal end 20" may extend to a position at or near the distal end aperture 30" of the handle 30.

Figure 2 shows a middle section 40' that extends distally from the proximal end 40 of the inner compression member 41. In one embodiment, the middle section 40' passes through the handle 30 (and may pass through the cannula 23 and/or bushings housed within the handle chamber 31 or other portions of the proximal portion 12). In one embodiment, the middle section 40' is elongate (at least 50.0 cm or longer as described below) and extends to a distance distally of the handle 30 and to a position at or near the medical system delivery device distal portion 13. It should be understood that, by describing the middle section 40' as passing through the handle 30, the middle section 40' does not necessarily need to pass proximally through the entire length of the handle 30, such as in an embodiment (by way of example and not by way of limitation) where the proximal end 40 of the inner compression member 41 is secured to a distal portion of the cannula 23 and/or the stylet distal end 20" extending within the handle chamber 31 to a position at or near the handle restraint 33.

In addition to holding a threaded tapered plug 21 and optionally the proximal end 40 of the inner compression member 41, the threaded tapered receptacle 22 may secure the proximal portion of the optional cannula 23. Glue 28' may be used at or near an interface of the cannula 23 and distal aperture of the threaded tapered receptacle 22. The glue 28' serves many functions, such as to keep dust from settling within the threaded tapered receptacle 22, to make the cannula 23 easier to clean, and to give aesthetics and a smooth feel to the device.

The handle 30 slidably receives the distal portion of the cannula 23 within the handle aperture 30' and handle chamber 31. As a result, the handle 30 is slideable relative to the stylet 20 (e.g., slideable relative to the threaded tapered plug 21, threaded tapered receptacle 22, and the cannula 23). In use, the physician grips the handle 30 in one hand and grips the stylet 20 (e.g., the receptacle 22) in the other hand. The physician holds the stylet 20 relatively stationary, which prevents the inner compression member and inner guide channel member and its stent carrying portion from moving proximally, and then withdraws the handle 30 proximally relative to the stationary stylet 20 and inner compression member 41. As a result, the physician is thereby retracting an outer guide channel member (discussed below) of the distal portion 13 of the delivery system 10 to expose and, ultimately, to deploy a stent locatable at the distal portion 13 of the delivery system 10. The handle 30 is in communication with-directly or indirectly through intervening parts-the outer guide channel member at the distal portion 13.

as shown in Figure 2, some of those optional parts may include the following: a first bushing 36 having an optional first bushing flange 35; a second bushing 36' having an optional second bushing flange 35'; an intermediate seal 37 intermediate the first and second bushing flanges 35, 35', respectively; a second seal 37' intermediate the second bushing flange 35' and a check flow body 38; and a detachable cap 39, such a Luer cap by way of example but not by way of limitation. In one embodiment, one or both of the intermediate seal 37 and the second seal 37' is from a class such as an O-ring. In another embodiment, one or both of the intermediate seal 37 and the second seal 37' is a cylinder or disk with a center aperture, and may be made from material that comprises an O-ring. The bushings 36, 36' are hollow plastic or metal tubes that take up space within the handle 30 so that the inner compression member has less room to buckle. Fully assembled in one embodiment, the first bushing 36 is inserted within the cannula 23 and the first bushing flange 35 is distal to and abutting the handle restraint 33, which is sized to interfere with the bushing flange 35 to prevent the bushing flange 35 from moving proximal to the handle restraint 33. The second bushing flange 35' is distal to and optionally abutting the bushing flange 35 so to prevent it from moving proximal the first bushing flange 35, and the second bushing 36' is inserted within an opening 139 of the check flow body 38. The intermediate seal 37 and the second seal 37' help to prevent fluids that could be used with the device (discussed below) from entering the handle chamber 31, which directs fluids distally, which fluids may be conveyed through an outer sheath 50 of the middle section delivery device 14 and system distal portion 13. In one embodiment, the handle restraint 33 is from a class such as a counter bore wherein the restraint 33 comprises, by way of example only and not by way of limitation, a flat-bottomed cylindrical enlargement of the handle chamber 31 sized for receiving a first bushing flange 35, an intermediate seal 37, a second bushing flange 35', and/or a check flow body proximal mating end 38" intermediate the restraint 33 and the handle distal aperture 30".

The handle 30 and check flow body 38 operatively couple with the handle distal aperture 30" receiving a check flow body proximal mating end 38" and being secured together by any suitable means, including but not limited to a crimp, friction fit, press fit, wedge, threading engagement, glue, adhesives, resins, welding (laser, spot, etc.), soldering, brazing, adhesives, chemical bonding materials, or combinations thereof. In one embodiment, the handle 30 comprises a coupling member 32 and the check flow body proximal mating end 38" comprises a coupling member 32', the coupling members 32, 32' being complementary to hold the handle 30 and check flow body proximal mating end 38" together. In one embodiment, the coupling members 32, 32' may form complementary threads. If it is desired to achieve quicker assembly for manufacturing purposes, then the coupling members 32, 32' may be an array of circumferential ridges that form an interference fit when pressed together. If a one-time snap fit is desired, then the coupling members 32, 32' may be circumferential ridges in the form of barbs. In another embodiment, the handle 30 and check flow-body proximal mating end 38" may be put together and taken apart for servicing, in which case the coupling members 32, 32' may be circumferential ridges in the form of knuckle threads (e.g., circumferential ridges forming complementary undulating waves). The operatively coupled handle 30 and check flow body proximal mating end 38" according to these embodiments may be fixed such that they do not rotate relative to each other, or may rotate while preventing undesired axial separation.

During use, the detachable cap 39 may be detached or opened and the device flushed with saline to remove air in order to help keep air out of the patient. The intermediate seal 37 and the second seal 37' ensure that any flushed fluid moves distally in the device and does not back up into the handle 30, such as between the handle restraint 33 and the first bushing 36, into the handle chamber 31, or out the handle proximal aperture 30'. The detachable cap 39 (such as a Luer cap) keeps saline from backing out of the check flow body 38, air from flowing into the check flow body 38, and blood from rushing out during periods of high blood pressure inside the patient.

The medical device delivery systems 10 may be used to deploy an implantable prosthesis that is a balloon expandable or self-expanding stent, prosthetic valve device, or other implantable articles provided on the distal portion of a delivery system. In operation, a physician inserts the distal portion and at least a portion of the middle section delivery device into a vessel passageway, and advances them through the vessel passageway to the desired location adjacent the target site within the vessel passageway of a patient. In a subsequent step, the physician moves the handle proximally, which withdraws the outer sheath and/or the outer guide channel member and releasably exposes the stent for deployment. In another step, the physician inflates the expandable member, such as a balloon, positioned under the stent inner surface to plastically deform the stent into a substantially permanent expanded condition. The physician may inflate the expandable members by injecting fluid such as saline from a syringe into the inner compression member 41, via pusher stylet 20, through a Luer fitting at the proximal end 20'. Therefore, the fluid is directed distally to the expandable member, filling the expandable member chamber and expanding the stent. The physician then deflates the balloon and removes the catheter or delivery device from the patient's body.

In one embodiment as shown in Figure 2, the handle 30 further comprises a check flow body distal mating end 38' and a connector cap 39' (optionally detachable) secured to the check flow body distal mating end 38', and a strain relief 29. In one embodiment, the connector cap 39' is from a class of fasteners such as nuts, and in one embodiment is a flare nut. The connector cap 39' functions to hold (or assist in holding in combination with the check flow body distal mating end 38') a flared proximal portion of an outer sheath 50 and/or a flared strain relief 29 disposed about (and optionally extending proximally from) that held portion of the outer sheath 50. The strain relief member 29 provides a kink resistant point where the outer sheath 50 connects to the connector cap 39' and/or the check flow body distal mating end 38'.

The check flow body distal mating end 38' and connector cap 39' may be operatively coupled mechanically, chemically, and/or chemical-mechanically. In one embodiment, the connector cap 39' is crimped, friction fitted, press fitted, and/or wedged into engagement onto the check flow body distal mating end 38'. In another embodiment for example, the check flow body distal mating end 38' and connector cap 39' are operatively coupled by glue, adhesives, resins, welding (laser, spot, etc.), soldering, brazing, adhesives, chemical bonding materials, or combinations thereof.

According to Figure 2A, yet another embodiment of the connector cap 39' comprises a handle first connector 130 and the check flow body distal mating end 38' comprises a handle second connector 132. According to Figure 2A, the handle first and second connectors 130, 132, respectively, function to operatively couple a strain relief member 29 operatively coupled to the proximal portion of the outer sheath 50 (discussed below). In one embodiment, the handle first connector 130 is from a class of fasteners such as nuts, and in one embodiment is a flare nut. Optionally, the distal portion of the second bushing 36' is sized (but for the second bushing flange 35') to be received within a check flow body proximal opening 139 in communication with the second connector 132.

Figure 2A shows an exploded longitudinally sectioned side view of one embodiment of a portion of the handle comprising a first connector 130 and a second connector 132. The handle first connector 130 further comprises a proximal portion 134 and a distal portion 136. An opening 138 at the proximal portion 134 and an opening 140 at the distal portion 136 and define a lumen 133 therebetween. There is an engaging surface 142 at or near the distal portion 136. A threaded first piece 146 is disposed within the lumen 133 and intermediate the handle first connector distal end opening 140 and proximal end opening 138. The handle second connector 132 further comprises a proximal portion 135 and a distal portion 137. An opening 141 at the distal portion 137 and check flow body proximal opening 139 (e.g., Fig. 2) at the proximal portion 135 define a lumen 131 therebetween. There is an engaging surface 143 at or near the distal portion 137. A threaded second piece 145 is disposed on the outside surface and intermediate the handle second connector distal end opening 141 and the check flow body proximal opening 139.

According to one embodiment shown in Figures 2A and 2B, the second connector distal portion 137 is received within the first connector proximal end opening 138. The first connector 130 and second connector 132 are operatively coupled by a threading engagement between the first connector threaded first piece 146 and the second connector threaded second piece 145. Alternatively, the first connector 130 and second connector 132 are operatively coupled mechanically, chemically, and/or chemical-mechanically. In one embodiment for example, the first connector 130 and second connector 132 are crimped, friction fit, press fit, and/or wedged into engagement. In another embodiment for example, the first connector 130 and second connector 132 are operatively coupled by glue, adhesives, resins, welding (laser, spot, etc.), soldering, brazing, adhesives, chemical bonding materials, or combinations thereof.

Figure 2B shows the second connector threaded second piece 145 operatively coupled to the first connector threaded first piece 146 such that the second connector proximal portion 135 is proximal to the first connector proximal portion 134 and the second connector distal portion 137 is located at or near the first connector distal portion 136. As shown in Figure 2B, the second connector engaging surface 143 is spaced proximal to the first connector engaging surface 142 for receiving and compressing a strain relief member second end portion therebetween.

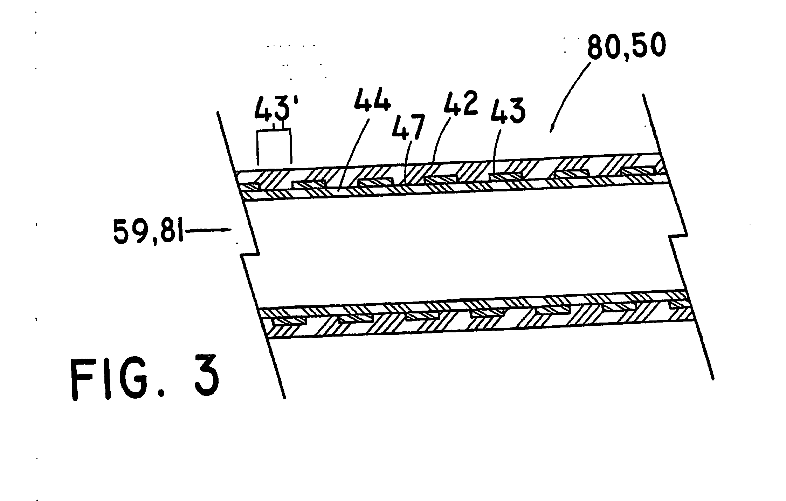

Figure 2C shows one embodiment of an optional strain relief member 29 comprising a tubular first end portion 118 and a flared second end portion 117. According to Figure 2C, the medical device delivery system includes an elongate outer sheath 50 (Figs. 3, 4, 5, 6, 7). Like elements from the previous drawings, embodiments, and description from above are labeled the same. The term elongate is used, not lexicographically but instead, to describe embodiments according to the embodiment that measures at least about 50.0 cm or measures within one of the ranges of lengths exceeding 50.0 cm and as more fully discussed above.

More particularly, Figure 2C shows that the outer sheath 50 comprises a proximal end portion 57 and a distal end portion 58. The distal end portion 58 comprises an opening 52 and the proximal end portion 57 comprises an opening 53; the openings define a passageway 59 therebetween. In one exemplary embodiment according to Figure 2C, the strain relief member tubular first and second end portions 118, 117, respectively, are disposed about and operatively coupled to the outer sheath proximal end portion 157. In another embodiment, the tubular first end portion 118 disposes about the outer sheath proximal end portion 157 while the flared second end portion 117 extends proximally from outer sheath proximal end portion 157. In addition, the strain relief member second end portion 117 and/or outer sheath proximal end portion 57 comprise an opening 123 in fluid communication with the outer sheath passageway 59.

By way of example only and not by way of limitation, the terms "operatively coupling," "operatively coupled," "coupling," "coupled," and variants thereof are not used lexicographically but instead are used to describe embodiments of the invention having a point, position, region, section, area, volume, or configuration at which two or more things are mechanically, chemically, and/or chemical-mechanically bonded, joined, adjoined, connected, associated, united, mated, interlocked, conjoined, fastened, held together, clamped, crimped, friction fit, pinched, press fit tight, nested, wedged, and/or otherwise associated by a joint, a junction, a juncture, a seam, a union, a socket, a melt bond, glue, adhesives, resins, welding (laser, spot, etc.), soldering, brazing, adhesives, chemical bonding materials, implanted arrangement, or combinations thereof.

Figure 2C shows the strain relief member second end portion 117 and/or outer sheath proximal end portion 57 being operatively coupled between the first connector 130 and the second connector 132, and the second connector lumen 131 being in fluid communication with the outer sheath passageway 59. In one embodiment, the strain relief member second end portion 117 and/or outer sheath proximal end portion 57 comprises a first opposing surface 124 and a second opposing surface 125. The first connector engaging surface 142 is disposed against the first opposing surface 124 and the second connector engaging surface 143 is disposed against the second opposing surface 125, whereby the strain relief member second end portion 117 and/or outer sheath proximal end portion 57 becomes operatively coupled between the first and second connector engaging surfaces 142,143, respectively. In one embodiment, the operatively coupled strain relief member second end portion 117 and/or outer sheath proximal end portion 57 is compressed (e.g., sandwiched) between the first and second connector engaging surfaces 142, 143.

Thus, the check flow body 38 provides an optional three way connector. The check flow body proximal mating end 38" and handle coupling member 32 are operatively coupled. The side port is controlled by the detachable connector cap 39. The body distal mating end 38' is operatively coupled to a second connector cap 39', or optionally the handle second connector 132 is received within and operatively coupled to a handle second connector cap 130.

The foregoing description of a proximal portion 12 of a medical device delivery system 10 according to one embodiment of the invention may be one assembly during shipping, or may include a two-part assembly or more. Otherwise stated, the stylet 20 and handle 30 may be sold already combined or may be combined after purchase by inserting the stylet cannula 23 into the handle at the hospital via the threaded tapered plug 21 and threaded tapered receptacle 22. An optional safety lock 34 helps to ensure against unintentional actuation by preventing distal movement of the stylet distal end 20" by extending inwardly within the handle chamber 30 through a slot in the handle outer wall distal to the handle proximal aperture 30'. Consequently, the optional safety lock 34 thereby maintains the handle 30 in an undeployed position until the physician is ready to deploy an implantable prosthesis (e.g., a self-expanding, balloon expandable, or non-expanding stent; prosthetic valve devices, and other implantable articles) at a selected location inside a patient's body.

### MIDDLE SECTION DELIVERY DEVICE

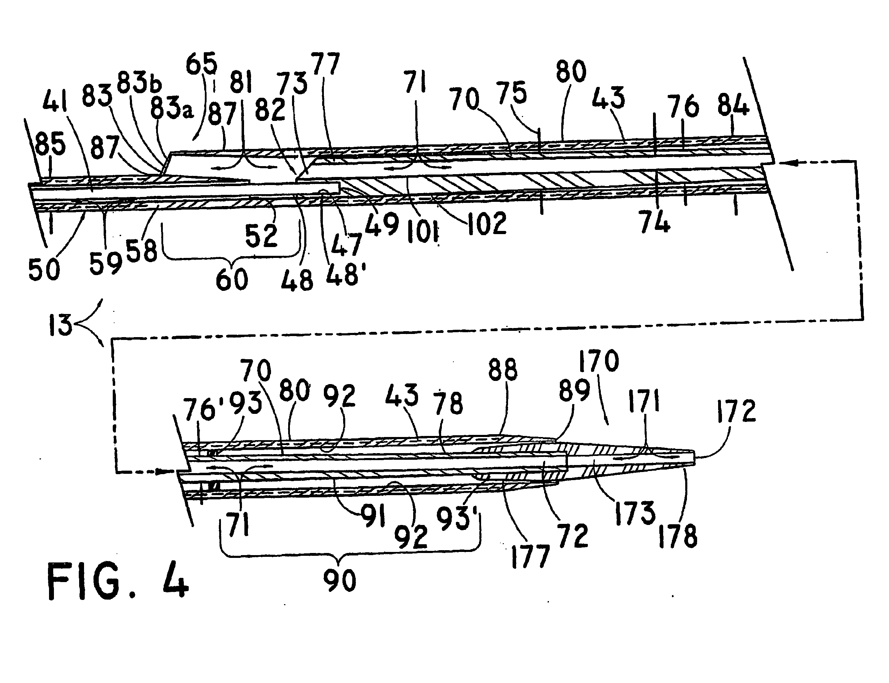

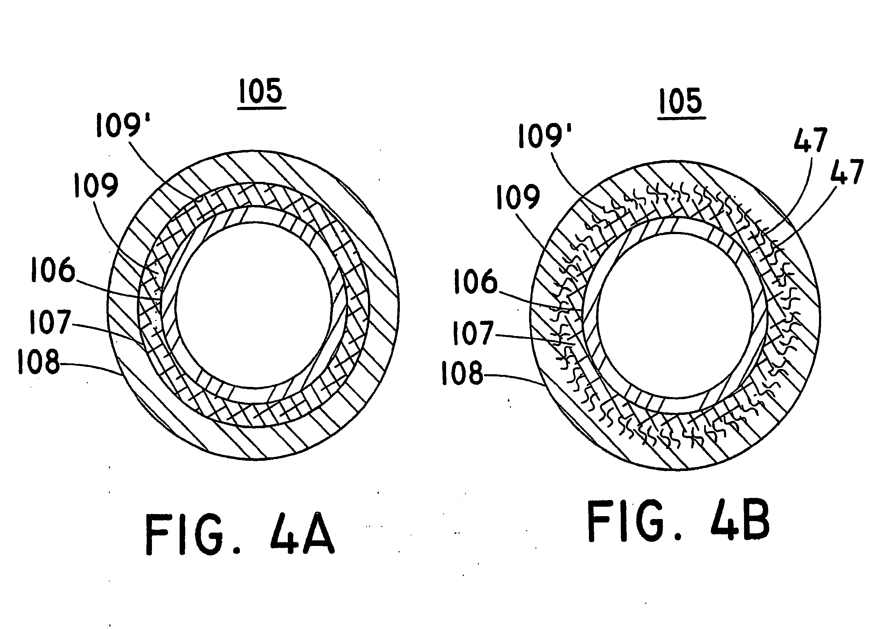

A delivery system 10 as shown in Figures 1 and 2 comprises a middle section delivery device 14. According to the invention, the middle section delivery device 14 is intermediate the proximal portion 12 (Figs;-1-,2) and the distal portion 13 (Figs. 1, 2) of the delivery system 10. The term "intermediate" is intended to describe embodiments of the invention whereby the middle section delivery device 14 is intermediary, intervening, lying or occurring between two extremes, or spatially in a middle position, state, or nature-though not necessarily equidistant-between the distal tip of the distal portion 13 and the proximal tip of the proximal portion 12. Furthermore, the middle section delivery device 14 may overlap or be partially inserted into a portion of the distal portion 13 and/or the proximal portion 12. In another embodiment, a portion of the middle section delivery device 14 (such as the sheath 50 explained below) and the distal end portion outer guide channel member 80 (discussed below; see Figs. 4, 5, 6, 7) may be an elongate tubular catheter or Flexor® sheath of integral construction.

According to the invention, a middle section delivery device 14 is a flexible, elongate (long, at least about 50.0 centimeters ("cm")) tubular assembly. In one embodiment, the middle section delivery device 14 is from approximately 100.0 centimeters ("cm") to approximately 125.0 cm for use when placing a distal portion 13 of the invention within a patient's body, although it may be sized longer or shorter as needed depending on the depth of the target site within the patient's body for delivering the stent. The term "tubular" in describing this embodiment includes any tube-like, cylindrical, elongated, shaft-like, rounded, oblong, or other elongated longitudinal shaft extending between the proximal portion 12 and the distal portion 13 and defining a longitudinal axis. As used herein and throughout to describe embodiments of the invention, the term "longitudinal axis" should be considered to be an approximate lengthwise axis, which may be straight or may at times even be curved because the middle section delivery device 14, for instance, is flexible and the distal portion 13 also may be substantially or partially flexible.

A middle section delivery device 14 comprises an outer sheath 50 (e.g., Figs. 2, 2C, 5, 6, 7). Figure 2C shows that the outer sheath 50 is generally tubular and comprises a proximal end portion 57 and a distal end portion 58 and defining a passageway 59 therebetween (e.g., Fig. 2C). In one embodiment, the distal end portion 58 comprises an opening 52 and the proximal end portion 57 comprises an opening 53, which openings define the passageway 59. The middle section delivery device 14 further comprises an elongate inner compression member 41 (e.g., Figs. 2, 2C, 5, 6,7). The outer sheath passageway 59 is configured for slideably receiving the inner compression member 41, a catheter, or other medical device.

Figure 3 depicts an enlarged, longitudinally sectioned view along a partial length of one embodiment of an outer sheath 50 for use as the middle section delivery device 14, with the delivery system's proximal and distal portions 12, 13, respectively, of the device being removed for clarity. In one embodiment, the outer sheath 50 comprises three layers: an inner layer 44 comprising Teflon material; a middle layer comprising a stainless steel circumferential spiral coil 43; and an outer layer 42 comprising a nylon, a polyether block amide ("PEBA"), and/or other melt bonding material discussed below. The outer layer 42 and inner layer 44 optionally may comprise a lubricious material, one example of which includes a fluorocarbon such as polytetrafluoroethylene (PTFE), to present a slideable surface to allow easier inserting and retracting the middle section delivery device 14 for deploying a self-expanding stent, as will be explained later.

The wall of the inner layer 44 of the outer sheath 50 has sufficient radial rigidity to decrease any tendency of bulging, kinking, and the like, under an internal radial expansile force. In other words, the inner layer 44 resists an inner object from protruding or becoming embedded into the inner layer 44, which is beneficial to the slideability of an outer sheath 50. The coil 43 may be compression fitted or wound around the inner layer 44. The coil 43 includes a plurality of turns, and preferably includes uniform spacings 43' between the turns of the coil 43. The coil 43 may be formed of any suitable material that will provide appropriate structural reinforcement, such as stainless steel flat wire or biologically compatible metals, polymers, plastics, alloys (including super-elastic alloys), or composite materials that are either biocompatible or capable of being made biocompatible.

Although the embodiment in Figure 3 shows a flat ribbon shaped wire coil 43, coils of other cross-sectional dimensions, such as round wire, may also be used. When flat wire stainless steel is used the coil 43 is optionally formed from wire that is about 0,00762 cm (0.003 inches) thick by about 0,0305 cm (0.012 inches) wide. In one embodiment, the turns of coil 43 are uniformly spaced 43' apart by approximately 0,03 cm (0.0118 inches). While Figure 3 shows an embodiment that uses coils 43 having uniformly spaced turns and a constant pitch, this is not required and coils 43 may be spaced 43' by non-uniform distances or at varying distances. In one embodiment, the ends of coil 43 are positioned approximately 0,5 cm (0.197 inches) proximal to the distal portion 13 and approximately 1,5 cm (0.591 inches) distal to the proximal portion 12_{.}

The outer sheath 50 for use with the middle section delivery device 14, and the outer guide channel member 80 (Figs. 4, 5, 6, 7) and/or the inner guide channel member 70 (Figs. 4, 5, 6, 7) for use with the distal portion 13, are available for purchase from Cook Incorporated, of Bloomington, Indiana under the trade name of "Flexor(R)." Examples of the Flexor(R) sheath devices, materials, and methods of manufacturing them are found in United States Patent Nos. 5,700,253 and 5,380,304. The Flexor® sheath is particularly suited for the outer sheath 50 of the middle section delivery device 14 and/or the outer guide channel member 80 of the distal second end portion 13 due to its thin PTFE liner on the inside wall of the inner layer 44, thin flat wire coil 43, and Nylon and/or PEBA overcoat 42 that captures the coil 43 and PTFE liner 44 and binds the structure together. The PTFE inner layer 44 of the Flexor® sheath resists an expansile inner object from protruding or becoming embedded into the inner layer 44 and, thereby, provides a slick, smooth surface that slides (e.g., across the surface of a stent if the Flexor® sheath is used with the distal portion 13 or across the surface of an inner compression member 41 if the Flexor® sheath is used with the middle section 14) relatively easily when retracted to expose, release, and deploy the stent or allow the outer sheath 50 to move relative to the inner compression member 41, and the outer guide channel member 80 to move relative to the inner guide channel member 70, during deployment of the stent.

As an alternative to purchasing the outer sheath 50 for use with the middle section 14 and the outer guide channel member 80 for use with the distal portion 13 from Cook Incorporated, one may manufacture the outer sheath and outer guide channel member from various component parts. For instance, one may purchase a tubular inner layer 44 comprising a lubricious material comprising a fluorocarbon such as polytetrafluoroethylene (PTFE or Teflon) from Zeus, Inc. in Orangeburg, South Carolina, and dispose that inner layer 44 over a mandrel. Alternatively, a sheet of material comprising Teflon may be positioned on a mandrel and formed into a tubular body for the inner layer 44 by any suitable means known to one skilled in the art.

The tubular inner layer 44 (whether formed from a sheet on a mandrel or purchased as a tube and slid onto a mandrel) may be slightly longer than the desired length described above for the outer sheath 50 and/or outer guide channel member 80, and slightly longer than the mandrel. In one embodiment, the tubular inner layer 44 may extend about 5.0 cm from each mandrel end. As explained below, the "loose" ends of the tubular inner layer 44 help during manufacturing of the device.

The mandrel-tubular inner layer 44 assembly is prepared for a middle layer comprising a stainless steel circumferential spiral coil 43 as described above and available for purchase from Cook Incorporated or Sabin Corporation in Bloomington, Indiana. As purchased, the coil 43 comes in a long, pre-coiled configuration and will be cut by hand or machine to the desired length either before or after winding the coil about the inner layer 44 to the desired length. As an alternative, one may manufacture the coil from raw material available from Fort Wayne Medical in Fort Wayne, Indiana, and process it into a spiral coil 43 shape.

The operator may apply the spiral coil 43 about the mandrel-tubular inner layer 44 assembly by hand or machine. If by hand, then an end of the spiral coil 43 may be started onto the tubular inner layer 44 by any suitable means, for example, such as hooking and winding (e.g., wrapping) the coil 43 around the tubular inner layer 44 in a pigtailed manner at an initial position a desired distance (e.g., 5.0 cm or more) from a first end of the tubular inner layer 44 and to a terminating position that is a desired distance (e.g., 5.0 cm or more) from a second end of the tubular inner layer 44, and then cutting the coil 43 at the terminating position before or after hooking the coil 43 onto the inner layer 44. If by machine, then chucks, for instance, may hold the opposing ends of the mandrel-tubular inner layer 44 assembly while the spiral coil 43 is threaded through an arm on a machine and started onto the tubular inner layer 44 at the initial position as described above. As the chucks rotate, the inner layer 44 rotates, and the arm moves axially down the length of the inner layer 44, thereby applying the coil 43 in a spiral configuration about the inner layer 44. The machine arm moves to a terminating position where the machine or operator cuts the coil before or after hooking the coil 43 onto the inner layer 44.

An operator then applies an outer layer 42 about the coil-inner layer-mandrel assembly. The outer layer 42 may comprise a polyether block amide, nylon, and/or a nylon natural tubing (individually and collectively, "PEBA" and/or "nylon"). The outer layer 42 preferably has a tubular configuration that disposes about (e.g., enveloping, surrounding, wrapping around, covering, overlaying, superposed over, encasing, ensheathing, and the like) a length of the coil-inner layer-mandrel assembly.

Heat shrink tubing, available from many suppliers, including Zeus, Inc. in Orangeburg, South Carolina for instance and also Cobalt Polymers in Cloverdale, California, may be disposed about the outer layer-coil-inner layer-mandrel assembly. Heating the assembly causes the outer layer 42 to melt. The inner surface of the outer layer 42 thereby seeps through spaces 43' in or between middle layer coils 43 and bonds to both the outer surface of the inner layer 44 and the coils 43. In one embodiment, the inner surface of the outer layer 42 forms a melt bond 47 (explained below) to the outer surface of the inner layer 44. Upon cooling, a solid-state bond results such that the assembly comprises the three layers discussed above. The operator removes the shrink wrap (e.g., by cutting) and withdraws the mandrel. The operator may cut the Flexor® sheath to a desired length for an outer sheath 50 and/or outer guide channel member 80.

The temperature, total rise time, and dwell time for the heat shrink-outer layer-coil-inner layer-mandrel assembly will vary depending on many factors including, for instance, the actual melt bonding material that the outer layer 42 comprises, and also the diameter of the desired Flexor® sheath. For example, the baking parameters for a 2.5 French Flexor® sheath may be approximately 193°C (380 degrees Fahrenheit) for about five minutes, while the baking parameters for a 4 French Flexor® sheath may be approximately 193°C (380 degrees Fahrenheit) for about six minutes.

As an alternative to a Flexor® sheath, the outer sheath 50 may comprise a construction of multifilar material. Such multifilar material or tubing may be obtained, for example, from Asahi-intec USA, Inc. (Newport Beach, California). Materials and methods of manufacturing a suitable multifilar tubing are described in Published United States Patent Application 2004/0116833 (Koto et al.) having an Application Serial No. 10/611,664 and entitled, "Wire-Stranded Hollow Coil Body, A Medical Equipment Made Therefrom and a Method of Making the Same,". Use of multifilar tubing in a vascular catheter device, for instance, is described in United States Patent No. 6,589,227 (Sonderskov Klint, et al.; Assigned to Cook Incorporated of Bloomington, Indiana and William Cook Europe of Bjaeverskov, Denmark).

In addition to the outer sheath 50, the middle section delivery device 14 further comprises an inner compression member 41. The delivery device 14 (and, thus, the outer sheath 50 and inner compression member 41) may be constructed to have any diameter and length required to fulfill its intended purposes.

The outer sheath 50, for instance, may be available in a variety of lengths, outer diameters, and inner diameters. In one embodiment, the outer sheath 50 may have a substantially uniform outer diameter in the range from approximately 2 French to approximately 7 French, and in one embodiment the diameter is from approximately 4 French to approximately 5 French in diameter. Otherwise stated, the outer sheath 50 may range from about 0.0254cm (0.010 inches) to about 0.2286cm (0.090 inches) in diameter, and in one embodiment the diameter is approximately 0.0127cm (0.050 inches). Likewise, the passageway 59 may be available in a variety of diameters. In one embodiment, the inner diameter ranges from about 0.08128cm (0.032 inches) to about 0.1016cm (0.040 inches), and in a preferred embodiment the passageway 59 is approximately 0.08128cm (0.032 inches). The diameter may be more or less than these examples, however, depending on the intended vessel passageway for the device. For instance, a larger vessel passageway (e.g., greater expandable inner diameter) may tolerate a bigger device with an outer sheath 50 having a correspondingly greater diameter. Conversely, a narrower vessel passageway may require a thinner outer sheath 50. Likewise, the overall length may vary. In one embodiment, the outer sheath 50 will have a length from about 50.0 cm (or about 19.685 inches) to about 125.0 cm (or about 49.213 inches), and more particularly between about 70.0 cm (or about 27.559 inches) and about 105.0 cm (or about 41.339 inches), and in yet another embodiment the length is approximately 100.0 cm (or about 39.370 inches).

The inner compression member 41 comprises an elongated pusher bar, stiffening member, or stiff polymer that helps to "push" the stent by pushing the stent carrying inner guide channel member at or near the distal portion 13 in order to counter the urge for the stent or stent carrying member to move as a result of an outer sheath or outer guide channel member being pulled over the stent; thereby "pushing" holds the stent in place at the desired deployment site within the patient's body. The inner compression member 41 "pushes" the stent by helping to prevent or minimize the inner guide channel member from prolapsing, recoiling, kinking, buckling, or moving; thereby keeping the inner guide channel member's stent platform on which the stent is disposed (discussed later) substantially stationary, for the most part, relative to the proximal retraction of the distal outer guide channel member (discussed below) that exposes and, thus, deploys the stent. The phrase "at or near" as used herein to describe an embodiment of the invention includes a location that is at, within, or a short distance such as about 0.1 cm to about 15.0 cm, although other ranges may apply, for instance from about 0.5 cm to about 10.0 cm.

The overall length of the inner compression member 41 may vary, as desired. In one embodiment the inner compression member 41 has a length from about 50.0 cm to about 175.0 cm, and more particularly between about 75.0 cm and 150.0 cm, and in one embodiment the length is approximately 125.0 cm to about 140.0 cm. A portion of the inner compression member 41 (e.g., the proximal end 40 and/or middle section 40') may be contained within the handle 30 and the stylet 20, as explained above (Fig. 2).

Likewise, the diameter or width of the inner compression member 41 may vary. In one embodiment, the inner compression member 41 has a diameter or width ranging from about 0.0254cm (0.010 inches) to about 0.0762cm (0.030 inches), by way of example only and not by way of limitation. In one embodiment, the inner compression member 41 has a diameter or width that is approximately 0.0406cm (0.016 inches). The diameter or width may be more or less than these illustrative ranges. For example, a deeper target site within a patient may require a thicker inner compression member 41 for greater push- ability, but may tolerate lesser flexibility. In addition, the material that the inner compression member 41 comprises determines whether a smaller and more flexible inner compression member 41 will give suitable flexibility, and also determines whether a wider inner compression member 41 may have the flexibility of a thinner inner compression member 41 made of different material. Furthermore, the inner compression member 41 may have a curved transverse cross-section, such as, for example, a circular cross-section, or it may have a polygonal cross-section, such as, for example, a rectangular cross-section. Alternatively, the transverse cross-section of the inner compression member may include both curved and straight portions. According to one embodiment, the inner compression member 41 may have a nonuniform diameter or width along its length. These various diameters, widths, and cross-sections may occur at the inner compression member proximal end 40, the inner compression member middle section 40', and/or the inner compression member distal mating end portion 48.

It should be understood that the diameter, width, and/or cross- section of the inner compression member 41 may taper. For example, the inner compression member 41 may taper toward the distal end portion as taught in the United States Provisional Patent Application filed on January 23, 2006 entitled, "Tapered Inner Compression Member and Tapered Inner Guide Channel Member for Medical Device Delivery Systems" and having a US publication number 2006-0259117.

Also, an inner compression member 41 may have an outer surface comprising a lubricious PTFE material and/or an inner surface 44 of the outer sheath 50 may comprise a lubricious PTFE material against the inner compression member 41, in order to allow easy retraction of the outer sheath 50, which is in communication with a distal outer guide channel member to deploy a self-expanding stent, as will be explained later.

Generally, the inner compression member 41 and outer sheath 50 may optionally be approximately the same in length, and the axial length of coil 43 will be less than the length of the inner compression member and outer sheath. In one embodiment, however, the inner compression member 41 comprises a proximal end 40 that extends proximal relative to the outer sheath. In yet another embodiment, the inner compression member extends to a position that is distal the outer sheath. In still another embodiment, the inner compression member 41 stops short of extending all the way to the distal tip of the delivery system 10, and may stop generally from 10 to 40 cm short of the distal tip of the delivery system 10, and in one embodiment it stops approximately 20 to 25 cm short of the distal tip of the delivery system 10, where the distal end portion of the inner compression member 41 is operatively coupled to a proximal portion of an inner guide channel member.

### SYSTEM DISTAL PORTION 13

Now turning to embodiments of a distal portion 13 of medical device delivery systems, Figures 4, 5, 6, and 7 show the distal portion 13 to be a relatively tubular body. Given the configuration of vessels, vessel passageways, a working channel of an endoscope, or an external accessory channel device used with an endoscope to be navigated, a mostly tubular distal end with a distal tapered, rounded, chamfered, or arrowhead shape may be better tolerated by the patient. Further, in certain embodiments, the distal portion of the distal portion 13 may be soft, rounded, and flexible so as to provide further protection for and care to the patient.

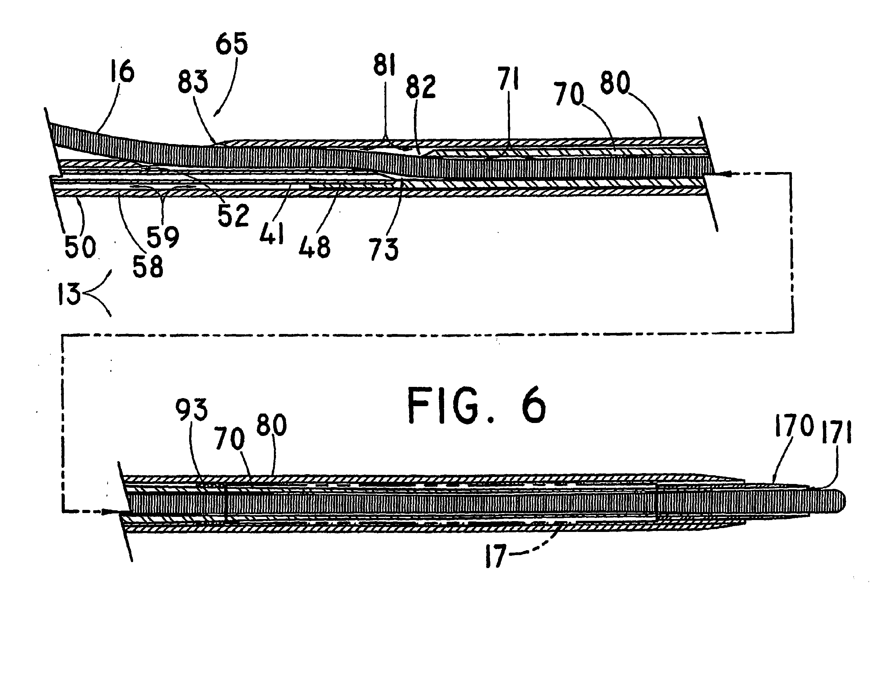

Figure 4 illustrates an embodiment of the distal portion 13 of a delivery system for the rapid insertion of self-expanding stents (for example) comprising an inner guide channel member 70, an outer guide channel member 80 axially slideable relative to the inner member 70, a self-expanding deployment device mounting region 90 (e.g., a stent mounting region), and a transition region 60. As used in connection with describing embodiments of the inner and outer guide channel members 70, 80, respectively, the term "guide channel" is understood to be any aperture, bore, cavity, chamber, channel, duct, flow passage, lumen, opening, orifice, or passageway that facilitates the conveyance, evacuation, flow, movement, passage, regulation, or ventilation of fluids, gases, or a diagnostic, monitoring, scope, catheter, other instrument, or more particularly a wire guide (Fig. 6) or another component of the distal end portion (e.g., an inner member 70 relative to the outer member channel 81).

The distal portion 13, according to the delivery system 10 and shown in Figures 4, 5, 6, and 7, may be made of any suitable material (natural, synthetic, plastic, rubber, metal, or combination thereof) that is rigid, strong, and resilient, although it should be understood that the material may also be pliable, elastic, and flexible. By way of illustration only and not by way of limitation, the distal end portion may comprise one or a combination of the following materials: metals and alloys such as nickel-titanium alloy ("nitinol") or medical grade stainless steel, and/or plastic and polymers such as polyether ether-ketone ("PEEK"), polytetrafluoroethylene (PTFE), nylon and/or a polyether block amide ("PEBA"), polyimide, polyurethane, cellulose acetate, cellulose nitrate, silicone, polyethylene terephthalate ("PET"), polyamide, polyester, polyorthoester, polyanhydride, polyether sulfone, polycarbonate, polypropylene, high molecular weight polyethylene, polytetrafluoroethylene, or mixtures or copolymers thereof, polylactic acid, polyglycolic acid or copolymers thereof, polycaprolactone, polyhydroxyalkanoate, polyhydroxy-butyrate valerate, polyhydroxy-butyrate valerate, or another polymer or suitable material. Where it will not contact the patient (e.g., it is contained within a sheath, working channel of an endoscope, or an external accessory channel device used with an endoscope), the middle section delivery device 14 and distal portion 13 do not need to be biocompatible. In contrast, where there is the possibility of patient contact, the material may need to be biocompatible or capable of being made biocompatible, such as by coating, chemical treatment, or the like.

The inner and outer guide channel members 70, 80, respectively, may be made of any suitable material described above for use with the distal portion 13. In one embodiment, the inner guide channel member 70 and the outer guide channel member 80 comprise PEEK material, which has the advantage of softening under heat before burning or degrading. PEEK tubing may be purchased from many suppliers, such as Zeus, Inc. in Orangeburg, South Carolina for instance.

Beginning with the inner guide channel member 70, a description will follow relating to features common to embodiments of a distal portion 13 of a delivery system 10 for the rapid insertion of "stents" according to the invention. The inner guide channel member 70 is generally tubular and comprises a first end portion 78 and a second end portion 77 defining a wire guide channel 71 therebetween. Optionally, the inner guide channel member 70 is configured to be slidably nested, fitted, secured, or otherwise positioned within the outer guide channel member 80 such that at least one of the inner guide channel member first or second end portions 78, 77, respectively, is axially intermediate an outer guide channel member first end portion 88 and an outer guide channel member second end portion 87.

The first end portion 78 of the inner guide channel member 70 further comprises a wire guide entry port 72, and the second end portion 77 has a wire guide exit port 73. The entry and exit ports 72, 73, respectively, define and are in communication via the wire guide channel 71. A port, in describing an embodiment of an inner guide channel member 70 and an outer guide channel member 80 according to the invention, includes any structure that functions as an entry or exit aperture, cutout, gap, hole, opening, orifice, passage, passageway, port, or portal. The inner guide channel member entry port 72 is sized to receive a wire guide into the inner member guide channel 71, and the inner guide channel member 70 is configured so that the wire guide may exit proximally out the inner guide channel member exit port 73. Optionally, the exit port 73 is located at or near the transition region 60. In one embodiment of the present invention, the inner member 70 is a cannula (or catheter) having an entry port 72 and an exit port 73 as previously described and defining a guide channel 71 therebetween.

The inner guide channel member 70 further comprises an outer self-expanding deployment device mounting region 90 (e.g., an outer stent mounting region) positioned intermediate the inner guide channel member entry and exit ports 72, 73, respectively. The length of the inner guide channel member 70 of any of the embodiments of the present invention may vary generally from about 10.0 to about 40.0 cm. In one alternative embodiment, the length of the inner guide channel member 70 is approximately 15.0 to approximately 25.0 cm. In another embodiment, the length of the inner guide channel member 70 is approximately 20.0 cm. Also, the length of the inner guide channel member 70 may depend on the intended stent, and in another embodiment the length of the inner guide channel member 70 is approximately 15.0 cm for an 8.0 cm stent.

The inner guide channel member 70 further comprises inner and outer diameters. In one embodiment, both diameters are substantially uniform over the entire length of the inner guide channel member 70. By way of example, an internal diameter 74 might measure approximately 0.0205 inches at or near the inner guide channel member proximal second end portion 77, at or near the inner guide channel member distal first end portion 78, and intermediate the first and second end portions 78, 77, respectively. Likewise, an inner guide channel member 70 might have an outer diameter 75 that measures approximalely 0,109 cm (0.0430 inches). Thus, the outer diameter 75 might measure approximately 0,109 cm (0.0430 inches) at or near the inner guide channel member proximal second end portion 77, at or near the inner guide channel member distal first end portion 78, and intermediate the first and second end portions 78, 77.

In an alternative embodiment to an inner guide channel member 70 having a substantially uniform outer diameter 75 along its length from about the second end portion 77 to about the first end portion 78, the inner guide channel member may also comprise a tapered outer diameter 76. In one embodiment, the inner guide channel member tapers distally to a second outer diameter 76' at or near the inner guide channel member first end portion 78 or intermediate the inner guide channel member first and second end portions 78, 77, respectively. The taper 76 has a decreased cross section, diameter, width, height, area, volume, thickness, and/or other configuration, shape, form, profile, structure, external outline, and/or contour relative to the outer diameter 75. In other words, the inner guide channel member second outer diameter 76' is smaller in cross section, diameter, width, height, area, volume, thickness, and/or other configuration, shape, form, profile, structure, external outline, and/or contour than the outer diameter 75.

Figure 4 further shows an optional atraumatic tip 170 coupled to the inner guide channel member first end portion 78. Extending distally from the inner guide channel member first end portion 78, the atraumatic tip 170 is tapered, rounded, chamfered, or arrowhead shape to be better tolerated by the patient. The atraumatic tip 170 comprises a distal first end portion 178 with a wire guide entry port 172 and a proximal second end portion 177 with a wire guide exit port 173, whereby the entry and exit ports define an atraumatic tip guide channel 171. The ports 172, 173 and channel 171 are sized to slideably receive a wire guide.

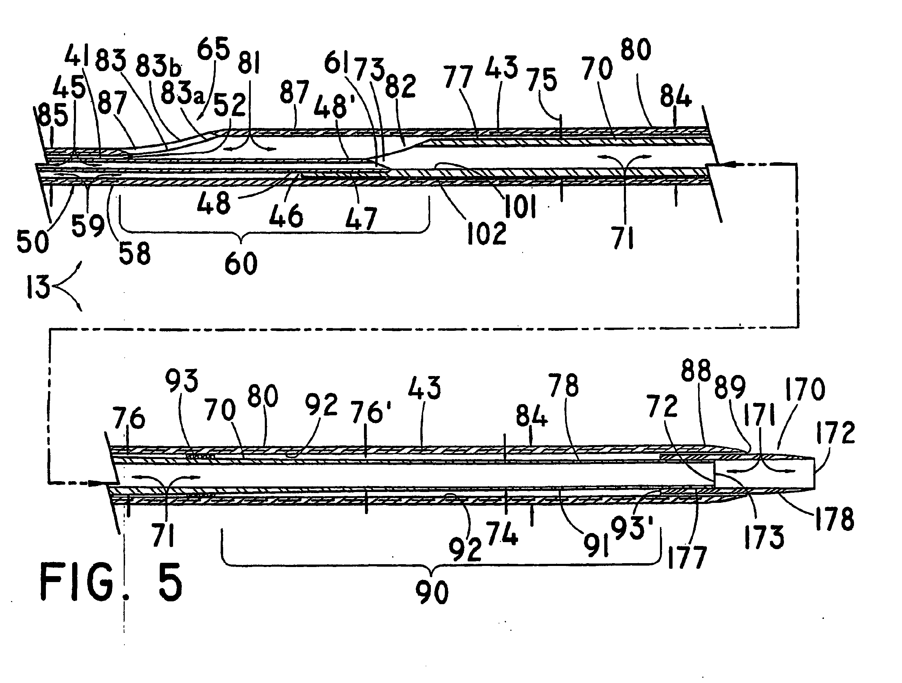

The atraumatic tip second end portion 177, as shown in Figure 4, may abut the outer guide channel member distal end portion 88 and, thereby, extend entirely distally beyond a distal opening 89 of the outer guide channel member first end portion 88. Optionally, the outer guide channel member distal opening 89 is spaced from the atraumatic tip 170 sufficient to allow delivery system to be flushed with saline that exits the distal portion to remove air in order to help keep air out of the patient, as explained above. In the alternative and as shown in Figure 5, the atraumatic tip 170 may be configured to have a second end portion 177 that is beveled such that the atraumatic tip second end portion 172 is partially positioned within the outer member guide channel 81 and partially proximal to the outer guide channel member distal opening 89. The beveled design of the atraumatic tip second end portion 177 forms a proximal stop against the outer guide channel member distal opening 89 while permitting the atraumatic tip second end portion 177 to be partially slidably nested, fitted, secured, or otherwise positioned within the outer guide channel member first end portion 88 so that the outer guide channel member first end portion 88 overlaps the atraumatic tip 170 to form a suitable seal that substantially occludes passage of a wire guide between the atraumatic tip 170 and the distal opening 89 of the outer member first end portion 88 (Fig. 5). Furthermore, the atraumatic tip second end portion 177 comprises a stent distal restraint 93' as explained below.

In Figure 4, the outer guide channel member 80 also is generally tubular and comprises a first end portion 88 and a second end portion 87. The outer guide channel member 80 further comprises a wire guide entry port 82 proximal to the first end portion 88 and a proximal wire guide exit port 83 located at or near the second end portion 87. The entry and exit ports, 82, 83, respectively, define a guide channel 81 of the outer guide channel member 80, wherein the ports 82, 83 and channel 81 are sized to slideably receive a wire guide. The entry port 82 is configured to receive a wire guide into the outer member guide channel 81, and in one embodiment, the entry port 82 is defined by the inner guide channel member exit port 73. In that embodiment, the wire guide moves proximally through the inner member guide channel 71 and egresses from the inner guide channel member exit port 73, wherein the proximal passage of the inner guide channel member exit port 73 is designated as the outer guide channel member wire guide entry port 82. The outer guide channel member proximal wire guide exit port 83 is configured so that a wire guide may egress proximally out the outer member exit port 83. In one embodiment, the outer guide channel member distal opening 89 and exit port 83 define the guide channel 81 therebetween.

In one embodiment, the Flexor® sheath, manufactured and sold by Cook Incorporated of Bloomington, Indiana, may be adapted for use with the distal portion 13 and/or the middle section delivery device 14. Otherwise stated, the Flexor® sheath, as shown in Figure 3 and described above, may be provided for the distal portion 13 and/or the middle section delivery device 14. For instance, the distal portion 13 may be constructed as comprising an integral Flexor® sheath tube with the middle section delivery device 14. Alternatively, a Flexor® tubing may be used for either the middle section delivery device 14 or the distal portion 13, or both. Then, the separable middle section delivery device 14 and distal portion 13 may be attached, adjoined, joined, or combined as taught herein below and/or in the United States Provisional Patent Application filed on April 20, 2005 entitled, "Delivery System and Devices for the Rapid Insertion of Self-Expanding Devices" and having a US Publication number 2006-0259117, the United States Provisional Patent Application filed on January 23, 2006 entitled, "Melt-Bonded Joint for Joining Sheaths Used in Medical Devices, and Methods of Forming the Melt-Bonded Joint" and having a US publication number US2006-0264906.

The Flexor® sheath has a PTFE inner lining 44 that provides a slick, smooth surface for sliding the outer sheath 50 and/or the outer guide channel member 80 proximally. With regard to the distal portion 13, the outer guide channel member 80 slides relative to the inner guide channel member 70, and the outer guide channel member inner surface 92 would be the inner layer 44 described above, thereby resulting in minimal friction to a stent 17 on the stent platform 91. The slideable inner surface 92 of the Flexor® sheath exhibits a second benefit of minimizing damage or misalignment to the stent. Indeed, because self-expanding stents continuously exert an expanding force against the inside surface 92 of the outer guide channel member 80, any substantial friction or drag between the stent and the inner surface 92 of the outer guide channel member 80 as the outer guide channel member 80 withdraws may damage the stent or cause the stent to be deployed slightly off of the target site.

The thin flat wire reinforcing coil 43 of the Flexor® sheath provides the outer guide channel member 80 with the necessary radial strength to constrain the stent over long periods of storage time. In contrast, where the inner surface 92 of an outer guide channel member 80 does not comprise the Flexor® sheath inner layer 44 or equivalent, the stent over time may tend to become imbedded in the inner surface 92 and, as a result, interfere with retraction of the outer guide channel member 80 at the time of deployment. In an outer guide channel member 80 that comprises a Flexor® sheath, in addition to the inner layer 44 and the reinforcing coil 43, the outer guide channel member 80 has a Flexor® sheath outer layer 42. The outer layer 42 comprises nylon and/or PEBA to provide the necessary stiffness for pushability, retraction, and control of the outer member 80 to facilitate proper deployment of the constrained self-expanding stent. Therefore, the Flexor® sheath is one non-limiting example of an embodiment of an outer sheath 50 and/or an outer guide channel member 80.

While Figure 4 shows an outer guide channel member 80 having the exit port 83 proximal to the entry port 82 in one embodiment of the outer guide channel member 80, the relative axial distances between the entry and exit ports 82, 83, respectively, vary when the outer guide channel member 80 is in a non-deployed state versus a deployed state, because the outer guide channel member 80 moves axially relative to the inner guide channel member 70. Otherwise stated, Figure 4 shows an embodiment where the exit port 83 is proximal to the entry port 82 in either a non-deployed stent position or in a deployed stent position. In a non-deployed stent position of another embodiment, however, the exit port 83 may be substantially co-plantar to or aligned with the entry port 82. In the fully deployed stent position, the exit port 83 may likewise be proximal, co-planar, or aligned with the entry port 82. Optionally, the entry port 82 and exit port 83 are located at or near the transition region 60 to be discussed below.

Furthermore, the outer guide channel member 80 has a stepped 84, 85 profile, whereby the outer guide channel member 80 comprises a first outer diameter 84 intermediate the outer guide channel member first and second end portions 88, 87, respectively, and a second smaller outer diameter 85 located at or near the outer guide channel member second end portion 87 in the vicinity of the transition region 60 and the breech position opening 65. The stepped 84, 85 profile includes an embodiment where the outer guide channel member 80 transitions to the distal end portion 58 of the outer sheath 50 of the middle section delivery device 14. In describing embodiments of the invention, however, the stepped 84, 85 profile shall be discussed in reference to the outer guide channel member 80 in particular, but it should be understood as including a stepped 84, 85 profile in reference to the transition region 60 of the distal portion 13 relative to the middle section delivery device 14 where the middle section delivery device 14 and distal portion 13 are formed from separate units such as, by way of example only and not by way of limitation, separate "Flexor®" sheaths where one comprises a first outer diameter 84 and the other comprises a second smaller outer diameter 85.

As shown in Figure 4, the second smaller outer diameter 85 of the outer guide channel member 80 is located proximal to the larger first outer diameter 84 and, thereby, comprises a stepped 84,85 profile. Having a second smaller outer diameter 85 reduces the profile of the outer guide channel member 80 and/or the outer guide channel member 80 transition to the middle section delivery device 14, which is advantageous in procedures involving narrow vessel passageways, endoscope working channels, or accessory channels for use with endoscopes. The difference in the first diameter 84 and the second diameter 85 may vary. By way of illustration, the second diameter 85 may be approximately one-fourth to approximately nine-tenths that of the first diameter 84. In another embodiment, the second diameter 85 may be about one-half that of the first diameter 84. In another embodiment, the first diameter 84 is roughly 5 French while the second diameter 85 is roughly 4 French.

In one embodiment of the stepped 84, 85 profile of the outer guide channel member 80, the second smaller outer diameter 85 is located at or near the outer guide channel member second end portion 87. The second end portion 87 may decrease precipitously from the first outer diameter 84 to the second smaller diameter 85. In a precipitous step, the change from the diameters occurs over a short length along the longitudinal axis of the distal portion 13. In a further example of a precipitous step, the plane formed by the exit port 83 may be substantially perpendicular to the longitudinal axis of the outer guide channel member 80. In an alternative embodiment, the second end portion 87 may decrease gradually from the first outer diameter 84 to the second smaller diameter 85. In a gradual step, the change from the two diameters occurs over a length of more than 1.0 millimeter ("mm") along the longitudinal axis of the distal portion 13 at or near the transition region 60 and breech position opening 65, and in one instance this change occurs over a length from about 1.0 mm to about 10.0 mm. In a further example of a gradual step, the plane formed by the exit port 83 may be at an angle other than 90 degrees relative to the longitudinal axis of the distal portion 13.

Figure 4 also shows a breech position opening 65 located at or near the second end portion 87 of the outer guide channel member 80 comprising the wire guide exit port 83. In other words, rather than the exit port 83 being an aperture in a lateral sidewall of the outer guide channel members 80 intermediate the first and second end portions 88, 87, respectively, in a breech position opening 65 embodiment the exit port 83 is at the rear, back, or proximal part of the distal portion 13 at or near the outer member second end portion 87 and the stepped 84, 85 profile such that it opens in the direction of the outer surface of the outer sheath 50.

The breech position opening 65 may be used for front-loading and the more common procedure of back-loading a wire guide (or catheter, for instance). In a back-loading procedure for a delivery system having a breech position opening 65, the wire guide may pass proximally through the guide channel 71 of the inner guide channel member 70, proximally through the guide channel 81 of the outer guide channel member 80, and leave the exit port 83 of the second end portion 87 of the outer guide channel member 80 from a breech position opening 65 in a rear, back, or proximal part of the distal portion 13. Conversely, in a front-loading procedure for a delivery system having a breech position opening 65, the physician may feed the wire guide distally into a breech position opening 65 at the rear, back, or proximal part of the distal portion 13 by entering the exit port 83 of the second end portion 87 and the guide channel 81 of the outer guide channel member 80 and through the guide channel 71 of the inner guide channel member 70, where the wire guide may exit from the wire guide entry port 72 of the inner guide channel member 70 and/or wire guide entry port 172 of the atraumatic tip 170.

In a distal portion 13 having a breech position opening 65 that comprises an exit port 83 located at a breech position of the transition region 60 according to the invention, the wire guide does not need to make any sharp turns away from the longitudinal axis of the distal portion 13 that may result in kinking of the wire guide. The breech position opening 65-comprising an exit port 83 according to embodiments of the invention, as those shown in Figures 4, 5, 6, and 7 by way of example and not by way of limitation-is located proximal to the inner guide channel member second end portion 77 and may be transverse or angled relative to the tubular distal portion 13 longitudinal axis. In other words, the wire guide exit port 83 may be positioned at or near a breech position opening 65 of the distal portion 13, wherein the exit port 83 is located at or near the rear, back, or proximal part of the outer guide channel member 80 and/or second end portion 87, rather than being positioned exclusively on the side (e.g., outer circumferential cylinder wall) of the outer guide channel member 80.

In Figure 4, the breech position opening 65 comprises an exit port 83 that is illustrated as being oblique, although other configurations of the exit port may be utilized to aid the wire guide in exiting the rear of the outer member. In one example, the exit port 83 may form a plane substantially perpendicular to the longitudinal axis of the outer guide channel member second end portion 87. In another example, the plane formed by the exit port 83 may be at an angle other than 90 degrees relative to the longitudinal axis of the distal portion 13. Optionally, the oblique exit port 83 of a breech position opening 65 has lateral walls 83a, 83b that act as guide rails to direct a wire guide proximally toward the middle section delivery device 14 and to run along the outside of the outer sheath 50.

The overall axial length of the exit port 83 of the breech position opening 65 may vary. In one embodiment, the length is approximately from about 1.0 mm to about 10.0 mm. Another embodiment has a length of approximately 5.0 mm. The overall width of the exit port 83 may also vary. In one example, the width of the exit port is approximately 1 French. In yet another instance, the width of the exit port 83 ranges from about 1 French to about 4 French. In another example, the width of the exit port 83 may be the approximate difference between the first outer diameter 84 and the second outer diameter 85 of the outer guide channel member 80.

At the transition region 60, the exit port 73 of the inner guide - channel member 70 is in communication with the outer guide channel member 80 wire guide entry port 82, while the second end portion 77 is operatively coupled to the distal mating end portion 48 of the inner guide channel member 70 as explained below. The length of the transition region 60 may vary. For instance, the transition region 60 may be approximately from about 0.5 cm to about 10.0 cm. In another embodiment, the transition region 60 has the approximate length of about 5.0 cm. Furthermore, the length of the transition region 60 is variable: from a shorter axial length when the outer guide channel member 80 is in a non-deployed axial position; to a greater axial length when the outer guide channel member 80 retracts proximally to deploy the stent. Likewise, the overall length of the transition region 60 varies in the embodiment where the exit port 83 is distal to the entry port 82 when the outer guide channel member 80 is in a non-deployed stent position, compared to the initial length of the transition region 60 in an embodiment where the exit port 83 is proximal to the entry port 82 when the outer guide channel member 80 is in a non-deployed stent position.

In one use of the transition region 60 according to an embodiment of the invention, the outer guide channel member entry port 82 receives a wire guide from the inner guide channel member exit port 73 and the wire guide thereby is received in the outer member guide channel 81. At the transition region 60, the inner member guide channel 71 and outer member guide channel 81 are approximately aligned relatively coaxially in one embodiment. Approximate alignment of the guide channels 71, 81 facilitates a smooth transition of the wire guide. Smooth transition optimally reduces any bending of the wire guide as the wire guide moves proximally from the inner member guide channel 71 to the outer member guide channel 81.

As shown in Figure 4, the distal portion 13 also comprises a self-expanding deployment device mounting region 90. This mounting region 90 may be used for implantable prosthesis such as expandable (self-expanding, balloon expandable, or otherwise expanding) and nonexpanding stents, prosthetic valve devices, and other implantable articles for placement inside a patient's body (the implantable prostheses being referred to individually and collectively as "stents" without limiting the invention) and therefore may be referred to as a stent mounting region to include the foregoing implantable prostheses.

The stent mounting region 90 comprises a stent platform 91 on an outside surface of the inner guide channel member 70 located at or near the inner guide channel member second end portion 78. In describing embodiments of the invention, the platform 91 "at or near" the inner guide channel member second end portion 78 includes a region intermediate the inner guide channel member entry port 72 and the inner guide channel member exit port 73. The platform 91 may be any stent mounting surface, including but not limited to the outside surface of the inner guide channel member 70, a recess, or an indentation located at or near the first end portion 78 of the inner guide channel member 70. In a non-deployed state, a self expanding stent for example (not shown) compresses against the stent platform 91 and disposes around the outside of the inner guide channel member 70.

The stent mounting region 90 controls the lateral movement (e.g., transverse expansion away from the inner guide channel member longitudinal axis) to avoid premature deployment of the stent. In order to control the lateral movement of the stent, the stent is sandwiched between the platform 91 on the inner surface of the stent and the inner surface 92 of the outer guide channel member 80 to keep the stent in a compressed state. Because the stent is bound from above by the inner surface 92 of the outer guide channel member 80 and bound from below by the platform 91 of the inner guide channel member 70, the stent mounting region 90 maintains the stent in a substantially compressed state and controls premature deployment of the stent.

In addition to controlling a stent's lateral movement, the stent mounting region 90 restrains the axial movement of a stent to control the stent movement away from the target site. A proximal restraint 93 controls proximal axial movement of the stent. In one embodiment, the proximal restraint 93 is sized to be large enough to make sufficient contact with the loaded proximal end portion of the stent without making frictional contact with the inner surface 92 of the outer guide channel member 80. In addition to helping to stop the stent's proximal movement in the non-deployed state, this restraint 93 assists with "pushing" the stent out of the distal portion 13 by helping to prevent the inner guide channel member 70 and/or the stent disposed on the stent mounting region 90 from migrating proximally when the outer guide channel member 80 retracts proximally relative to the stationary inner guide channel member 70 in order to expose and deploy the stent. Optionally, the restraint 93 may be radiopaque so as to aid in stent positioning within the vessel passageway at or near the target site within a patient. In one embodiment, an optional distal restraint 93' is large enough to make sufficient contact with the loaded distal end portion of the stent to control axially distal movement of the stent. Similarly, in another embodiment the proximal second end portion 177 of an optional atraumatic tip 170 controls the stent's distal axial movement. Indeed, because the medical device delivery system may be used for deploying an implantable prosthesis that comprises balloon expandable or non-expanding stents, prosthetic valve devices, and other implantable articles at a selected location inside a patient's body, the proximal restraint 93 and distal restraint 93' control the axial distal movement of the implantable prosthesis. Optionally, the distal restraint 93' and/or atraumatic tip 170 may comprise radiopaque materials so as to aid in stent positioning within the vessel passageway at or near the target site within a patient.

Figure 4 also illustrates that the inner compression member 41 and inner guide channel member second end portion 77 may be operatively coupled by any suitable means. In one embodiment, a melt bond 47 (described below) operatively couples an inner compression member distal mating end portion 48 ("mating end 48" or "mating end portion 48") and the second end portion 77 of the inner guide channel member 70. A melt bond 47 provides surface-to-surface contact between an outer engaging surface 48' of the distal mating end portion 48 and the inner guide channel second end portion 77, thereby forming a more solid connection between the inner compression member 41 and the inner guide channel member 70.

In one embodiment, the inner compression member outer engaging surface 48' may form a melt bond 47 to an inner surface 101 of the inner guide channel member second end 77. Alternatively, the inner compression member outer engaging surface 48' may form a melt bond 47 to the outer surface 102 of the inner guide channel second end 77. In yet another embodiment, the distal mating end 48 of a solid inner compression member 41 as shown in Figure 4 is implanted 49 between (and/or melt bonded 47 between) inner and outer surfaces 101, 102, respectively, of the inner guide channel member second end 77, as taught in United States Provisional Patent Apptication Number 2006/0263456 filed on January 23, 2006 entitled, "Internal Joint for Medical Devices, and Methods of Making the Internal Joint," and having an application serial number 60/761,313, and the non-provisional application filed on April 20, 2006 by the same title and claiming the benefit of the filing date application serial number 60/761,313 under 35 U. S. C. §119(e).

As used to describe an embodiment of the invention, melt bonding 47 (for shorthand purposes in describing embodiments according to the invention, melt bonding 47 includes implanting 49) comprises any suitable means for melting, liquefying, softening, making semi-molten, molten, fusing, or making malleable, pliant, supple, moldable, ductile, or otherwise penetrable by another component or fused to melt bonding material comprising the other element. For instance, melt bonding 47 involves bringing two components together at an interface, wherein one (or preferably both) of the component interfaces are in the melted state. Strictly speaking, true melt bonding 47 requires that both of the components be melted at the interface and that they may be sufficiently chemically and physically compatible such that they fuse together upon cooling.

The melt bonding materials comprising the two components may be the same or substantially same materials. In the alternative, the melt bonding materials may be different, so long as they have substantially similar melting points at standard atmospheric pressure such that the materials soften (or liquefy) under heat and thereby fuse together in a solid state melt bond 47 joining the first and second melt bonding materials of the components. If the materials had melting points that were too different, then one material may degrade or burn and the like before the second material begins to melt.

Melt bonding 47 may be single layer interface whereby one component interface/surface mates to a second component interface/surface, or may be multi-layer interface whereby one component is implanted 49 into a second component and then surrounded by the second component. The chemical compatibility can best be expressed in terms of having similar values for surface energy and/or solubility parameter. In simple terms, similar materials tend to have a mutual affinity and a greater propensity to adhere to one another than do dissimilar materials. Melt bonding includes bonding whereby one component is melted while the other component is at or above its melting point.

Melt bonding materials may have different "melt bonding" temperatures at which they soften and become almost tacky without substantial degradation. Melt bonding materials are available from vendors, including Zeus, Inc. in Orangeburg, South Carolina for instance; Cobalt Polymers in Cloverdale, California; and under the trade name of Pebax® PEBA from the Arkema Group. The melt bonding materials may include one or a combination of a class of suitable materials comprising nylon, nylon natural tubing, polyether block amide, polyetheretherketone, thermoplastic, acrylonitrile-butadiene-styrene copolymer, polypropylene, polyamide, ionomer, polycarbonate, polyphenylene oxide, polyphenylene sulphide, acrylic, liquid crystal polymer, polyolefin, polyethylene acrylate acid, polyvinylidene fluoride, polyvinyl, and polyvinyl chloride.

In one embodiment, PEEK material is used for the melt bonding material. PEEK melts at about 334°C (633°F), so the material may be heated from about 331°C (628°F) to about 336°C (638°F). For instance, a radiofrequency loop heater may be used for heating the melt bonding materials. Such a machine is available from Magnaforce, Incorporated and sold under the name and model Heatstation 1500. Another such machine is available from Cath-Tip, Inc. and is sold under the model and name Cath-Tip II. There is a rise dwell and cool down time for the process. The total rise time is approximately 20 seconds and dwell time is approximately 10 seconds. During the dwell time the temperature is approximately 316°C (600°F). In one embodiment where nylon or PEBA are used, heating is at about 204°C (400°F), with dwell time of about 10 seconds.

Figures 4A and 4B present a schematic representation of a cross section 105 of components before and after melt bonding. The cross section 105 of Figure 4A, for example, represents an inner component 106, a middle component 107, and an outer component 108. While all components are shown having interfaces in abutting physical contact, they need only be close enough to form a melt bond therebetween. Indeed, as previously explained in connection with the Flexor® sheath's outer layer 42 and inner layer 44, there may even be a middle layer comprising a coil 43 having spacings 43' through which the melt bonding material of the outer layer 42 may move to be into contact with the inner layer 44.

In the example represented in Figure 4A, the middle and outer components 107, 108, respectively, are intended to be melt bonded. The middle component 107 comprises a first melt bonding material 109 while the outer component 108 comprises a second melt bonding material 109', which may be the same material or may be separate materials that have similar melting points at atmospheric pressure.

Figure 4B shows some of the first melt bonding material 109 of the middle component 107 moving into some of the second melt bonding material 109' of the outer component 108. Likewise, some of the second melt bonding material 109' of the outer component 108 moves into some of the first melt bonding material 109 of the middle component 107. It should be noted that both of the first and second materials 109, 109' need not move into the other. Rather, the first and second materials 109, 109' need only bond at an interface, with or without mixing and the like. By way of example, the Flexor® sheath's outer layer 42 may melt to the middle layer coil 43 (which has not melted) and bond to the outer surface of the inner layer 44 with or without the outer surface of the inner layer 44 melting into the outer layer 42.

Figure 4B further shows that the first and second melt bonding materials 109, 109', respectively, of the middle component 107 and the outer component 108 or other components that have been melt bonded, upon cooling to solid state will form a melt bond 47 operatively coupling the components and/or the melt bonding materials that comprise the components. This results in additional strength and helps to form a more solid connection to the melt bonded components, because a solid-state bond results from using a suitable form of heat for melting and solidifying (e.g., fusing and/or cross-linking bonds formed at the melt bonded material interfaces).

Figure 5 illustrates a schematic view showing an alternative embodiment of a distal portion 13 of a delivery system for the rapid insertion of stents comprising an inner guide channel member 70, an outer guide channel member 80 axially slideable relative to the inner member 70, a deployment device mounting region 90 (e.g., a stent mounting region), and a transition region 60. Like elements from the previous drawings, embodiments, and description from above are labeled the same. In this embodiment, the inner compression member 41 optionally comprises a passageway 45 (e.g., hollow, having a lumen) that facilitates the conveyance, ventilation, flow, movement, blockage, evacuation, or regulation of medication and/or fluids or accommodates the insertion of a diagnostic, monitoring, scope, or other instrument.

The tubular inner compression member 41 may have a uniform inside diameter ranging from about 0.1339cm to about 0.3353cm (0.0527 to about 0.132 inches). The wall thickness of the tubular inner compression member 41 is approximately 0.0038cm (0.0015 inch). These dimensions are illustrative only, and the inner diameter and wall thickness may be constructed to be of any size necessary to accomplish the purposes for which the delivery system is to be employed (i.e., limited by the vessel passageway or working channel in which the device is to be used).

In addition, this inner compression member 41 has an optional distal one-way valve 61. Thus, the valve 61 may serve a dual function. First, a one-way valve is relatively resistant to contamination from bodily fluids entering the inner compression member passageway 45. Second, it allows the movement of medication and/or fluids to exit distally the inner compression member 41 passageway 45 at or near the transition region 60 and may direct medication and/or fluids into the inner member guide channel 71 and/or the outer member guide channel 81.

Indeed, the inner compression member passageway 45 may facilitate using the medical device delivery system for deploying an implantable prosthesis that comprise balloon expandable stents, prosthetic valve devices, and other implantable articles (individually and collectively, "stent") at a selected location inside a patient's body. The stent is disposed at the deployment device mounting region 90 intermediate the proximal restraint 93 and distal restraint 93' to control the axial distal movement of the implantable prosthesis.

In one embodiment for using the delivery system with a balloon expandable implantable prosthesis, the inner compression member distal mating end portion outer engaging surface 48' operatively couples to the inner guide channel member outer surface 102 (or is welded to an outer surface of a metal cannula that has the inner guide channel member second end portion 77 glued within the cannula lumen), and an inflation member (e.g., a balloon) extends distally from the inner compression member distal mating end portion and is disposed over the proximal restraint 93 and distally about the platform 91 of the stent mounting region 90 such that the balloon is located under the stent. The stent is positioned within the vessel passageway at or near the target site within a patient, wherein the outer sheath 50 and outer guide channel member 80 is axially slideable relative to the inner compression member 41 and inner guide channel member 70 upon corresponding axial slideable movement of the handle 30, thereby exposing and, ultimately, deploying the stent from the stent mounting region 90. The stylet 20 may be adapted to receive a syringe for allowing inflation fluid, such as saline, to travel from and through the proximal end 40 of the inner compression member 41 and out the valve 61 at the distal end portion 48 in order to fill the inflation chamber of the balloon. Therefore, balloon expands under the stent and, as a result, the stent expands radially to plastically deform the stent into a substantially permanent expanded condition. The physician then deflates the balloon and removes the inner guide channel member 70 and remainder of the delivery system from the patient's body. This description of using the delivery system for balloon expandable implantable prosthesis is given by way of example and not by way of limitation. Alternatively, a tubular inflation fluid carrying device is in the outer sheath passageway 59 and extends from the system proximal portion 12 to the system distal portion 13 and operatively couples to an inflation member disposed under the stent.

In one embodiment of the distal portion 13 of a delivery system illustrated in Figure 5, an internal joint 46 comprises a melt bond 47 that operatively couples the inner guide channel member distal mating end portion 48 and the second end portion 77 of the inner guide channel member 70. For example, the inner compression member outer engaging surface 48' may form a melt bond 47 to the inner surface 101 (or alternatively to the outer surface 102) of the inner guide channel member second end portion 77, as taught above.

The embodiment shown in Figure 5 also illustrates that the exit ports 83 and 73 may have various configurations. First, these exit ports curve, and second, compared to Figure 4 they slope over a longer overall axial length to aid the wire guide in exiting the inner member and outer member, respectively. Furthermore, the exit port 83 thereby has longer axial lateral walls 83a, 83b for acting as guide rails to direct a wire guide proximally toward the middle section 14 and to run along the outside of the outer sheath 50.

Moving to the atraumatic tip 170 as illustrated in Figure 5, this is a little less arrowhead-shaped compared to the atraumatic tip 170 shown in Figure 4. Instead, the atraumatic tip in Figure 5 has a second end portion 177 that comprises a right cylindrical tubular configuration. Furthermore, the sides of the atraumatic tip second end portion 177 are more uniformly parallel and do not form a proximal stop against the outer guide channel member distal opening 89 as in a beveled embodiment of the atraumatic tip second end portion 177 as illustrated in Figure 4. The atraumatic tip second end portion 177 optionally comprises a stent distal restraint 93' for controlling distal axial movement of the implantable prosthesis when the medical device delivery system is used for deploying balloon expandable or non-expanding stents, prosthetic valve devices, and other implantable articles at a selected location inside a patient's body.

Turning now to Figure 6, that figure shows a partially sectioned distal portion 13 in accordance with an embodiment of the device according to Figure 5 with a wire guide 16 inserted therein. In a back-loading procedure, the wire guide 16 enters the guide channel 171 of the atraumatic tip 170 and travels proximally toward the inner guide channel member 70. The wire guide 16 then enters the inner member guide channel 71 and travels proximally toward the outer guide channel member 80 via the entry port 82 and enters the outer member guide channel 81 and out the exit port 83. The less common front-loading procedure could be described as above but conversely stated.

In Figure 6, the inner and outer guide channels 71, 81, respectively, are substantially aligned coaxially along an approximate center longitudinal axis of the distal portion 13. Because the channels 71, 81 are substantially aligned, the wire guide 16 moves through the inner member guide channel 71 to the outer member guide channel 81 and out the outer guide channel member exit port 83 at or near the breech position opening 65 with relatively little kinking, bending, buckling, or bowing. It should be noted that for the ease of showing the wire guide 16, the wire guide 16 proximal to the exit port 83 is shown slightly offset from outer sheath 50, though the wire guide 16 may actually run along the outside of the outer sheath 50 or in a groove (not shown) in the outer sheath 50.

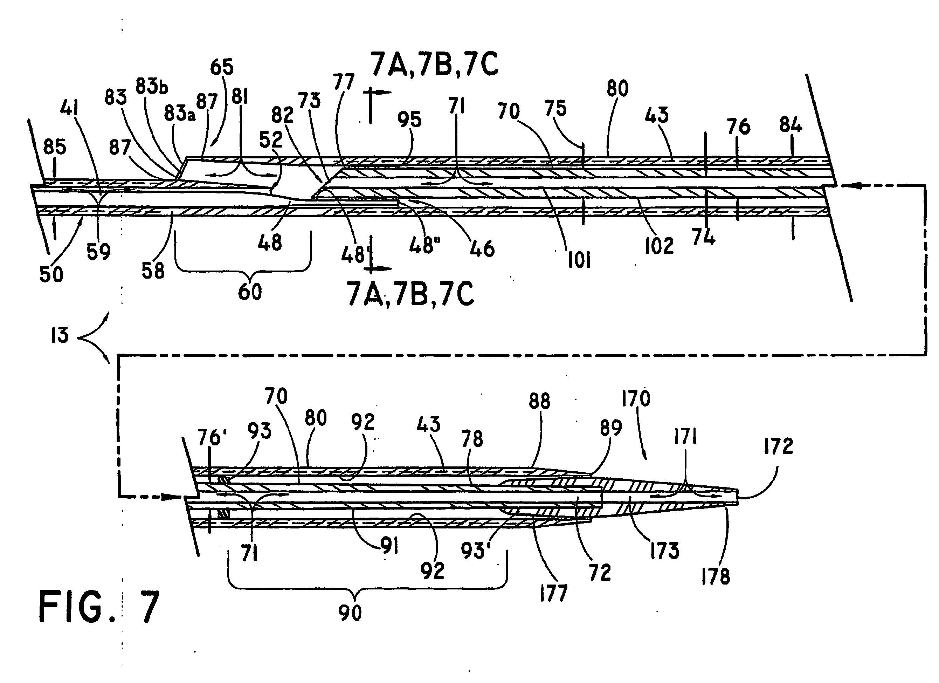

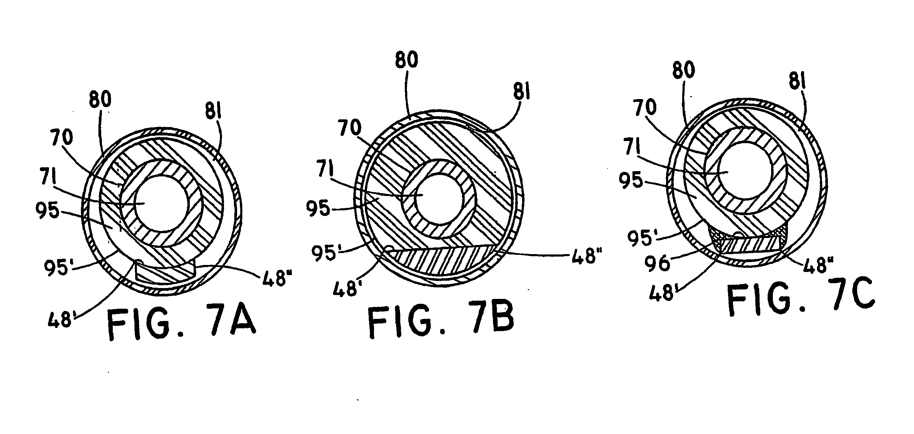

Figure 7 illustrates a longitudinally sectioned side view showing an alternative embodiment of a distal portion 13 of a delivery system for the rapid insertion of stents comprising an inner guide channel member 70, an outer guide channel member 80 axially slideable relative to the inner member 70, a deployment device mounting region 90 (e.g., a stent mounting region), and a transition region 60. Like elements from the previous drawings, embodiments, and description from above are labeled the same. This embodiment represents an alternative embodiment of a joint 46 for operatively coupling the inner compression member 41 and inner guide channel member 70 with a cannula 95.

In one embodiment, the cannula 95 is a hollow, rigid tube, cylinder, ring, cannula (with or without a trocar), or other coupling device comprising metal such as medical grade stainless steel or super-elastic alloys (e.g., nitinol) to name but a few non-limiting examples. In one embodiment, the cannula 95 comprises a generally right cylindrical configuration or is elliptical, hyperbolic, parabolic, curved, polygonal, rectangular, or irregular in shape or cross section. The cannula 95 is sized for receiving the inner guide channel second end portion 77 and/or the inner guide channel second end portion outer diameter 75. The outer surface 102 of the inner guide channel second end portion 77 is operatively coupled to an inner engaging surface of the securing body 95 by glue, adhesives, resins, chemical bonding materials or combinations thereof and the like (collectively and individually, "glue"). By way of example only, the glue may be Loctite 4061 instant adhesive, formulated to polymerise rapidly in thin films between two surfaces to form rigid thermoplastics. Loctite 4061 instant adhesive is a medical device adhesive particularly suitable for a wide variety of substrates such as rubber, plastics, and metals, ant it is available from the Loctite Corporation.

In addition to securing the outer surface 102 of the inner guide channel member second end portion 77 to an inner engaging surface of the cannula 95, the cannula 95 also operatively couples the inner guide channel member distal mating end portion 48. An outer engaging surface 48' of the mating end portion 48 is in an abutting relationship (e.g., touching, in contact directly or by intervening parts, or adjacent) to an outer engaging surface of the cannula 95, and the distal mating end portion 48 and cannula 95 are operatively coupled by any suitable means, including but not limiting to welding, soldering, brazing, or fusing. Soldering and brazing are used if a semi-permanent connection between the distal mating end portion 48 and the cannula 95 is desired, because solder or braze metals have a lower melting point than the metals that are joined. Thus, when sufficient heat is applied to melt the solder or braze metal, they form an alloy with the surfaces of the distal mating end portion 48 and the cannula 95 and, upon solidification, thus form a joint that can be unfastened during manufacturing (e.g., to redo in the event of a poor connection) by reheating without destroying the parts that have been joined. In contrast, welding involves melting the outer engaging surface 48' of the distal mating end portion 48 and an outer engaging surface of the cannula 95 at the interface, or involves combining temperature and pressure so as to cause localized coalescence. Consequently, in most instances higher temperatures are involved than for soldering, and the union is permanent.

Where the inner compression member distal mating end portion 48 and the cannula 95 are connected, an optional tube may be disposed abut the joint 46. The tubing has the advantage of minimizing some of the sharp edges created by a welded, soldered, or fused joint. In one embodiment, the tube is a melt bonding tube disposed about and melt bonded to the joint 46. Whereas Figure 7 shows the distal most tip of the distal mating end portion 48 flush with (e.g., substantially co-planar) the distal end portion of the cannula 95, it may alternatively be set back approximately 0.5 mm proximally from the distal end portion of the cannula 95. The set back arrangement allows solder, weld, or fusion to form a smooth transition and fill the space between that distal end tip and the cannula 95. This would also minimize the profile compared to placing more of a circumferential solder, weld, or fusion about the joint.

According to Figures 7, 7A, 7B, and 7C, the distal mating end portion 48 comprises a contoured configuration 48" that is complementary to an outer engaging surface 95' of the cannula 95. Thus, in an embodiment comprising a cannula 95 that is curved or otherwise circular in cross-section, then Figure 7A shows that the contoured configuration 48" is fluted so that the outer engaging surface 48' is capable of being in an abutting relationship (e.g., touching, in contact directly or by intervening parts, or adjacent) relative to a curved or circular outer engaging surface 95' of the cannula 95. A fluted contoured configuration 48" comprises any curved, shoehorn shape, celery shape, semicircular shape, crescent shape, wishbone shape, saddle shape, C-shaped, V-shaped, U-shaped, or other arcuate configuration. In another embodiment, an outer engaging surface 95' of the cannula 95 could have a flat portion, and Figure 7B shows that the contoured configuration 48" is likewise flat so that the outer engaging surface 48' is capable of being in an abutting relationship (e.g., touching, in contact directly or by intervening parts, or adjacent) relative to the flat portion of the outer engaging surface of the cannula 95. Even when the outer engaging surface 95' of the cannula 95 is curved or circular in cross section, however, Figure 7C shows that the inner compression member contoured configuration 48" could be flat, because the soldering, brazing, or fusing may fill in the space between the outer engaging surface 48' and a tangent that the flat configuration 48" forms to the curved portion of the outer surface 95' of the cannula 95. Similarly, if welding 96 is used, then the flat configuration 48" will form to a curved or circular outer engaging surface 95' of the cannula 95.

In addition, the contoured configuration 48" maintains low profile, high-strength, and flexibility of the connection between the inner compression member distal mating end portion 48 and the cannula 95. The contoured configuration 48" is in contrast to a rounded inner compression member distal mating end portion 48, which would have a greater diameter at the connection between the inner compression member distal mating end portion 48 and the cannula 95.

I n order to create the contoured configuration 48", the inner compression member distal mating end portion 48 may be formed, sheared, casted, or molded. By way of example only, forming can be done both hot and cold (except for stamping, which is always done cold) in order to modify the shape and/or physical properties of the material comprising the inner compression member distal mating end portion 48. Common forming processes include rolling the distal mating end portion 48 (between one or two rollers), stretching, forging, straight bending, and stamping.

Additional embodiments of the joint 46, cannula 95, and inner compression member distal mating end portion 48 comprising a contoured configuration 48" are described in the United States Patent Application filed on April 20, 2006 entitled, "Joint for Operatively Coupling a Contoured Inner Compression Member and an Inner Guide Channel Member for Medical Device Delivery Systems" and having a US publication number US 2006-02591173.

### INSERT BODY FOR INTERNAL JOINT

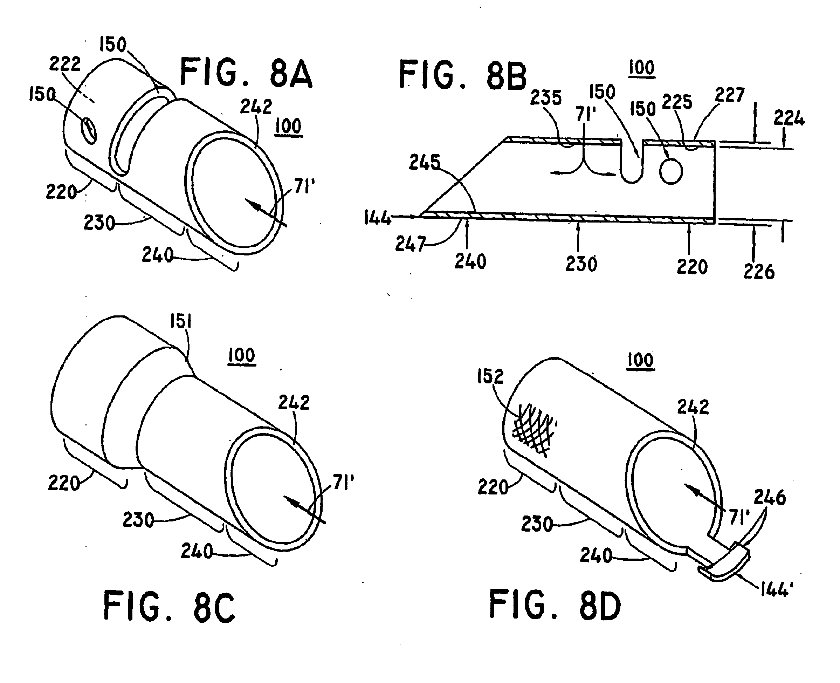

Figure 8A shows one embodiment of an insert body 100 for joining the inner compression member 41 and the inner guide channel member 70. Figure 8B is a longitudinal, sectional side view of Figure 8A.

In Figure 8A, the insert body 100 comprises a longitudinally dimensioned distal mating end portion 220 (insert mating end portion 220) having an entry port 222, a proximal connecting end portion 240 (insert connecting end portion 240) with an exit port 242, and an intermediate guide channel portion 230 (insert intermediate portion 230) longitudinally disposed between the insert mating end portion 220 and insert connecting end portion 240. The entry and exit ports 222, 242, respectively, define a lumen 71' through the longitudinally dimensioned insert mating end portion 220, intermediate guide channel portion 230, and insert connecting end portion 240. The insert body 100 may be substantially rigid, or in the alternative may be pliable, elastic, and flexible, and may be made of any suitable material (natural, synthetic, plastic, rubber, metal, or combination thereof) that is utilized for the system distal portion 13 or outer sheath 50 as described above.

The terms "longitudinal" and "longitudinally" in describing features of the insert body 100 should be considered to be an approximate lengthwise section, which may be straight or may at times even be curved because the insert body 100 and portions thereof may be flexible or partially flexible. Additionally, it should be understood that the insert body 100 may be one integral piece, such that the insert intermediate portion 230 describes the portion intermediate the insert mating end portion 220 and the insert connecting end portion 240. Alternatively, the insert mating end portion 220, insert intermediate portion 230, and/or insert connecting end portion 240 may be separate pieces joined together by any suitable means.

Figure 8B shows the insert mating end portion 220 having inner and outer diameters 224, 226, respectively, inner and outer surfaces 225, 227, respectively, and at least one securing portion 150. The securing portion 150 is configured for operatively (e.g., effectively, effective to produce) coupling the insert body 100 and the inner guide channel member 70, or for operatively coupling the insert body 100 and an outer sleeve 180 (discussed below), or for operatively coupling the insert body 100, the inner guide channel member 70, and the outer sleeve 180.

The terms "operatively coupling," "operatively coupled," "coupling," "coupled," and variants thereof are not used lexicographically but instead are used to describe embodiments of the invention as explained above. More particularly, the securing portion 150 may operatively couple the insert mating end portion 220 to one or more other components by direct contact, such as with a wedge effect, a press-fit-tight configuration, a surface roughness (e.g., sandblasting, etching, knurling, grinding, threading, milling, drilling, chemical treatment, or other roughing preparation), a tongue and groove joint, interlocking protrusion and indentation, and/or an internal screw thread and external screw thread. Alternatively or in addition to direct contact, the securing portion 150 may operatively couple the insert mating end portion 220 to one or more other components by utilizing a melt bond, glue, adhesives, resins, welding (laser, spot, etc.), soldering, brazing, adhesives, chemical bonding materials or combinations thereof. Additional non-limiting examples of embodiments of a securing portion 150 for operatively coupling the insert body 100 to the inner member 70 and/or the outer sleeve 180 are described below.

In one embodiment, the insert mating end portion 220 of the insert body 100 is substantially tubular. Alternatively, the insert mating end portion 220 is substantially annular (e.g., circumferential, circular, cylindrical, rounded, oblong, and the like). Optionally, the insert mating end portion 220 has a substantially solid perimeter or circumference between the inner and outer surfaces 225, 227, respectively. The insert mating end portion 220 may also comprise, however, an open structure, such as by way of example only and not by way of limitation open spaces and interstices formed by a framework of wires, cords, threads, woven strands, wire screen or mesh of suitable materials (natural, synthetic, plastic, rubber, metal, or combination thereof). Also, the securing portion 150 optionally may extend like an aperture (e.g., a slot or perforation) from the insert mating end portion inner surface 225 to the insert mating end portion outer surface 227 and there through.

The insert intermediate portion 230 is a structure configured to allow a wire guide, catheter, medical device, or tool to pass from the insert mating end portion 220 to the exit port 242 of the insert connecting end portion 240, and comprises an inner surface 235 defining the section of the insert body lumen 71' along the longitudinally dimensioned insert intermediate portion 230. By way of example, the insert intermediate portion 230 may be generally tubular, although in other exemplary embodiments the insert intermediate portion 230 may also be a columnar, conical, curved, shaft-like, or cantilevered structure including said insert body lumen 71'. Optionally, the insert intermediate portion 230 is flexible, and can be any suitable thickness (e.g., inner and outer diameter) that will provide structural integrity sufficient to be pushable yet still sized to fit slideably within the outer member guide channel 81. Alternatively, the insert intermediate portion 230 may be tapered. The term "taper" in describing embodiments of the invention means that the inner and/or outer diameter of the insert intermediate portion 230 gradually becomes smaller in the proximal direction relative to the insert mating end portion outer diameter 226 or inner diameter 224. For instance, a taper may be formed by altering the width, height, thickness, and/or cross sectional area of the insert intermediate portion 230.

Turning to the insert connecting end portion 240, the insert connecting end portion 240 comprises an inner surface 245 and an outer surface 247. The insert body lumen 71' extends along at least a portion of the insert connecting end portion 240 and in fluid communication with an insert connecting end portion exit port 242. Optionally, the exit port 242 has an oblique angle, although other configurations of the exit port 242 may be utilized to aid the wire guide, catheter, medical device, or tool, for example, in exiting the insert connecting end portion 240. In one example, the exit port 242 may form a plane substantially perpendicular to the longitudinal axis of the insert intermediate portion 230. In another example, the plane formed by the exit port 242 may be at an angle other than 90 degrees relative to the longitudinal axis of the insert intermediate portion 230. The exit port 242 may have edges that are chamfered, smooth, or rounded.

The insert connecting end portion 240 further comprises an inner compression member connector 144 configured to operatively couple an engaging surface 48' of an inner compression member distal mating end portion 48. The inner compression member connector 144 may be integral with the insert connecting end portion 240 or separate and attached, adjoined, joined, or combined to the insert connecting end portion 240 by any suitable means.

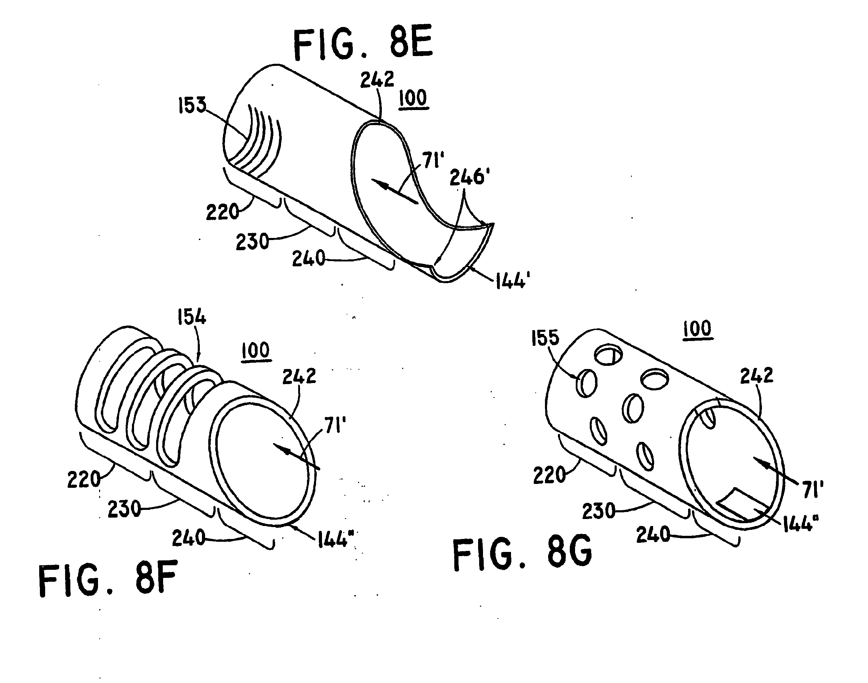

In one embodiment, the inner compression member connector 144 may be any symmetric or asymmetric mechanical structure (e.g., tubular, circular, semi-circular, slotted or circumferential, ringed, band clamp, sleeve, collared, or crimping pinchers, folds, or ridges) for clamping, clutching, gripping, crimping, pinching, fastening, hooking, or joining the insert connecting end portion 240 and inner compression member distal mating end portion 48 (individually and collectively, a crimping connector 144' or connector 144') (see Figs. 8D and 8E). Alternatively, the inner compression member connector 144 may be any chemical or chemical-mechanical means, such as by melt bond, glue, adhesives, resins, welding (laser, spot, etc.), soldering, brazing, adhesives, chemical bonding materials or combinations thereof and the like for holding the insert connecting end portion 240 and inner compression member distal mating end portion 48 (individually and collectively, a bonding connector 144" or connector 144") (see Figs. 8F and 8G). In yet another embodiment, the inner compression member connector 144 comprises a combination crimping connector 144' and bonding connector 144".

If a crimping connector 144' is utilized, then the insert connecting end portion inner surface 245 may be compressed about the inner compression member distal mating end portion engaging surface 48'. More specifically, the insert connecting end portion inner surface 245 is deformed about the inner compression member distal mating end portion engaging surface 48' to hold the insert connecting end portion 240 to the inner compression member distal mating end portion 48. In addition to being the insert connecting end portion inner surface 245, the crimping connector 144' may also be a collar, or attachment to the insert connecting end portion 240, and having one or two sides, projections, pinchers, folds, or ridges (see Figs. 8D and 8E) that crimp onto the inner compression member distal mating end portion engaging surface 48'. Because it can be a cold-working technique, crimping can be used to form a strong bond between a metallic crimping connector 144' and a metallic or even non-metallic inner compression member distal mating end portion 48.

If a bonding connector 144" is utilized, then the inner compression member distal mating end portion engaging surface 48' may be bonded to the insert connecting end portion inner surface 245 (see Fig. 8F) or, alternatively, to the insert connecting end portion outer surface 247 (see Fig. 8G). For example, the inner compression member distal mating end portion engaging surface 48' may be melt bonded directly to the insert connecting end portion inner or outer surfaces 245, 247, respectively. Furthermore, glue, adhesives, resins, chemical bonding materials or combinations may be applied to the inner compression member distal mating end portion engaging surface 48' and/or to either the insert connecting end portion inner or outer surfaces 245, 247 and then the engaging surface 48' and insert connecting end portion inner or outer surface 245,247 brought together and the bonding connector 144" allowed to curse. As another embodiment of a bonding connector 144", the inner compression member distal mating end portion engaging surface 48' and insert connecting end portion inner or outer surface 245, 247 may be brought together and joined by welding (laser, spot, etc.), soldering, or brazing.

Figures 8C through 8G show alternative embodiments of an insert body 100. In Figure 8C, the insert mating end portion 220 comprises a securing portion 151 that is flared. By flaring the securing portion 151 of the insert mating end portion 220, the securing portion 151 operatively couples to the inner guide channel member second end portion 77 by fitting over the outside of the inner guide channel member second end portion 77, by fitting within the guide channel 71 so that the inner guide channel member second end portion 77 drapes over the flared securing portion 151, or by implanting (discussed below) within the inner guide channel member second end portion 77 or between the inner guide channel member second end portion 77 and an outer sleeve (discussed below). So configured, the securing portion 151 operatively couples the inner guide channel member second end portion 77 by a friction fit, by using a bonding material, by using a crimping technique, by using a melt bond, and/or combinations thereof. The flared securing portion 151 and other securing portions help to "push" the stent or stent carrying inner guide channel member 70 distally in order to counter the urge for the stent or stent carrying member to prolapse, recoil, kink, buckle, bunch, or move proximally with the withdrawing of the outer guide channel member 80.

Figure 8D shows an insert body 100 having a crimping connector 144' at or near the insert connecting end portion 240. This crimping connector 144' has opposing projections 246, whereby the inner compression member distal mating end portion 48 is crimped between the projections 246. Figure 8D also shows a securing portion 152 on the inner and/or outer surfaces 225, 227 of the insert mating end portion 220 so as to operatively couple the insert mating end portion 220 and the inner guide channel member second end portion 77 inner contacting interface 103 and/or outer contacting interface 104 (discussed below). The securing portion 152 comprises any substance, compound, molecule, or polymeric material (whether comprising a solid, liquid, fluid, gel, gas, or vapor) chemically bonded via covalent bonds, ionic bonds, or intermolecular bonds (such as ion-dipole forces, dipole-dipole forces, London dispersion forces, and/or hydrogen bonding), adhered, or otherwise applied by the method(s) of laminating, taping, dipping, spraying, depositing, vapor deposition, wrapping (thermally fusing together), painting and curing, and the like. In the particular illustrated, the securing portion 152 is a layer of melt bonding material comprising, by way of example only and not by way of limitation, a nylon, nylon natural tubing, or PEBA.

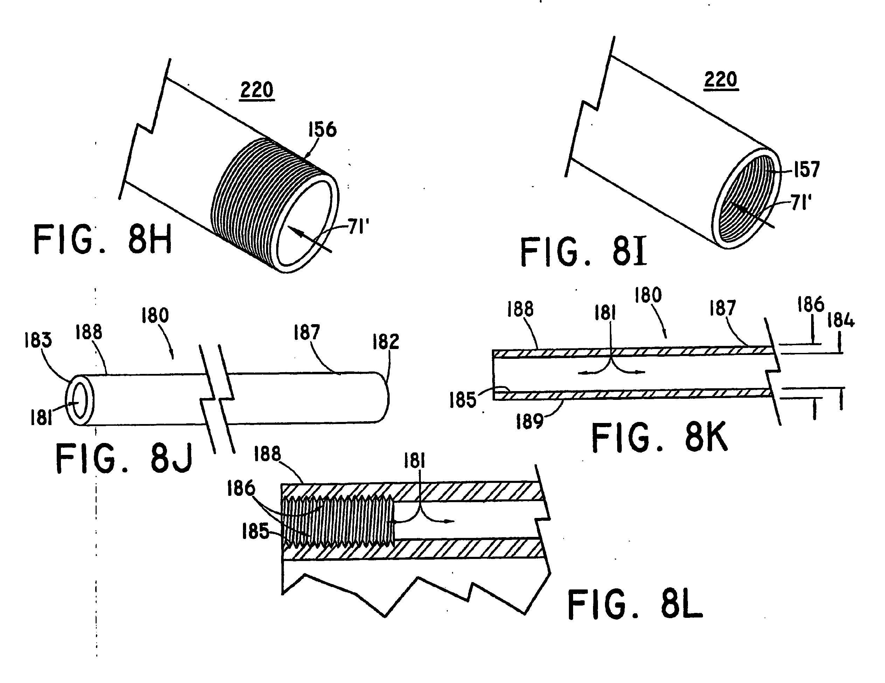

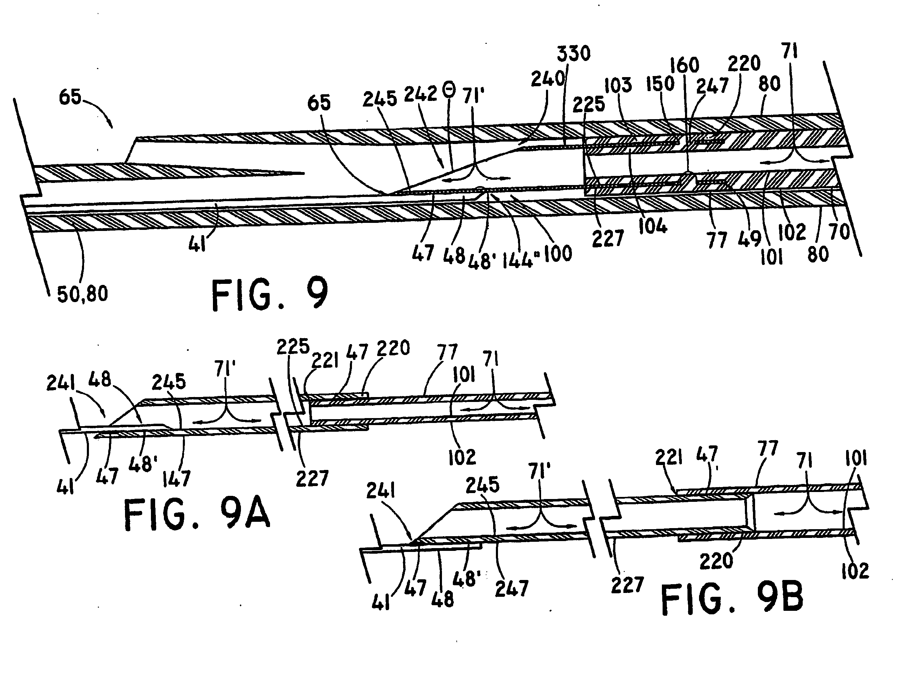

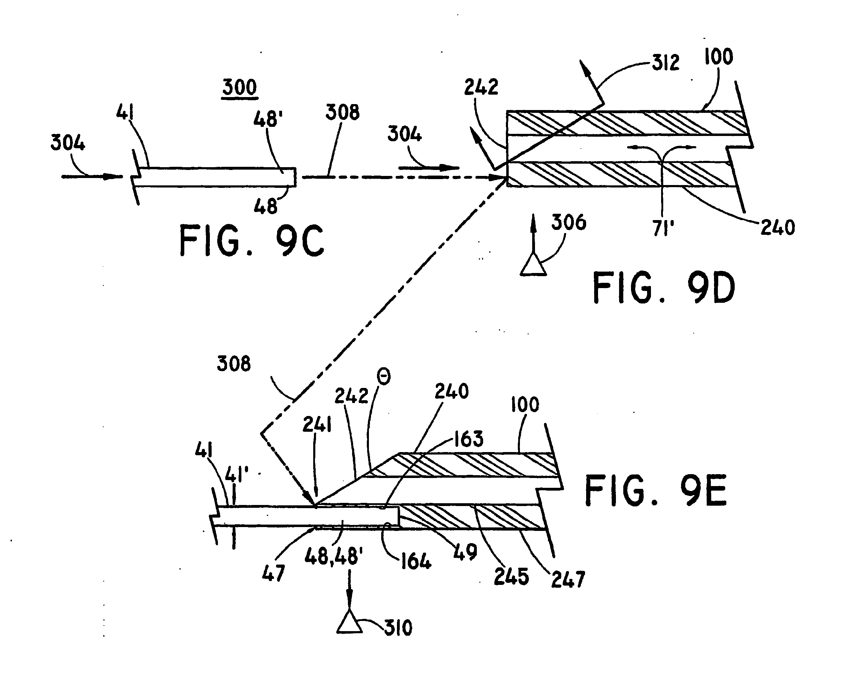

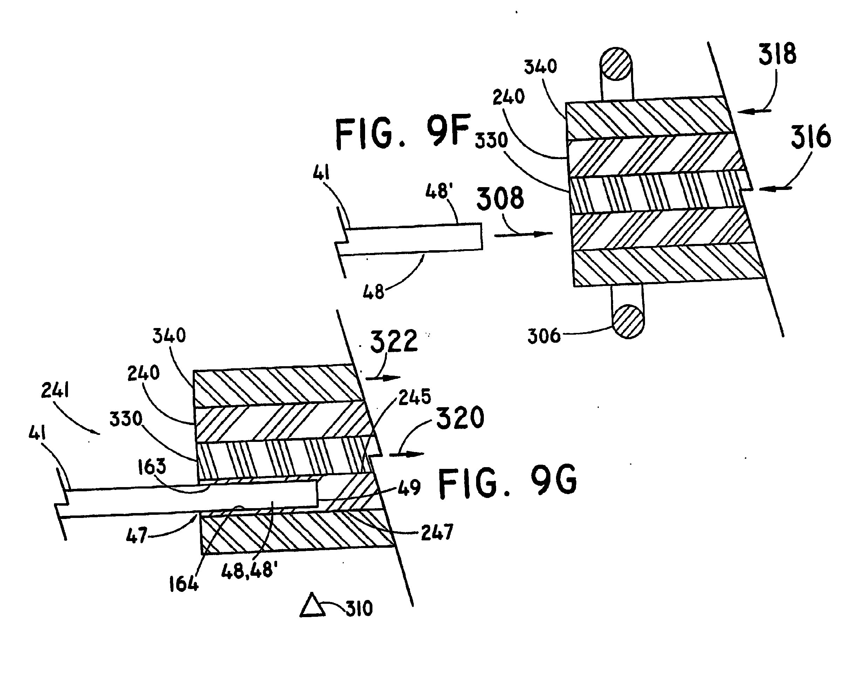

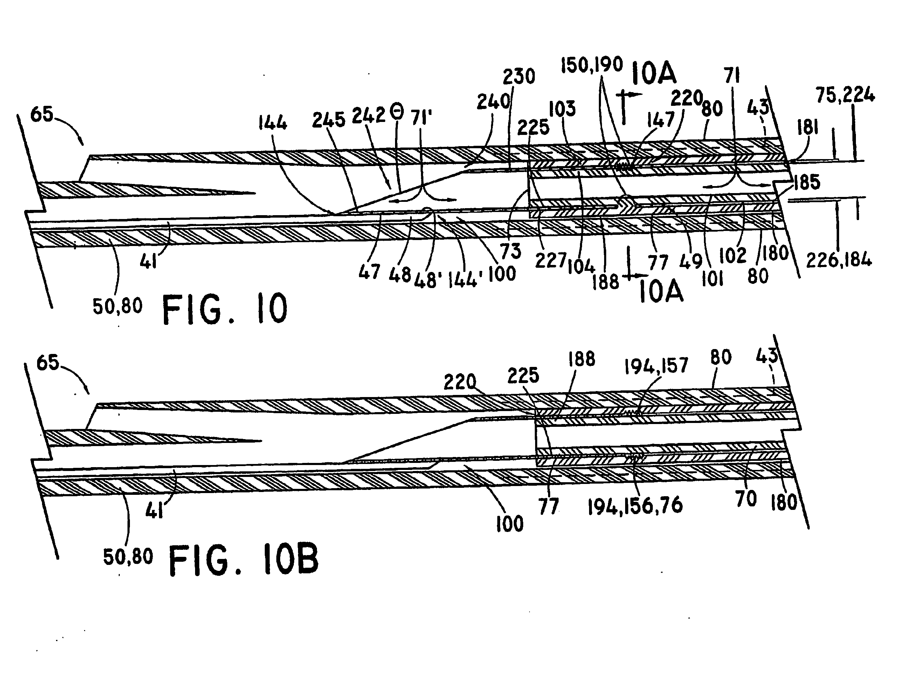

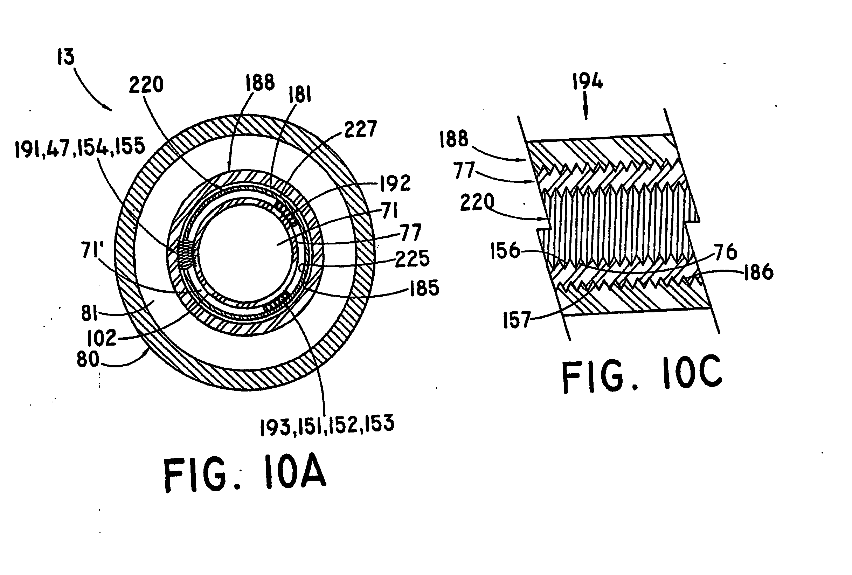

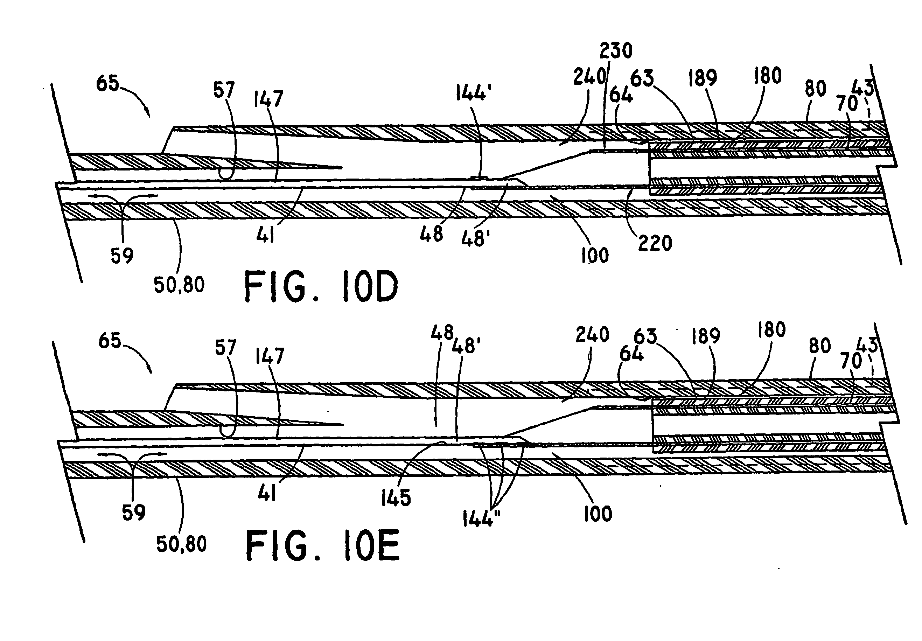

Figure 8E shows an insert body 100 having a crimping connector 144' formed at or near the insert connecting end portion 240. This crimping connector 144' has opposing sides 246', whereby the inner compression member distal mating end portion 48 is crimped between the projections 246'. Figure 8E also shows a securing portion 153 for operatively coupling the insert mating end portion 220 to the inner guide channel member second end portion 77 and/or as taught below an outer sleeve mounting end portion 188 (see Figures 8J, 8K, 8L). The securing portion 153 comprises mechanical and/or chemical etching, striations, sandblasting, laser-cut, machined, threading, milling, drilling, chemical treatment, projections, knurls, ribs, ridges, indentations, cutouts, textured, naturally rough surface (e.g., micro scratches, not 100% smooth) or surface marked by roughness through treatment or by any means, or otherwise preparing the insert mating end portion outer surface 227 and/or inner surface 225 to improve the bonding properties of the insert mating end portion 220 to inner guide channel member second end portion inner contacting interface 103 and/or outer contacting interface 104. In one embodiment, the securing portion 153 comprises a thread or threads for operatively coupling to the outer sleeve mounting end portion 188 (see Fig. 10). In the embodiment illustrated in Figure 8E, the securing portion 153 is defined as the natural or treated surface roughness of the insert mating end portion inner or outer surfaces 225, 227, respectively (see Figs. 8B and 10), for operatively coupling the insert mating end portion inner surface 225 to an inner guide channel member outer surface 102 and/or an insert mating end portion outer surface 227 to the outer sleeve inner surface 185 by a nesting configuration, by a wedge effect, by a press-fit-tight configuration, or by implanting 49 and/or melt bonding 47 (see Fig. 9).

Figure 8F and Figure 8G have insert mating end portions 220 with securing portions 154, 155, respectively, for operatively coupling the insert mating end portion 220 to the inner guide channel member second end portion 77 and/or the outer sleeve mounting end 188 (see Figures 8J, 8K, 8L, and 10). The securing portions 154, 155 comprise one or more apertures. In Figure 8F, the securing portion 154 comprises a slot. In Figure 8G, the securing portion 155 comprises a perforation. The slotted securing portion 154 and perforated securing portion 155 may be laser-cut, machined, milled, or drilled. The slotted securing portion 154 increases the flexibility of the insert mating end portion 220. In addition, both the perforated securing portion 155 and slotted securing portion 154 optimize the bonding retention properties for securing the insert mating end portion 220 to the outer guide channel member second end portion 87. By way of example, the insert mating end portion 220 may be implanted within the inner guide channel member second end portion 77 or disposed between the inner guide channel member second end portion 77 and an outer sleeve 180. In such a case, materials that form inner or outer surfaces of the inner guide channel member second end portion 77-or of the outer sleeve 180 and the inner guide channel member second end portion 77-are joined during a melt bonding process, thereby securing the insert mating end portion 220 between surfaces (or interfaces) of the inner guide channel member second end portion 77, or sandwiched between a surface (or interface) the inner guide channel member second end portion 77 and a surface (or interface) of the outer sleeve 180, as shown by way of example in Figures 9 and 10, 10D, 10E, and 10F.

Figures 8H and 8I show alternative schematic embodiments of securing portions 156, 157, respectively, for operatively coupling the insert mating end portion 220 to the inner guide channel member second end portion 77 and/or the outer sleeve mounting end portion 188 (see Figures 8J, 8K, 8L, and 10). Optionally, the insert mating end portion 220 may have securing portions 156, 157 comprising internal and/or external threads, respectively. For instance, Figure 8H shows an insert mating end portion 220, broken away, having a lumen 71' and comprising an external threaded securing portion 157. Figure 8I shows an insert mating end portion 220, broken away, having a lumen 71' and comprising an internal threaded securing portion 156. In one embodiment, the insert mating end portion 220 may comprise both the internal threaded securing portion 156 and an external threaded securing portion 157 for operatively coupling the insert mating end portion 220 to the inner guide channel member 70 and/or outer sleeve 180 (see Figs. 10B and 10C).

It should be understood that securing portions 150, 151, 152, 153, 154, 155, 156, and 157 may be used in combination with other securing portions and also with other embodiments of the insert body 100. By way of example and not by way of limitation, an insert mating end portion 220 may have the securing portion 151 described with Figure 8C as well as the securing portion 152 described with Figure 8D. Also, connectors 144, 144', and 144" may be used in combination with other connectors. Thus, the crimping connector 144' described with Figure 8E may be used in conjunction with the bonding connector 144" described with Figure 8F. These combinations are only exemplary and not limiting.

The length of the insert body 100 may vary from about 10.0 to 40.0 mm. In one particular embodiment, the length is approximately 15.0 to 25.0 mm. In still another embodiment, the length is approximately 20.0 mm. The diameter of the lumen 71' also may vary. For instance, the inner diameter 224 may range from about 1 French to about 4 French, although this may vary as desired. The outer diameter 226 may range from about 2 French to about 5 French, although this may vary as desired. The opening 242 may be at an angle between a range of zero to about 90 degrees. The insert body 100 may comprise any of the materials as previously described as making up the system distal portion 13 or the middle section delivery device 14. In one embodiment, the insert body 100 comprises a stainless steel cannula. In another embodiment, the insert body 100 is a cannula comprising a super-elastic alloy, such as nitinol or equivalents thereof.

Figure 8J shows a schematic perspective view of an optional outer sleeve 180 that operatively couples to the insert mating end portion 220. Optionally, the outer sleeve 180 has a generally tubular configuration that is disposed about (e.g., envelopes, surrounds, wraps around, covers, overlays, superposes over, encases, ensheaths, melt bonds to, and the like) at least one securing portion 150-157 of the insert mating end portion 220 (see Figs. 8A-8I). In addition, the outer sleeve 180 has a distal first end portion 187 (outer sleeve first end portion 187) with a first port 182 and a proximal mounting end portion 188 (outer sleeve mounting end portion 188) with a second port 183 and a sleeve lumen 181 extending therethrough. In particular, the outer sleeve mounting end portion 188 is configured to operatively couple the optional outer sleeve 180 to the insert mating end portion 220.

Figure 8K shows a longitudinally sectioned side view, broken away, of the outer sleeve 180 of Figure 8J. The outer sleeve mounting end portion 188 has an inner diameter 184 substantially similar to the insert body mating end portion outer diameter 226 such that the outer sleeve inner diameter 184 concentrically disposes about at least a portion of the insert mating end portion 220 and substantially aligns with (e.g., proximal to, even with, abutting, juxtaposed, adjoining, in contact, at or near, contiguous, and/or corresponding to) the at least one insert securing portion 150-157 of the insert mating end portion 220 (see Figs. 8A-8I). Thus, the outer sleeve inner diameter 184 and insert mating end portion outer diameter 226 may operatively couple the outer sleeve mounting end portion 188 and the insert mating end portion 220 by any suitable means such as, and one embodiment, by a wedge effect, a press-fit-tight configuration, or surface roughness. In another embodiment, the outer sleeve mounting end portion 188 has an inner surface 185 comprising glue, adhesive, resin, or chemical bonding material for operatively coupling the outer sleeve inner surface 185 and the insert mating end portion outer surface 227. In yet another embodiment, the outer sleeve inner surface 185 comprises melt bonding material for operatively coupling to an insert securing portion 150-155. The outer sleeve inner surface 185 and insert mating end portion outer surface 227 may also be operatively coupled by welding (laser, spot, etc.), soldering, or brazing. In addition to its outer sleeve inner surface 185, the outer sleeve mounting end portion 188 also comprises an outer sleeve outer surface 189 (see Fig. 8K).

Figure 8L shows an alternative embodiment, longitudinally sectioned and broken away, of the outer sleeve 180 according to Figure 8J. The outer sleeve mounting end portion 188 comprises a lumen 181 and one or more internal threads 186 projecting into the lumen for operatively coupling to the insert securing portion 157 (see Fig. 8H). Thus, it should be understood in this embodiment that the lumen is variable between peaks and valleys of the internal threads 186. An inner diameter 184 but may be measured by the narrowest width of the lumen 181. In addition, the outer sleeve internal threads 186 and/or insert securing portion 157 may comprise a bonding material (e.g., glue, adhesive, resin, chemical bonding materials, and the like) for further operatively coupling the outer sleeve threads 186 and the insert securing portion 157.

An outer sleeve 180 according to the invention may be formed of any suitable material that will provide appropriate structural reinforcement. In one embodiment, the outer sleeve 180 comprises nylon, nylon natural tubing, or PEBA. Alternatively, the outer sleeve 180 comprises medical grade stainless steel or other metals, polymers, plastics, alloys (including super- elastic alloys), or composite materials. The outer sleeve 180 may be flexible. The length of the outer sleeve 180 of any of the embodiments may vary generally from about 5 to 15 cm. The inner diameter 184 may range from about 1 French to about 4 French, although this need not be uniform (e.g., it may taper or vary as with threads) and may be longer or shorter, as desired. The outer diameter 186 may range from about 2 French to about 5 French, although this may vary (e.g., it may taper) and may be longer or shorter, as desired.

### INTERNAL CANNULATED JOINT

Figure 9, as another exemplary embodiment of a system distal portion 13, shows a partially sectional view and broken away of the system distal portion 13 having an insert body 100, as described more fully above, for operatively coupling the inner compression member 41 and the inner guide channel member 70. One embodiment of the insert body 100 comprises a longitudinally dimensioned substantially (approximately) annular insert mating end portion 220, an insert connecting end portion 240 having an inner surface 245 and outer surface 247, and an insert intermediate portion 230, and further having a lumen 71' extending there through. In particular, the insert connecting end portion 240 includes an inner compression member connector 144 (which may comprise a crimping connector 144' and/or a bonding connector 144" as described above) configured to secure an engaging surface 48' of an inner compression member distal mating end portion 48.

In order to operatively couple the insert body 100 and the inner guide channel member 70, the insert mating end portion 220 is implanted 49 into the second end portion 77 of the inner guide channel member 70. Implanting 49 describes an embodiment whereby the insert mating end portion 220 penetrates the inner guide channel member second end portion 77, and as a result, the insert mating end portion 220 is embedded in the inner guide channel member second end portion 77. Stated otherwise, implanting 49 describes an embodiment wherein the insert mating end portion 220 is inserted and inlaid between inner and outer surfaces 101, 102, respectively, of the inner guide channel member second end portion 77, which surfaces 101, 102 are relative to the wire guide channel 71 of the inner guide channel member 70.

Therefore, the implanted 49 insert mating end portion 220 defines an inner guide channel member second end portion 77 having an insert engaging inner contacting interface 103 and an insert engaging outer contacting interface 104 (hereinafter "inner contacting interface," "outer contacting interface," "contacting interface(s)") between the inner and outer surfaces 101, 102, respectively, of the inner guide channel member second end portion 77. This describes an inner contacting interface 103 of the inner guide channel member second end portion 77 that would be between the implanted 49 insert mating end portion 220 and the inner guide channel member second end portion inner surface 101. Likewise, the outer contacting interface 104 of the inner guide channel member second end portion 77 is between the implanted 49 insert mating end portion 220 and the inner guide channel member second end portion outer surface 102.

Because implanting 49 disposes the insert mating end portion 220 into the material comprising the inner guide channel member second end portion 77, the insert mating end portion 220 is substantially enveloped by the inner guide channel member second end portion's outer contacting interface 104 and inner contacting interface 103, which inner guide channel member second enc portion contacting interfaces 103, 104 operatively couple the insert mating end portion 220 inner and outer surfaces 225, 227, respectively. Implanting 49 is typically accomplished by softening (e.g., with heat sufficient to partially melt the material) the inner guide channel member second end portion 77, and then cooling so as to solidify the inner guide channel member second end portion inner and outer contacting interfaces 103, 104 around the insert mating end portion 220. In other words, implanting 49 provides a mechanical, chemical, and/or chemical-mechanical surface-to-surface contact between the insert mating end portion inner surface 225 and the inner contacting interface 103 of the inner guide channel member second end portion 77. Likewise, implanting provides a mechanical, chemically, and/or chemical-mechanically surface-to-surface contact between the insert mating end portion outer surface 227 and outer contacting interface 104 of the inner guide channel second end portion 77. The insert mating end portion 220 is implanted 49 to provide a pullout strength of at least 5 newtons and preferably a pullout strength of at least 20 newtons.

Embodiments of the invention also comprise an optional junction 160 operatively coupling at least one of the inner guide channel member second end portion inner contacting interface 103 and/or the outer contacting interface 104 to any one or more or combinations of a securing portion "150" (individually and collectively, 150, 151, 152, 153, 154, 155, 156, and/or 157) of an insert mating end portion 220. For instance, the optional junction 160 may comprise a bonding material (e.g., glue, adhesive, resin, chemical bonding materials, and the like) operatively coupling at least one inner guide channel member second end portion contacting interface 103, 104 and the insert mating end portion securing portion 150. By way of a further example, the optional junction 160 may comprise a melt bond 47 (described below) operatively coupling the least one inner guide channel member second end portion contacting interface 103, 104 and the insert mating end portion securing portion 150. The optional juncture 160 provides the operatively coupled insert mating end portion 220 and inner guide channel member second end portion 77 with a pullout strength of at least 5 newtons and preferably a pullout strength of at least 20 newtons.

In one embodiment, the optional junction 160 is formed by the inner guide channel member second end portion inner contacting interface 103 being directly bonded to the inner guide channel member second end portion outer contacting interface 104 through the insert mating end portion slotted securing portion 154 and/or a perforated securing portion 155 (described above) by a melt bond 47 (described above and below). This helps to form a more solid connection, and additional strength, between the insert body 100 and the inner guide channel member 70. A solid-state bond results from using a suitable form of heat for melting and then solidifying (e.g., fusing and/or cross-linking bonds formed at the melt bonded material interfaces) material of the inner guide channel member second end portion inner and outer contacting interfaces 103, 104 at the junction 160 and about the insert mating end portion inner and outer surfaces 225, 227. In another embodiment, a junction 160 may comprise a flared securing portion 151 or one or more mechanically and/or chemically etched securing portions 152, 153 (described above), or a combination thereof. The flared securing portion 151, mechanically etched securing portion 152, and/or chemically etched securing portion 153 may be melt bonded 47 to the inner guide channel member second end portion inner contacting interface 103 and/or to the inner guide channel member second end portion outer contacting interface 104. One embodiment utilizes a plurality (two or more) of optional junctions 160.

It should be understood that, when describing embodiments of implanting 49 the insert mating end portion 220 into the inner guide channel member second end portion 77, or describing embodiments of disposing the insert mating end portion 220 such that it is sandwiched between the inner guide channel member second end portion outer surface 102 and the outer sleeve 180 as described below (see Figs. 10 and 10A-10E), the entire longitudinally dimensioned length of the insert mating end portion 220 need not be implanted or sandwiched. Rather, only a length that is sufficient to secure the insert mating end portion 220 to the inner guide channel member second end portion 77 and/or the inner guide channel member second end portion 77 and outer sleeve 180 need be implanted 49 or sandwiched. A longer length generally increases bonding strength but may be less flexible, while a shorter length generally gives lesser bonding strength but may improve flexibility. Alternatively, the insert mating end portion 220 may be tapered in order to reduce thickness in the region where the insert mating end portion 220 is implanted into the inner guide channel member second end portion 77, as described above.

In one embodiment, the insert mating end portion 220 may be implanted 49 from approximately 1.0 mm to approximately 10.0 mm. In another embodiment, the insert mating end portion 220 may be implanted from approximately 3.0 mm to approximately 7.0 mm, and in still another embodiment the insert mating end portion 220 may be implanted approximately 5.0 mm. The optional juncture 160 may comprise a bonding material (e.g., glue, adhesive, resin, chemical bonding materials, and the like) and/or melt bond 47 having an area from about 0.5 mm² to approximately 1.0 mm² (or more) relative to the insert contacting surfaces.

As used to describe an embodiment of the invention, melt bonding 47 (for shorthand purposes in describing embodiments according to the invention, the term melt bonding 47 includes implanting 49 that results in a melt bond 47 between two components, surfaces, layers, and/or interfaces) comprises any suitable means for melting, semi-melting, making molten or semi-molten, liquefying, softening, softened, making tacky, or fusing, or making malleable, pliant, supple, moldable, ductile, or otherwise penetrable (individually and collectively, "melt," "melting," "melted," and variants thereof) by another component such as the insert body 100. For instance, a radiofrequency loop heater may be used to melt the inner guide channel member second end 77. Such a machine is available from Magnaforce, Incorporated and sold under the name and model Heatstation 1500. Another such machine is available from Cath-Tip, Inc. and is sold under the model and name Cath-Tip II.

Melt bonding 47 involves bringing two components together at an interface, wherein one or both of the component interfaces are in the melted, tacky state. Melt bonding 47 may be single layer interface whereby one component interface/surface mates to a second component interface/surface, or may be multi-layer interface whereby one component is implanted 49 into a second component as described above. Melt bonding typically requires that one or both of the components be melted at the interface, and that they may be sufficiently chemically and physically compatible such that they fuse together upon cooling. The chemical compatibility may be expressed in terms of having similar values for surface energy and/or solubility parameter. In simple terms, similar materials may tend to have a mutual affinity and a greater propensity to adhere to one another than do dissimilar materials. As used herein, melt bonding 47 includes bonding whereby one component is melted while the other component is at or above its melting point.

Melt bonding 47 between the insert mating end portion inner and outer surfaces 225, 227, respectively and the inner guide channel member second end portion inner or outer contacting interfaces 103, 104 is almost instantaneous once the melting temperature is reached and, likewise, the inner compression member distal mating end portion engaging surface 48' and one of the insert connecting end portion inner or outer surfaces 245, 247, respectively. The result is a solid-state bond (e.g., fusing, chemical bonding, and/or cross-linking bonds formed at the melt bonded material interfaces) between the material of the insert mating end portion inner and outer surfaces 225, 227 and the inner guide channel member second end portion inner and outer contacting interfaces 103, 104 and, likewise, the material of the inner compression member distal mating end portion engaging surface 48' and one of the insert connecting end portion inner or outer surfaces 245, 247, respectively. A melt bond 47 according to embodiments of the invention provides a pull apart strength of at least 5 newtons and preferably a pull apart strength of at least 20 newtons.

Melt-bonding material(s) described above may be utilized (collectively and individually as "nylon" and/or "PEBA"), and may be used alone or in a combination of two or more to form a melt bond 47 to operatively couple one of the inner guide channel member engaging surfaces (e.g., inner surface 101 and outer surface 102) and one of the insert mating end portion engaging surfaces (e.g., inner surface 225 and outer surfaces 227) to form a first connection 221 and to operatively couple the inner compression member distal mating end portion engaging surface 48' to one of the insert connecting end portion engaging surfaces (e.g., inner surface 245 and outer surface 247) to form a second connection 241 (see Figs. 9A and 9B discussed below).

Materials have different "melt bonding" temperatures at which the material will melt without substantial degradation. In one embodiment where PEEK tubing is used for the inner guide channel member second end portion 77, for instance, the PEEK melts at about 334°C (633°F). Thus, the inner guide channel member second end 77 is heated from about 331°C (628°F) to about 337°C (638°F). There is a rise dwell and cool down time for the process. The total rise time is approximately 20 seconds and the dwell time is approximately 10 seconds. During the dwell time the temperature is approximately 316°C (600F). At or near the end of the dwell time, the inner guide channel member second end portion 77 has melted and the insert mating end portion 220 is then implanted 49 into the melted inner guide channel member second end portion 77. Otherwise stated, the inner member second end portion 77 softens under heat and, before that tubing burns or degrades, the insert mating end portion 220 is pushed quickly into the wall of the softened PEEK between the inner guide channel member second end portion inner surface 101 and the second end outer surface 102.

The implanted insert mating end portion 220 and inner guide channel member second end portion 77 are cooled by any means for allowing the melted inner guide channel member second end portion 77 to return to solid state (e.g., become solid, again). Thus, the joined insert mating end portion 220 and inner guide channel member second end portion 77 may be brought back down to room temperature. In one embodiment, the cool down time is approximately 30 seconds. As a result, the insert mating end portion 220 has become implanted 49 into the melted wall of the tubing between the inner guide channel member second end portion inner and outer surfaces 101, 102, respectively. When PEEK (for example) melts it expands, and when it cools it shrinks, and therefore, the PEEK will grip tighter onto the implanted insert mating end portion 220 through this process to give additional mechanical bonding as described above.

Figure 9 also shows an insert connecting end portion 240 comprising an inner compression member connector 144 configured to secure an engaging surface 48' of an inner compression member mating end portion 48. Shown is a bonding connector 144" such as a melt bond, laser weld, spot weld, or any one or more bonding connectors 144" described above and providing surface-to-surface contact between the outer engaging surface 48' of the inner compression member distal mating end portion 48 and an insert connecting end portion outer surface 247, thereby forming a more solid connection between the inner compression member 41 and the insert connecting end portion 240. Alternatively, the bonding connector 144" may join the outer engaging surface 48' of the inner compression member distal mating end portion 48 and the insert connecting end portion inner surface 245. A bonding connector 144" between the outer engaging surface 48' of the inner compression member distal mating end portion 48 and the insert connecting end portion inner or outer surfaces 245, 247, respectively, is almost instantaneous once the melting temperature is reached, resulting in a solid-state bond the outer engaging surface 48' and the insert connecting end portion inner or outer surface 245, 247.

As shown in Figure 9, the insert connecting end portion exit port 242 may have an oblique acute angle θ. In one embodiment, the insert connecting end portion 240 may be skived to give the second end an angle θ from about 40 degrees to about 50 degrees, and in another embodiment the angle is about 45 degrees.

Figures 9A and 9B are longitudinally sectioned, broken away, schematic views showing alternative embodiments of an internal joint according to the invention. Optionally, the inner guide channel member second end portion 77 disposes about the insert mating end portion 220 such that the insert mating end portion 220 extends at least partially within the inner member guide channel 71, or alternatively, the insert mating end potion 220 disposes about the inner guide channel member second end portion 77 such that inner guide channel member second end portion 77 extend at least partially within the insert body lumen 71'. The lumen 71' and channel 71 are in fluid communication.

A first connection 221 operatively couples the inner guide channel member second end portion 77 and the insert mating end portion 220. Optionally, the first connection 221 comprising a wedge effect, a friction fit, a press-fit-tight configuration, crimping, welding (laser, spot, etc.), soldering, brazing, bonding material (e.g., glue, adhesive, resin, chemical bonding materials, and the like), or combinations thereof. The second connection 241 operatively couples the inner compression member distal mating end portion 48 and the insert connecting end portion 240. Optionally, the second connection 241 comprises crimping, welding (laser, spot, etc.), soldering, brazing, bonding material (e.g., glue, adhesive, resin, chemical bonding materials, and the like), or combinations thereof. In one embodiment, the second connection 241 comprises an inner compression member connector 144 (see Fig. 9).

In one embodiment, at least one of the first connection 221 and second connection 241 comprises a melt bond 47. The first connection melt bond 47 may operatively couple one of the inner guide channel member second end portion engaging surfaces (e.g., inner surface 101 and outer surface 102) and one of the insert mating end portion engaging surfaces (e.g., inner surface 225 and outer surfaces 227) to form a first connection 221. A second connection melt bond 47 may operatively couple the inner compression member distal mating end portion engaging surface 48' to one of the insert connecting end portion engaging surfaces (e.g., inner surface 245 and outer surface 247) to form a second connection 241.

In Figure 9A, for example, one embodiment of the invention comprises an insert mating end portion 220 having an inner surface 225, wherein the inner surface 225 comprises melt bonding material or is bonded to melt bonding material of the inner guide channel member second end portion outer surface 102 and, optionally, the inner guide channel member second end portion outer surface 102 may comprise melt bonding material or bond to melt bonding material of the insert mating end portion inner surface 225. The inner guide channel member second end portion 77 is sized to be inserted into the insert body lumen 71' such that the insert mating end portion inner surface 225 disposes about the inner guide channel member second end portion outer surface 102 and the insert body lumen 71' is in fluid communication with the inner member guide channel 71. The insert mating end portion inner surface 225 and the inner guide channel member second end portion outer surface 102 are melt bonded 47 at the first connection 221. The inner compression member 41 comprises a distal mating end portion 48 having an engaging surface 48' operatively coupled to the inner surface 245 of the insert connecting end portion 240, whereby optionally the inner compression member distal mating end portion engaging surface 48' and the insert connecting end portion inner surface 245 are melt bonded 47 at the second connection 241. Alternatively, the inner compression member distal mating end portion engaging surface 48' and the insert connecting end portion outer surface 247 are melt bonded 47 at the second connection 241 (see Fig. 9B).

In an alternative embodiment illustrated in Figure 9B, the insert mating end portion outer surface 227 comprises melt bonding material or is bonded to melt bonding material of the inner guide channel member second end portion inner surface 101 and, optionally, the inner guide channel member second end portion inner surface 101 comprises melt bonding material or is bonded to melt bonding material of the insert mating end portion outer surface 227. The insert mating end portion 220 is sized to be inserted into the inner member guide channel 71 such that the inner guide channel member second end portion inner surface 101 disposes about the inert mating end portion outer surface 227 and the inner member guide channel 71 is in fluid communication with the insert body lumen 71'. The insert mating end portion outer surface 227 and the inner guide channel member second end portion inner surface 101 are melt bonded 47 at the first connection 221. The inner compression member 41 comprises a distal mating end portion 48 having an engaging surface 48' operatively coupled to the outer surface 247 of the insert connecting end portion 240, whereby optionally the engaging surface 48' and insert connecting end portion outer surface 247 are melt bonded 47 at the second connection 241. Alternatively, the inner compression member distal mating end portion engaging surface 48' and the insert connecting end portion inner surface 245 are melt bonded 47 at the second connection 241 (see Fig. 9A).

Figures 9C through 9G shows a second connection 241 wherein the first connection is formed by implanting 49 (and optionally melt bonding 47) an inner compression member 41 as described above into an insert body 100 as described above. More particularly, an inner compression member 41 (e.g., Figs. 2, 2C, 4, 5, 6, 7, 7A-7C, 9, 9A-9G, 10, 10A) comprises a distal mating end portion 48 having an outer engaging surface 48'. Furthermore, an insert body 100 (e.g., Figs. 8A-8I, 9, 9A, and 9B) comprises an insert mating end portion 220 having an entry port 222 and an insert connecting end portion 240 with an exit port 242, the entry and exit ports 222, 242, respectively, define a insert body lumen 71' therebetween such that insert connecting end portion 240 has inner and outer surfaces 245, 247, respectively. The inner compression member distal mating end portion 48 is implanted 49 (and optionally melt bonded 47) into the insert connecting end portion 240 of the insert body 100 to form a second connection 241.

Figures 9C, 9D, 9E, 9F, and 9G show a method 300 of providing a second connection 241 for operatively coupling a distal mating end portion 48 of an inner compression member 41 to an insert body 100 to be used with a delivery apparatus such as, by way of example only, the rapid insertion of medical devices that includes a proximal end, elongate flexible middle section delivery device having said inner compression member, and a system distal portion having an outer guide channel member and said inner guide channel member. In general, the second connection 241 is formed by implanting 49 (and optionally melt bonding 47) an inner compression member distal mating end portion 48 into the insert connecting end portion 240 of the insert body 100.

In Figure 9C, an elongate inner compression member 41 is provided 302 having features and comprising materials as described above, including a distal mating end portion 48 having an outer engaging surface 48'. Indeed, a stainless steel stylet available from Cook Incorporated may be used for the inner compression member. In one embodiment, the inner compression member 41 is solid. The length may vary, and in one embodiment may be about 139 cm for a delivery system 10 for the rapid insertion of an 8.0 cm self-expanding stent. For stents that are longer, the inner compression member 41 may be longer, because the shorter stent usually takes a shorter inner guide channel member 70. The inner compression member outer diameter may vary. In one embodiment, the outer diameter is approximately 0.06096cm (0.024 inches). Through centerless grinding, the mating end 48 that is melt bonded to the inner guide channel member second end portion 77 PEEK tubing is tapered down to an outer diameter approximately 0.04064cm (.016 inches) over a length of approximately 5.0 cm.

In Figure 9D, an insert body 100 also is provided 304 having features and comprising materials as described above, including a distal insert mating end portion 220 (see Figs. 8A-8I) and a proximal insert connecting end portion 240 and defining an insert body lumen 71 ', an insert connecting end portion exit port 242, and whereby the insert connecting end portion 240 further has an inner surface 245 and an outer surface 247. In one embodiment, this comprises PEEK tubing. In another embodiment, the insert body 100 is a plastic cannula.

Figure 9D further shows that the insert connecting end portion 240 is melted 306 by any suitable means, such as heat. For instance, a radiofrequency loop heater may be used to melt 306 the insert connecting end portion 240. Such a machine is available from Magnaforce, Incorporated and sold under the name and model Heatstation 1500. Another such machine is available from Cath-Tip, Inc. and is sold under the model and name Cath-Tip II.

The inner compression member 41 may be melt bonded (step 306) to the insert body 100 without being implanted into insert connecting end portion 240. As shown in Figures 4, 5, and 7, for example, an outer engaging surface 48' of the inner compression member distal mating end portion 48 may form a melt bond 47 to an inner surface 101 of the inner guide channel second end portion 77 or, alternatively, to the outer surface 102 of the inner guide channel second end portion 77. Likewise, the outer engaging surface 48' of the inner compression member distal mating end portion 48 may form a melt bond 47 to an insert connecting end portion inner surface 245 or, alternatively, to the insert connecting end portion outer surface 247.

Materials have different "melt bonding" temperatures at which the material will melt without substantial degradation. In one embodiment where PEEK tubing is used, because PEEK melts at about 633°F, the insert body connecting end 140 is heated from about 628°F to about 638°F. There is a rise dwell and cool down time for the process. The total rise time is approximately 20 seconds and dwell time is approximately 10 seconds. During the dwell time the temperature is approximately 600°F.

Figure 9D further shows optional skiving 312. The insert connecting end portion 240 may be skived 312 to give it an oblique angle θ from about 40 degrees to about 50 degrees, and in another embodiment the angle is about 45 degrees.

Turning to Figure 9E, at or near the end of the dwell time, the insert connecting end portion 240 has melted and is softened. The inner compression member distal mating end 48 is implanted 308 into the melted insert connecting end portion 240. Otherwise stated, the connecting end tubing under heat has softened and, before the tubing burns or degrades, the inner compression member distal mating end portion 48 is moved quickly into the wall of the softened PEEK between the insert connecting end portion inner and outer surfaces 245, 247, respectively, such that the outer engaging surface 48' of the inner compression member distal mating end portion 48 operatively couples by, for example, a melt bond 47 to insert connecting end portion inner and outer contacting interfaces 163, 164 between the insert connecting end portion inner and outer surfaces 245, 247.

The implanted inner compression member mating end 48 and the insert connecting end portion 240 are cooled 310 (Figure 9E). As used to describe an embodiment of the invention, the cooling 310 step is any means for allowing the melted insert connecting end portion 240 to return to solid state (e.g., become solid, again). Thus, the joined inner compression member distal mating end portion 48 and the insert connecting end portion 240 may be brought back down to room temperature. In one embodiment, the cool down time is approximately 30 seconds. As a result, the mating end has become implanted into the melted wall of the PEEK tubing (for instance) comprising the insert connecting end portion 240. This forms a solid joint, because when PEEK (for instance) melts it expands, and when it cools it shrinks and grips tighter onto the inner compression member distal mating end portion 48. This gives additional mechanical bonding as described above.

Optionally, as Figure 9F shows, the insert connecting end portion 240 according to Figure 9D may have a mandrel 330 positioned (step 316) within at least a portion of the insert body lumen 71' such as the insert body lumen 71' at the insert connecting end portion 240 for helping to maintain patency of the insert connecting end portion 240 during the melting step 306 and/or implanting step 308. In one embodiment, the mandrel 330 is a stainless steel mandrel having a Teflon coating, a round cross-section, and a diameter that measures approximately 0.0205 inches.

Optionally, also shown in Figure 9F, a tool 340 is disposed about (step 318) at least a portion of the insert connecting end portion 240 for helping to maintain patency of the insert connecting end portion 240 during the melting step 306 and/or implanting step 308 and to conduct heat during the melting step 306. In one embodiment, the tool 340 is a metal cannula having an inner diameter that measures approximately 0.0430 inches.

At this point, the insert connecting end portion 240 is sandwiched between the mandrel 330 and the tool 340. The assembly is then melted 306 by being placed within a radiofrequency heater loop until the insert connecting end portion 240 softens. The inner compression member distal mating end portion 48 is then implanted 308 by being pushed into the softened insert connecting end portion 240.

Figure 9G shows the inner compression member distal mating end portion 48 implanted 49 between the insert connecting end portion inner and outer surfaces 245, 247, respectively, such that the inner compression member distal mating end portion outer engaging surface 48' operatively couples by, for example, a melt bond 47 to insert connecting end portion inner and outer contacting interfaces 163, 164 between the insert connecting end portion inner and outer surfaces 245, 247.

After cooling 310, the mandrel 330 is then removed (step 320) by any suitable means. Also, the tool 340 is removed (step 322) by any suitable means.

A method of manufacturing and of providing a medical device having a second as taught herein need not be performed sequentially. For instance, in method 300, an insert body 100 may be provided 304, and then the inner compression member 41 provided 302. Also, a mandrel 330 may be positioned 316 before or after skiving 312 or the tool 340 is disposed about 318 the insert body connecting end 140. Likewise, the mandrel 330 may be removed (step 320) before or after the tool 340 is removed (step 322).

Moreover, the inner compression member 41 may be an elongate catheter or any other elongate member (tubular or solid) comprising a mating end having an outer engaging surface. Furthermore, the insert body 100 may be a cannula or other tubular member comprising a first end portion and a second end portion, whereby the second end portion has inner and outer surfaces.

Likewise, the method 300 can be used to explain how to manufacture the embodiment of the insert body 100 operatively coupling to the inner guide channel member 70 at the first connection 221. The first connection 221 (see Figs. 9A and 9B discussed below) comprises implanting 49 the mating end portion 220 of the insert body 100 into the second end portion 77 of the inner guide channel member 70.- According to method 300, the insert mating end portion 220 may be implanted 49 between the inner and outer surfaces 101, 102, respectively, of the inner guide channel member second end portion 77 such that the insert mating end portion inner and outer surfaces 225, 227, respectively, operatively couples by, for example, a melt bond 47 to inner and outer contacting interfaces 103, 104 between the inner and outer surfaces 101, 102 of the inner guide channel member second end 77.

Turning to Figure 10, as another exemplary embodiment of a system distal portion 13 comprising an outer guide channel member 80 (optionally having a coil 43), shows a partially sectional view and broken away of the system distal portion 13 having an insert body 100, as described more fully above, for joining the inner compression member 41 and the inner guide channel member 70. A device according to this embodiment includes an elongate inner compression member 41, an insert body 100, an inner guide channel member 70, and an outer sleeve 180.

In Figure 10, the elongate inner compression member 41 has a proximal end 40 (see, e.g., Fig. 1) and a distal mating end portion 48. The distal mating end portion 48 includes an outer engaging surface 48'.

Figure 10 also shows an exemplary embodiment of the insert body 100 as previously described. The insert body 100 comprises a longitudinally dimensioned distal mating end portion 220 (insert mating end portion 230) having an entry port 222 (see Fig. 8A), a proximal connecting end portion 240 (insert connecting end portion 230) with an exit port 242 that is optionally obliquely angled θ as described above, and an intermediate guide channel portion 230 (insert intermediate portion 230) longitudinally disposed between the insert mating end portion 220 and the insert connecting end portion 240. The entry and exit ports 222, 242, respectively, define an insert body lumen 71' through the longitudinally dimensioned insert mating end portion 220, the longitudinally dimensioned insert intermediate portion 230, and the insert connecting end portion 240.

The insert mating end portion 220 comprises inner and outer diameters 224, 226, respectively, and inner and outer surfaces 225, 227, respectively, for operatively coupling to the inner guide channel member second end portion 77 and/or to the outer sleeve proximal mounting end portion 188. The insert intermediate portion 230 further comprises an inner surface 235 and the insert body lumen 71'. The insert connecting end portion 240 comprises an inner compression member connector 144 secured to the inner compression member distal mating end portion outer engaging surface 48'. While Figure 10 shows a bonding connector 144", the inner compression member connector 144 may also be a crimping connector 144' or combination of a crimping connector 144' and a bonding connector 144". The insert mating end portion 220 is substantially annular, but may also be any other suitable configuration as taught above.

Optionally, the insert mating end portion 220 further comprises at least one securing portion 150 (e.g., securing portions 151-157) for operatively coupling the insert mating end portion 220 to the inner guide channel member second end portion 77 and/or for operatively coupling the insert mating end portion 220 to the outer sleeve proximal mounting end 188. It should be understood that the at least one securing portion 150 may comprise one or more securing portions 151, 152, 153, 154, 155, 156, and 157 or combinations thereof (individually and collectively "securing portion 150").

Although the securing portion 150 is represented with hash marks at one location in Figure 10, there could be more than one securing portion 150 operatively coupling the insert mating end portion 220 to the inner guide channel member second end portion 77. Indeed, a second securing portion 150 at a different location along the longitudinally dimensioned insert mating end portion 220 may operatively couple to the outer sleeve proximal mounting end portion 188. Optionally, the securing portion 150 could substantially cover a majority of the insert mating end portion outer surface 227, such as when the securing portion 150 comprises securing portions 151, 152, 153 (see Figs. 8C-8E, and where 151, 152, and 153 are individually and collectively denoted by the securing portion 150 in Figure 10), for operatively coupling to an inner guide channel member second end portion outer surface 102, an inner guide channel member second end portion outer diameter 75, an outer sleeve inner surface 185, and/or an outer sleeve inner diameter 184. Also, an insert securing portion 157 (see Fig. 8H) and outer sleeve internal threads 186 (see Fig. 8L) may operatively couple the insert mating end portion 220 to outer sleeve proximal mounting end 188 (see Figs. 10B and 10C) while a second securing portion (e.g., 151, 152, 153 (see Figs. 8C-8E) by way of example and not by way of limitation) may operatively couple the insert mating end portion 220 to the inner guide channel member second end portion outer surface 102. Indeed, a securing portion 153 (see Fig. 8E) may operatively couple the insert mating end portion outer surface 227 to the outer sleeve inner surface 185 by a wedge effect, a press-fit-tight configuration, or surface roughness, while a securing portion 152 (see Fig. 8D) or securing portion 156 (see Fig. 8I) may operatively couple the insert mating end portion inner surface 225 to the inner member second end portion outer surface 102 (wherein 152 and 153 are individually and collectively denoted by the securing portion 150 in Figure 10).

In Figure 10, an inner guide channel member has a distal first end portion 78 (see Fig. 5) and a proximal second end portion 77. The first end portion 78 includes a wire guide entry port 72 (see Fig. 5) and the second end portion 77 includes a wire guide exit port 73, the exit and entry ports 72, 73, respectively, defining a wire guide channel 71 therebetween. Figure 10 also shows the inner guide channel member second end portion 77 having an outer surface 102 and an outer diameter 75 substantially similar to the insert mating end portion inner diameter 224. The inner guide channel member second end portion 77 optionally may be concentrically disposed within an insert mating end portion entry port 222 (see Fig. 8A) so that the inner guide channel member second end portion 77 and insert mating end portion 220 optionally may operatively couple via an wedge effect, friction fit, or a press-fit-tight configuration. The inner guide channel member second end portion 77 optionally extends proximally within the insert body lumen 71' to one or more securing portions 150 of the insert mating end portion 220.

In Figure 10, the outer sleeve 180 proximal mounting end portion 188 is disposed about the insert mating end portion 220 (e.g., the insert mating end portion 220 is at least partially received within the outer sleeve lumen 181). The insert mating end portion 220 is disposed about the inner guide channel member second end portion 77 (e.g., the inner guide channel member second end portion 77 is at least partially received within the insert body lumen 71').

Embodiments also comprise a junction 190 for operatively coupling the insert mating end portion 220, the inner guide channel member second end portion 77, and the outer sleeve mounting end portion 188. One embodiment of a junction 190 is a nesting configuration, whereby at least a portion of the inner guide channel member second end portion 77 fits within at least a portion of the insert both lumen 71' and the insert mating end portion 220 in turn fits within at least a portion of the outer sleeve lumen 181. In other words, the insert mating end portion inner surface 225 is operatively coupled to the inner guide channel member second end portion outer surface 102 by a wedge effect, friction fit, or a press-fit-tight configuration having a pullout strength of at least 5 newtons and preferably a pullout strength of at least 20 newtons, while the insert mating end portion outer surface 227 is operatively coupled to the outer sleeve inner surface 185 by a wedge effect, friction fit, or a press-fit-tight configuration having a pullout strength of at least 5 newtons and preferably a pullout strength of at least 20 newtons. In one embodiment, the strength of the junction 190 may be measured by providing a pullout or pull apart strength of the insert mating end portion 220 and the inner guide channel member second end portion 77 of at least 5 newtons and preferably at least 20 newtons, and a pullout or pull apart strength of the insert mating end portion 220 and the outer sleeve proximal mounting end portion 188 of at least 5 newtons and preferably at least 20 newtons.

The junction 190 according to one nesting embodiment comprises an inner guide channel member second end portion outer diameter 75 being substantially similar to (or tapering to a diameter slightly greater than) an insert mating end portion inner diameter 224 and being disposed within the insert body lumen 71' at or near the insert mating end portion 220, while an insert mating end portion outer diameter 226 is substantially similar to (or tapering to a diameter slightly greater than) an outer sleeve inner diameter 184 and is disposed within the outer sleeve lumen 181 at or near the outer sleeve proximal mounting end portion 188. Optionally, the inner guide channel member second end portion outer surface 102, the insert connecting end portion inner surface 245, and/or the outer sleeve inner surface 185 comprise a surface roughness (e.g., sandblasting, etching, knurling, grinding, threading, milling, drilling, chemical treatment, or other roughing preparation) sufficient to improve the nested fit and pullout strength of the junction 190. Optionally, the inner guide channel member second end portion outer surface 102, the insert connecting end portion inner surface 245, the insert connecting end portion outer surface 247, and/or the outer sleeve inner surface 185 may further comprise any one or more or combinations of a securing portion described above 151, 152, 153 (see Figs. 8C-8E) in order to improve the nested fit and pullout strength of the junction 190. Furthermore, the junction 190 may comprise glue, adhesives, resins, chemical bonding materials, a melt bond, and/or combinations thereof at one or more of the inner member outer surface 102, the insert connecting end portion inner surface 245, the insert connecting end portion outer surface 247, and/or the outer sleeve inner surface 185 for operatively coupling the insert mating end portion 220, the inner guide channel member second end portion 77, and/or the outer sleeve mounting end portion 188.

In another embodiment of a junction 190, the outer sleeve 180 may be bonded directly to the inner guide channel member 70 even though the insert body 100 is sandwiched between the outer sleeve inner surface 185 and the inner guide channel member second end portion outer surface 102. For instance, the outer sleeve inner surface 185 may comprise a melt bonding material and the insert mating end portion 220 may comprise a slotted securing portion 154 (see Fig. 8F) and/or a perforated securing portion 155 (see Fig. 8G) extending between the insert mating end portion inner and outer surfaces 225, 227, respectively. Therefore, the outer sleeve inner surface 185 may be directly bonded to the inner guide channel member second end portion outer surface 102 via a melt bond that extends from the outer sleeve inner surface 185, through the slot and/or perforation in the insert mating end portion 220, and to the inner guide channel member second end portion outer surface 102. The junction 190 according to this embodiment helps to form a more solid connection and provides additional strength between the insert mating end portion 220, the inner guide channel member 70, and the outer sleeve 180. A solid-state bond results from using a suitable form of heat for melting and then solidifying (e.g., fusing and/or cross-linking bonds formed at the melt bonded material interfaces) the material of the outer sleeve inner surface 185 and the inner guide channel member second end portion outer surface 102 at the junction 190. Bonded as thus, the inner guide channel member second end portion outer surface 102 and insert mating end portion inner surface 225 are operatively coupled to have a pullout strength of at least 5 newtons and preferably a pullout strength of at least 20 newtons, while the insert mating end portion outer surface 227 and outer sleeve inner surface 185 are operatively coupled to have a pullout strength of at least 5 newtons and preferably a pullout strength of at least 20 newtons. In one embodiment, the strength of the junction 190 may be measured by providing a pullout or pull apart strength of the insert mating end portion 220 and the inner guide channel member second end portion 77 of at least 5 newtons and preferably at least 20 newtons, and a pullout or pull apart strength of the insert mating end portion 220 and the outer sleeve proximal mounting end portion 188 of at least 5 newtons and preferably at least 20 newtons.

Figure 10A schematically shows a cross sectional view of a system distal portion 13 of Figure 10 taken along the lines A-A wherein a junction 190 is further designated as a junction 191, 192, and 193 comprising an insert body having one or more of a variety of securing portions 150-155, a melt bond 47, and/or a bonding material (e.g., glue, adhesive, resin, chemical bonding materials, melt bond, and the like) as taught herein above for operatively coupling the insert mating end portion inner surface 225 of the insert mating end portion 220 to the outer surface 102 of the inner guide channel member second end portion 77 and/or insert mating end portion outer surface 227 to the inner surface 185 of the outer sleeve mounting end portion 188. The junctions 191, 192, and 193 comprising said securing member 150-155 operatively couple the inner guide channel member second end portion 77 to the insert mating end portion 220 and/or the outer sleeve proximal mounting end portion 188 to the insert mating end portion 220 and/or all three (e.g., the inner guide channel member second end portion 77, the insert mating end portion 220, and the proximal outer sleeve mounting end portion 188) to have a strength of at least 5 newtons and preferably a strength of at least 20 newtons. In one embodiment, the strength of the junctions 191, 192, 193 and/or securing members 150-155 may be measured by providing a pullout or pull apart strength of the insert mating end portion 220 and the inner guide channel member second end portion 77 of at least 5 newtons and preferably at least 20 newtons, and a pullout or pull apart strength of the insert mating end portion 220 and the outer sleeve proximal mounting end portion 188 of at least 5 newtons and preferably at least 20 newtons.

A melt bonded junction 191, as schematically shown in Figure 10A, may comprise a melt bond 47 that operatively couples the inner guide channel member second end portion 77, the insert mating end portion 220, and the outer sleeve mounting end portion 188. By way of example only and not by way of limitation, the melt bond 47 may be formed through an insert slotted securing portion 154 and/or an insert perforated securing portion 155 in the insert mating end portion 220 to thereby join the outer sleeve inner surface 185 to the inner guide channel member second end portion outer surface 102.

A bonding junction 192, as schematically shown in Figure 10A, may comprise a bonding material (e.g., glue, adhesive, resin, chemical bonding materials, melt bond, and the like) on the inner guide channel member second end portion outer surface 102, on the insert mating end portion inner surface 225, or on both surfaces 102, 225. It should also be understood that the bonding junction 192 may operatively couple the insert mating end portion 220 and the outer sleeve mounting end portion 188 by placing the bonding material on the insert mating end portion outer surface 227, on the outer sleeve inner surface 185, or on both surfaces 127, 185.

A connecting junction 193, as schematically shown in Figure 10A, may comprise securing members 151, 152, and/or 153 (see Figs. 8C-8E) on the outer sleeve inner surface 185, on the insert mating end portion outer surface 227, or both for operatively coupling the outer sleeve mounting end portion 188 and insert mating end portion 220 via a nesting configuration, a wedge effect, a press-fit-tight configuration, and/or a crimping technique between the outer sleeve inner surface 185 and the insert mating end portion outer surface 227. It should also be understood that a crimping junction 193 may operatively couple the inner guide channel member second end portion outer surface 102 and insert mating end portion inner surface 225, and/or a crimping technique for operatively coupling the inner guide channel member second end portion 77 and the insert mating end portion 220. In addition, a connecting junction 193 may comprise placing a bonding material (e.g., glue, adhesive, resin, chemical bonding materials, melt bond, and the like) on the outer surface 102 of the inner guide channel member second end portion 77, the insert mating end portion inner surface 225, the insert mating end portion outer surface 227, and/or the outer sleeve inner surface 185.

This cross sectional view also shows an outer guide channel member 80 having a guide channel 81 with an outer sleeve proximal mounting end portion 188 disposed therein. The outer sleeve mounting end portion 188 has a lumen 181 having an insert mating end portion 220 disposed therein. The insert mating end portion 220 has a lumen 71' having an inner guide channel member second end portion 77 disposed therein. The inner guide channel member second end portion 77 comprises a channel 71. As with the other drawings, the channel 81, lumen 181, lumen 71, and lumen 71' are not to scale. Instead, they are emphasized for clarity in order to show the proximal outer sleeve mounting end portion 188 disposed about the distal insert mating end portion 220, and the insert mating end portion 220 disposed about the inner guide channel member second end portion 77. Assembled as thus, the inner guide channel member second end portion outer surface 102 may substantially abut the insert mating end portion inner surface 225, and the insert mating end portion outer surface 227 may substantially abut the outer sleeve inner surface 185.

Figure 10B is a longitudinal side view, broken away, of Figure 10, according to another alternative embodiment of a junction 194, whereby the inner guide channel member second end portion 77 screws into the insert mating end portion 220 and/or the insert mating end portion 220 screws into the outer sleeve mounting end portion 188. In other words, one junction 194 comprises an insert securing portion 156 for operatively coupling external thread(s) 76 of an inner guide channel member second end portion 77, and another junction 194 comprises internal threads 186 of an outer sleeve mounting end portion 188 for operatively coupling the insert securing portion 157 of the insert mating end portion 220. The junction 194 provides a strength of at least 5 newtons and preferably a strength of at least 20 newtons. Also, it should be understood that the insert securing portion 156 may operatively couple the inner guide channel member second end portion 77 while the insert mating end portion 220 operatively couples the outer sleeve mounting end portion 188 by some other means taught herein; conversely, the sleeve internal threading 186 may operatively couple the insert securing portion 157 while the insert mating end portion 220 operatively couples the inner guide channel member second end portion 77 by some other means taught herein. In one embodiment, the strength of the junction 194 and/or securing portions 156-157 may be measured by providing a pullout or pull apart strength of the insert mating end portion 220 and the inner guide channel member second end portion 77 of at least 5 newtons and preferably at least 20 newtons, and a pullout or pull apart strength of the insert mating end portion 220 and the outer sleeve proximal mounting end portion 188 of at least 5 newtons and preferably at least 20 newtons.

The junction may further comprise a bonding connector 158 on one or more of the insert securing portions 156, 157, the outer sleeve internal threading 186, and/or the inner guide channel member external threading 76. For example, the bonding connector 158 may comprise glue, adhesives, resins, chemical bonding materials, or combinations thereof applied to the insert securing portion 156 (see Fig. 8I), an at least one external thread 76 of an inner guide channel member second end portion 77 (see Fig. 10B), or both for operatively coupling the inner guide channel member second end portion 77 and the insert mating end portion 220 (see Figs. 10B and 10C). Likewise, the bonding connector 158 may be applied to the insert securing portion 157 (see Fig. 8H), the one or more internal threads 186 of the outer sleeve 180 (see Fig. 10B), or both for operatively coupling the insert mating end portion 220 and the outer sleeve mounting end portion 188 (see Figs. 10B and 10C). As another embodiment of a bonding connector 158, the inner guide channel member second end portion 77 and insert mating end portion 220 may be brought together and operatively coupled with welding (laser, spot, etc.), soldering, or brazing. Similarly, the outer sleeve mounting end portion 188 and insert mating end portion 220 may be brought together and operatively coupled by welding (laser, spot, etc.), soldering, or brazing.

Figure 10C is a longitudinal side view, broken away, of the junction 194 taken along lines A-A according to the embodiment shown in Figure 10B. The insert mating end portion 220 comprises the at least one insert securing portion 156 for operatively coupling the at least one external thread 76 of an inner guide channel member second end portion 77, while the insert securing portion 157 operatively couples at least one internal thread 186 of an outer sleeve mounting end portion 188.

Figure 10D is a longitudinal side view, broken away, of a system distal portion 13 of Figure 10 according to an alternative embodiment of an insert body 100 having an insert connecting end portion 240 comprising a crimping connector 144' configured to operatively couple an engaging surface 48' of an inner compression member distal mating end portion 48. The crimping connector 144' wraps around the inner compression member distal mating end portion 48 and crushes against the engaging surface 48' to provide a pullout strength of at least 5 newtons, and preferably a pullout strength of at least 20 newtons. Furthermore, the crimping connector 144' moves the inner compression member distal mating end portion 48 toward the insert central axis to ensure that a wire guide, catheter, or other medical device or tool (not shown) does not enter an outer sheath passageway 59 between an inner surface 57 of the outer sheath 50 and an outer surface 147 of the inner compression member 41. Yet another aspect to the crimping connector 144' according to this embodiment is a clearance 64 provided between the outer guide channel member inner surface 63 and the outer sleeve outer surface 189. This clearance 64 prevents or reduces drag by the outer sleeve 180 when the outer guide channel member 80 retracts proximally to deploy the stent 17 (not shown), which may impede the stent deployment process.

Figure 10E is a longitudinal side view, broken away, of a system distal portion 13 of Figure 10 according to an alternative embodiment of an insert body 100 having an insert connecting end portion 240 comprising a bonding connector 144" configured to operatively couple an engaging surface 48' of an inner compression member distal mating end portion 48. The bonding connector 144" comprises one or more laser welds and/or spot welds for operatively coupling the insert connecting end portion inner surface 245 and the inner compression member distal mating end portion engaging surface 48' to provide a pullout strength of at least 5 newtons and preferably a pullout strength of at least 20 newtons. Furthermore, the bonding connector 144" moves the inner compression member distal mating end portion 48 toward the insert central axis to ensure that a wire guide, catheter, or other medical device or tool (not shown) does not enter an outer sheath passageway 59 between an inner surface 57 of the outer sheath 50 and an outer surface 147 of the inner compression member 41. Yet another aspect to the bonding connector 144" according to this embodiment is a clearance 64 provided between the outer guide channel member inner surface 63 and the sleeve outer surface 189. This clearance 64 prevents or reduces drag by the outer sleeve 180 when the outer guide channel member 80 retracts proximally to deploy the stent (not shown), which may impede the stent deployment process.

It is intended that the foregoing detailed description of an internal cannulated joint for use with medical device delivery systems and medical devices, and methods of forming the internal cannulated joint, be regarded as illustrative rather than limiting, and that it be understood that it is the following claims, including all equivalents, that are intended to define the scope of this invention. Terms are to be given their reasonable plain and ordinary meaning. Also, the embodiment of any figure and features thereof may be combined with the embodiments depicted in other figures. Other features known in the art and not inconsistent with the structure and function of the present invention may be added to the embodiments.

While particular elements, embodiments, and applications of the present invention have been shown and described, it will be understood, of course, that the invention is not limited thereto since modifications may be made by those skilled in the art, particularly in light of the foregoing teachings. Therefore, it is therefore contemplated by the appended claims to cover such modifications as incorporate those features which come within the scope of the invention.

## Claims

1. An internal joint for use in a medical device delivery system that employs a catheter, the internal joint comprising:
an elongate inne compression member (41) having a proximal end portion (40) and a distal mating end portion (48), the inner compression member mating end portion having an engaging surface (48').
an inner guide channel member (70) having a first end portion (78), a second end portion (77), and defining a channel therebetwen (71), the second end portion having inner (101) and outer (102) surfaces; and
an elongate insert body (100) comprising a distal mating end portion (220) having a first connection (221) operatively coupled to the inner guide channel member second end portion (77) and a proximal connecting end portion (240) having a second connection (241) operatively coupled to the inner compression member distal mating end portion (48), such that the compression member distal mating end portion is proximally displaced from the inner guide channel member second end portion.

2. The joint of claim 1, wherein at least one of the first connection (221) and second connection (241) comprises a melt bond.

3. The joint of claim 2 wherein the insert mating end portion (220) comprises an entry port (222) and inner (225) and outer (227) surfaces, the insert connecting end portion (240) comprises an exit port (242) and inner (245) and outer (247) surfaces, and the ports define a lumen (71') extending therebetween in fluid communication with the channel.

4. The joint of claim 3 wherein the first connection (221) comprises the melt bond and operatively couples the insert mating end portion inner surface (225) and the inner guide channel member second end portion outer surface (227).

5. The joint of claim 3 wherein the first connection comprises the melt bond and operatively couples the insert mating end portion outer surface (227) and the inner guide channel member second end portion inner surface (101).

6. The joint of claim 3 wherein the second connection (249) comprises the melt bond and operatively couples the inner compression member distale mating end portion outer engaging surface (481) and one of the insert connecting end portion inner and outer surfaces.

7. The joint of claim 2 further comprising a system proximal portion (12) operatively coupled to the inner compression member proximal end (40), the system proximal portion further comprising a handle (30), wherein the elongate inner compression member extends at least about 50.0 cm from the handle to the inner guide channel member (70).

8. The joint of claim 7 wherein the inner guide channel member (70) further comprises a deployment device mounting region (90) for deploying one of a stent, prosthetic valve device, and other implantable article inside a patient's body.

9. The joint of claim 2 wherein the melt bond comprises a melt-bonding material selected from the group consisting of nylon, nylon natural tubing, polyether block amide, polyetheretherketone, thermoplastic, thermosetting plastic, resin, polypropylene, polyethylene, polyester, polyamide, ionomer, polycarbonate, polyphenylene oxide, polyphenylene sulphide, acrylic, liquid crystal polymer, polyolefin, polyethylene acrylate acid, polyvinylidene fluoride, polyvinyl, polyvinyl chloride, and polytetrafluorethylene.

10. The joint of claim 2 wherein the melt bond provides a pull apart strength of at least 5 Newtons and preferably a pull apart strength of at least 20 Newtons.

11. The joint of claim 1, wherein the distal insert mating end portion (220) is implanted into the inner guide channel member second end portion (77).

12. The joint of claim 11 wherein the insert connecting end portion (240) further comprises an inner compression member connector (144) that operatively couples the insert connecting end portion (240) and the inner compression member distal mating end portion engaging surface (48').

13. The joint of claim 11 wherein the inner guide channel member first end portion (78) has an entry port (72) and the inner guide channel member second end portion (77) has an exit port (73).

14. The joint of claim 11 wherein the insert mating end portion has the inner surface (225) and the outer surface (227), and wherein the insert mating end portion is implanted between an inner guide channel member second end portion inner surface (101) and the inner guide channel member second end portion outer surface (102).

15. The joint of claim 14 wherein the implanted insert mating end portion forms an inner guide channel member second end portion inner contacting interface (103) and an inner guide channel member second end portion outer contacting interface (104).

16. The joint of claim 15 wherein the inner guide channel member second end portion inner contacting interface (103) is bonded to the insert mating end portion inner surface (225).

17. The joint of claim 15 wherein the inner guide channel member second end portion outer contacting interface (104) is bonded to the insert mating end portion outer surface (227).

18. The joint of claim 11 wherein the insert mating end portion (220) and insert connecting end portion (240) define a lumen (71) therebetween.

19. The joint of claim 18 wherein the insert mating end portion includes an entry port (222), and wherein the insert connecting end portion includes an exit port (242).

20. The joint of claim 1, wherein said insert distal mating end portion has an inner diameter (224), an outer diameter (226), and a lumen (71');
said inner guide channel member second end portion (77) has an outer diameter substantially similar to the insert mating end portion inner diameter and being disposed within the insert distal mating end portion lumen;
said joint further comprising an outer sleeve (180) having a first end portion (187) and a mounting end portion (188) and a sleeve lumen extending therethrough, the outer sleeve mounting end portion having an inner diameter substantially similar to the insert mating end portion outer diameter and being concentrically disposed about the insert mating end portion; and
wherein the insert mating end portion, the inner guide channel member second end portion, and the outer sleeve mounting end portion are operatively coupled at a junction.

21. The joint of claim 20 wherein the insert mating end portion further comprises inner and outer surfaces and at least one securing portion (151, 152).

22. The joint of claim 21 wherein the inner guide channel member outer surface (102) is substantially aligned with the at least one insert mating end portion securing portion.

23. The joint of claim 21 wherein the outer sleeve further comprises an inner surface (185) formed of a melt bonding material and being substantially aligned with at least one insert mating end portion securing aperture.

24. The joint of claim 20 wherein the insert mating end portion includes the entry port (222), and wherein the insert connecting end portion includes the exit port (242).

25. A medical device delivery system comprising an internal joint according to claim 1, the delivery system further comprising:
an elongate longitudinal flexible middle section delivery device (14), extending intermediate a system proximal portion (12) and a system distal portion (13), the middle section delivery device having an outer sheath (50) containing a passageway said elongate compression member (41) extending through the outer sheath passageway;
said inner guide channel member (70) being arranged at the system distal portion, , the inner guide channel member first end portion (78) including a wire guide entry port (72) and the second end portion including a wire guide exit port (73), the ports defining a wire guide channel therebetween (71);
an outer guide channel member (80) axially movable relative to the inner guide channel member at the system distal portion, the outer guide channel member including a distal first end portion (88) having an opening (89) proximal second end portion (87) having an exit port (83), first end opening and second end exit port defining a guide channel, and configured to have a stepped profile comprising a first outer diameter (84) intermediate the outer guide channel member first and second end portions and a second smaller outer diameter (85) located at or near the outer guide channel member second end portion;
a self-expanding deployment device mounting region (90) disposed at the system distal portion within the outer guide channel member, the mounting region including a proximal restraint (93), an inner guide channel member stent platfom (91), and an outer guide channel member inner surface (92), and
a transition region (60) arranged at the system distal portion proximal to the self-expanding deployment device mounting region, wherein the inner guide channel member exit port is in communication with the outer guide channel member exit port, and having a breech position opening (65) located proximal to the inner guide channel member exit port.

26. The system of claim 25 wherein the insert body further comprises an entry port (222) at or near the insert mating end portion (220), an exit port (242) at or near the insert connecting end portion (240), and the insert body entry and exit ports defining a lumen therebetween in fluid communication with the guide channel (71) of the inner guide channel member.

27. The system of claim 25 wherein a junction (190) operatively couples the insert mating end portion and the inner guide channel member second end portion.

## Patentansprüche

1. Internes Verbindungsstück zur Verwendung in einem medizinischen Ausgabesystem, das einen Katheter verwendet, wobei das interne Verbindungsstück Folgendes umfasst: ein längliches inneres Kompressionselement (41) mit einem proximalen Endabschnitt (40) und einem distalen Gegenendabschnitt (48), wobei der Gegenendabschnitt des inneren Kompressionselements eine Eingriffsfläche (48') aufweist; ein inneres Führungskanalelement (70), das einen ersten Endabschnitt (78) und einen zweiten Endabschnitt (77) aufweist und einen Kanal (71) dazwischen definiert, wobei der zweite Endabschnitt Innenseiten (101) und Außenseiten (102) aufweist; und einen länglichen Einsatzkörper (100), der einen distalen Gegenendabschnitt (220) mit einer ersten Verbindung (221), die mit dem zweiten Endabschnitt (77) des inneren Führungskanalelements in Wirkverbindung steht, und einen proximalen verbindenden Endabschnitt (240) mit einer zweiten Verbindung (241), die mit dem distalen Gegenendabschnitt (48) des inneren Kompressionselements in Wirkverbindung steht, umfasst, so dass der distale Gegenendabschnitt des Kompressionselements proximal vom zweiten Endabschnitt des inneren Führungskanalelements verschoben wird.

2. Verbindungsstück nach Anspruch 1, worin die erste Verbindung (221) und/oder die zweite Verbindung (241) eine Schmelzklebeverbindung umfassen.

3. Verbindungsstück nach Anspruch 2, worin der Gegenendabschnitt (220) des Einsatzes eine Eingangsöffnung (222) und Innenseiten (225) und Außenseiten (227) umfasst, wobei der verbindende Endabschnitt (240) des Einsatzes eine Ausgangsöffnung (242) und Innenseiten (245) und Außenseiten (247) umfasst, und wobei die Öffnungen ein Lumen (71') definieren, das sich zwischen ihnen und in Fluidverbindung mit dem Kanal erstreckt.

4. Verbindungsstück nach Anspruch 3, worin die erste Verbindung (221) die Schmelzklebeverbindung umfasst und die Innenseite (225) des Gegenendabschnittes des Einsatzes mit der Außenseite (227) des zweiten Endabschnittes des inneren Führungskanalelements in Wirkverbindung bringt.

5. Verbindungsstück nach Anspruch 3, worin die erste Verbindung die Schmelzklebeverbindung umfasst und die Außenseite (227) des Gegenendabschnittes des Einsatzes mit der Innenseite (101) des zweiten Endabschnittes des inneren Führungskanalelements in Wirkverbindung bringt.

6. Verbindungsstück nach Anspruch 3, worin die zweite Verbindung (249) die Schmelzklebeverbindung umfasst und die äußere Eingriffsfläche (481) des distalen Gegenendabschnittes des inneren Kompressionselements mit der Innen- oder Außenseite des verbindenden Endabschnittes des Einsatzes in Wirkverbindung bringt.

7. Verbindungsstück nach Anspruch 2, das ferner einen proximalen Systemabschnitt (12) umfasst, der mit dem proximalen Ende (40) des inneren Kompressionselements in Wirkverbindung steht, wobei der proximale Systemabschnitt ferner einen Handgriff (30) umfasst, worin sich das längliche innere Kompressionselement über zumindest 50,0 cm vom Handgriff zum inneren Führungskanalelement (70) erstreckt.

8. Verbindungsstück nach Anspruch 7, worin das innere Führungskanalelement (70) ferner eine Ausgabevorrichtung-Befestigungsregion (90) zum Ausgeben eines Stents, einer Klappenprothese oder anderer implantierbarer Gegenstände im Körper eines Patienten umfasst.

9. Verbindungsstück nach Anspruch 2, worin die Schmelzklebeverbindung ein aus der Gruppe von Nylon, Nylon-Naturschlauch, Polyetherblockamid, Polyetheretherketon, Thermoplastik, Duroplastik, Harz, Polypropylen, Polyethylen, Polyester, Polyamid, Ionomer, Polycarbonat, Polyphenylenoxid, Polyphenylensulfid, Acryl, Flüssigkristallpolymer, Polyolefin, Polyethylenacrylatsäure, Polyvinylidenfluorid, Polyvinyl, Polyvinylchlorid und Polytetrafluorethylen ausgewähltes Schmelzklebematerial umfasst.

10. Verbindungsstück nach Anspruch 2, worin die Schmelzklebeverbindung eine Auseinanderziehkraft von zumindest 5 Newton und vorzugsweise eine Auseinanderziehkraft von zumindest 20 Newton liefert.

11. Verbindungsstück nach Anspruch 1, worin der Gegenendabschnitt (220) des distalen Einsatzes in den zweiten Endabschnitt (77) des inneren Führungskanalelements implantiert wird.

12. Verbindungsstück nach Anspruch 11, worin der verbindende Endabschnitt (240) des Einsatzes ferner eine innere Kompressionselementverbindung (144) umfasst, die den verbindenden Endabschnitt (240) des Einsatzes mit der Eingriffsfläche (48') des distalen Gegenendabschnittes des inneren Kompressionselements in Wirkverbindung bringt.

13. Verbindungsstück nach Anspruch 11, worin der erste Endabschnitt (78) des inneren Führungskanalelements eine Eingangsöffnung (72) aufweist und der zweite Endabschnitt (77) des inneren Führungskanalelements eine Ausgangsöffnung (73) aufweist.

14. Verbindungsstück nach Anspruch 11, worin der Gegenendabschnitt des Einsatzes die Innenseite (225) und die Außenseite (227) aufweist und worin der Gegenendabschnitt des Einsatzes zwischen einer Innenseite (101) des zweiten Endabschnittes des inneren Führungskanalelements und der Außenseite (102) des zweiten Endabschnittes des inneren Führungskanalelements implantiert wird.

15. Verbindungsstück nach Anspruch 14, worin der implantierte Gegenendabschnitt des Einsatzes eine inneren Kontaktgrenzfläche (103) des zweiten Endabschnittes des inneren Führungskanalelements und eine äußere Kontaktgrenzfläche (104) des zweiten Endabschnittes des inneren Führungskanalelements bildet.

16. Verbindungsstück nach Anspruch 15, worin die innere Kontaktgrenzfläche (103) des zweiten Endabschnittes des inneren Führungskanalelements mit der Innenseite (225) des Gegenendabschnittes des Einsatzes verbunden ist.

17. Verbindungsstück nach Anspruch 15, worin die äußere Kontaktgrenzfläche (104) des zweiten Endabschnittes des inneren Führungskanalelements mit der Außenseite (227) des Gegenendabschnittes des Einsatzes verbunden ist.

18. Verbindungsstück nach Anspruch 11, worin der Gegenendabschnitt (220) des Einsatzes und der verbindende Endabschnitt (240) des Einsatzes ein Lumen (71) zwischen sich definieren.

19. Verbindungsstück nach Anspruch 18, worin der Gegenendabschnitt des Einsatzes eine Eingangsöffnung (222) aufweist und worin der verbindende Endabschnitt des Einsatzes eine Ausgangsöffnung (242) aufweist.

20. Verbindungsstück nach Anspruch 1, worin der distale Gegenendabschnitt des Einsatzes einen Innendurchmesser (224), einen Außendurchmesser (226) und ein Lumen (71') aufweist; wobei der zweite Endabschnitt (77) des inneren Führungskanalelements einen Außendurchmesser aufweist, der dem Innendurchmesser des Gegenendabschnittes des Einsatzes im Wesentlichen ähnelt und im Lumen des distalen Gegenendabschnittes des Einsatzes angeordnet ist; wobei das Verbindungsstück ferner eine Außenhülse (180) mit einem ersten Endabschnitt (187) und einem Gegenendabschnitt (188) und einem sich dort hindurch erstreckenden Hülsenlumen umfasst, wobei der Befestigungsendabschnitt der Außenhülse einen Innendurchmesser aufweist, der dem Außendurchmesser des Gegenendabschnittes des Einsatzes im Wesentlichen ähnelt und konzentrisch um den Gegenendabschnitt des Einsatzes angeordnet ist; und worin der Gegenendabschnitt des Einsatzes, der zweite Endabschnitt des inneren Führungskanalelements und der Befestigungsendabschnitt der Außenhülse an einer Verbindungsstelle in Wirkverbindung miteinander stehen.

21. Verbindungsstück nach Anspruch 20, worin der Gegenendabschnitt des Einsatzes ferner Innen- und Außenseiten und zumindest einen Fixierungsabschnitt (151, 152) umfasst.

22. Verbindungsstück nach Anspruch 21, worin die Außenseite (102) des inneren Führungskanalelements im Wesentlichen mit dem zumindest einen Fixierungsabschnitt des Gegenendabschnittes des Einsatzes ausgerichtet ist.

23. Verbindungsstück nach Anspruch 21, worin die Außenhülse ferner eine aus einem Schmelzklebematerial gebildete Innenseite (185) umfasst, die im Wesentlichen mit zumindest einer Fixierungsöffnung des Gegenendabschnittes des Einsatzes ausgerichtet ist.

24. Verbindungsstück nach Anspruch 20, worin der Gegenendabschnitt des Einsatzes die Eingangsöffnung (222) aufweist und worin der verbindende Endabschnitt des Einsatzes die Ausgangsöffnung (242) aufweist.

25. Medizinisches Vorrichtungsausgabesystem, das ein internes Verbindungsstück nach Anspruch 1 umfasst, wobei das Ausgabesystem ferner Folgendes umfasst:
eine längliche, ins Längsrichtung flexible Mittelabschnitt-Ausgabevorrichtung (14), die sich zwischen einem proximalen Systemabschnitt (12) und einem distalen Systemabschnitt (13) erstreckt, wobei die Mittelabschnitt-Ausgabevorrichtung eine Außenschleuse (50) aufweist, die einen Durchgang enthält, wobei sich das längliche Kompressionselement (41) durch den Durchgang in der Außenschleuse erstreckt; wobei das innere Führungskanalelement (70) am distalen Systemabschnitt angeordnet ist, der erste Endabschnitt (78) des inneren Führungskanalelements eine Führungsdraht-Eingangsöffnung (72) aufweist und der zweite Endabschnitt eine Führungsdraht-Ausgangsöffnung (73) aufweist, wobei die Öffnungen einen Führungsdrahtkanal (71) zwischen sich definieren; ein äußeres Führungskanalelement (80), das axial relativ zum inneren Führungskanalelement am distalen Systemabschnitt beweglich ist, wobei das äußere Führungskanalelement einen ersten distalen Endabschnitt (88) mit einer Öffnung (89) und einen zweiten proximalen Endabschnitt (87) mit einer Ausgangsöffnung (83) aufweist, wobei die erste Endöffnung und die Ausgangsöffnung im zweiten Ende einen Führungskanal definieren, der so konfiguriert ist, dass er ein abgestuftes Profil mit einem ersten Außendurchmesser (84) zwischen den ersten und zweiten Endabschnitten des äußeren Führungskanalelements und einem zweiten kleineren Außendurchmesser (85) an dem oder in der Nähe des zweiten Endabschnittes des äußeren Führungskanalelements umfasst; eine selbstexpandierende Ausgabevorrichtung-Befestigungsregion (90) am distalen Systemabschnitt innerhalb des äußeren Führungskanalelements; wobei die Befestigungsregion eine proximale Halterung (93), eine innere Führungskanalelement-Stentplattform (91) und eine Innenseite (92) des äußeren Führungskanalelements aufweist; und eine Übergangsregion (60) am distalen Systemabschnitt proximal zur selbstexpandierenden Ausgabevorrichtung-Befestigungsregion, worin die Ausgangsöffnung des inneren Führungskanalelements mit der Ausgangsöffnung des äußeren Führungskanalelements in Verbindung steht, und eine Verschlusspositionsöffnung (65) proximal zur Ausgangsöffnung des inneren Führungskanalelements aufweist.

26. System nach Anspruch 25, worin der Einsatzkörper ferner eine Eingangsöffnung (222) an oder in der Nähe des Gegenendabschnittes (220) des Einsatzes, eine Ausgangsöffnung (242) an oder in der Nähe des verbindenden Endabschnittes (240) des Einsatzes aufweist und wobei die Eingangs- und Ausgangsöffnungen des Einsatzkörpers zwischen sich ein Lumen definieren, das mit dem Führungskanal (71) des inneren Führungskanalelements in Fluidverbindung steht.

27. System nach Anspruch 25, worin eine Verbindungsstelle (190) den Gegenendabschnitt des Einsatzes mit dem zweiten Endabschnitt des inneren Führungskanalelements in Wirkverbindung bringt.

## Revendications

1. Joint interne pour un système de mise en place de dispositifs médicaux qui utilise un cathéter, le joint interne comportant :
un organe (41) de compression intérieur allongé pourvu d'une portion (40) d'extrémité proximale et d'une portion (48) d'extrémité distale correspondante, la portion d'extrémité correspondante de l'organe de compression intérieur présentant une surface (48') d'accrochage ;
un organe (70) de canal de guidage intérieur pourvu d'une première portion (78) d'extrémité, d'une deuxième portion (77) d'extrémité, et définissant un canal entre elles (71), la deuxième portion d'extrémité présentant des surfaces intérieure (101) et extérieure (102) ; et
un corps (100) de pièce rapportée allongé comportant une portion (220) d'extrémité distale correspondante pourvue d'un premier raccordement (221) accouplé de manière fonctionnelle à la deuxième portion (77) d'extrémité de l'organe de canal de guidage intérieur et une portion (240) d'extrémité de raccordement proximale pourvue d'un deuxième raccordement (241) accouplé de manière fonctionnelle à la portion (48) d'extrémité distale correspondante de l'organe de compression intérieur, de telle sorte que la portion d'extrémité distale correspondante de l'organe de compression est déplacée de manière proximale par rapport à la deuxième portion d'extrémité de l'organe de canal de guidage intérieur.

2. Joint selon la revendication 1, dans lequel au moins un desdits premier raccordement (221) et deuxième raccordement (241) comporte un liant fondu.

3. Joint selon la revendication 2, dans lequel la portion (220) d'extrémité correspondante de la pièce rapportée comporte un orifice (222) d'entrée et des surfaces intérieure (225) et extérieure (227), la portion (240) d'extrémité de raccordement de la pièce rapportée comporte un orifice (242) de sortie et des surfaces intérieure (245) et extérieure (247), et les orifices définissent une lumière (71') s'étendant entre elles en communication fluidique avec le canal.

4. Joint selon la revendication 3, dans lequel le premier raccordement (221) comporte le liant fondu et accouple de manière fonctionnelle la surface (225) intérieure de la portion d'extrémité correspondante de la pièce rapportée et la surface (227) extérieure de la deuxième portion d'extrémité de l'organe de canal de guidage intérieur.

5. Joint selon la revendication 3, dans lequel le premier raccordement comporte le liant fondu et accouple de manière fonctionnelle la surface (227) extérieure de la portion d'extrémité correspondante de la pièce rapportée et la surface (101) intérieure de la deuxième portion d'extrémité de l'organe de canal de guidage intérieur.

6. Joint selon la revendication 3, dans lequel le deuxième raccordement (241) comporte le liant fondu et accouple de manière fonctionnelle la surface (48') d'accrochage extérieure de la portion d'extrémité distale correspondante de l'organe de compression intérieur et une des surfaces intérieure et extérieure de la portion d'extrémité de raccordement de la pièce rapportée.

7. Joint selon la revendication 2, comportant en outre une portion (12) proximale de système accouplée de manière fonctionnelle à l'extrémité (40) proximale de l'organe de compression intérieur, la portion proximale de système comportant en outre une poignée (30), dans lequel l'organe de compression intérieur allongé s'étend sur au moins environ 50 cm depuis la poignée jusqu'à l'organe (70) de canal de guidage intérieur.

8. Joint selon la revendication 7, dans lequel l'organe (70) de canal de guidage intérieur comporte en outre une région (90) de montage du dispositif de déploiement pour déployer un extenseur, un dispositif de soupape prothétique, et/ou un autre article implantable à l'intérieur du corps d'un patient.

9. Joint selon la revendication 2, dans lequel le liant fondu comporte un matériau de liaison fondu choisi dans le groupe comportant du nylon, un tube naturel de nylon, un copolymère séquencé d'amide et de polyéther, du polyétherétherkétone, du thermoplastique, du plastique thermodurcissable, de la résine, du polypropylène, du polyéthylène, du polyester, du polyamide, un ionomère, du polycarbonate, de l'oxyde de polyphénylène, un polysulfure de phénylène, de l'acrylique, un polymère à cristaux liquides, une polyoléfine, de l'acide d'acrylate de polyéthylène, du fluorure de polyvinylidène, du polyvinyle, du chlorure de polyvinyle, et du polytétrafluoréthylène.

10. Joint selon la revendication 2, dans lequel le liant fondu fournit une résistance à l'arrachement d'au moins 5 newtons et de préférence une résistance à l'arrachement d'au moins 20 newtons.

11. Joint selon la revendication 1, dans lequel la portion (220) d'extrémité correspondante de la pièce rapportée distale est implantée dans la deuxième portion (77) d'extrémité de l'organe de canal de guidage.

12. Joint selon la revendication 11, dans lequel la portion (240) d'extrémité de raccordement de la pièce rapportée comporte en outre un raccord (144) d'organe de compression intérieur qui accouple de manière fonctionnelle la portion (240) d'extrémité de raccordement de la pièce rapportée et la surface (48') d'accrochage de la portion d'extrémité distale correspondante de l'organe de compression intérieur.

13. Joint selon la revendication 11, dans lequel la première portion (78) d'extrémité de l'organe de canal de guidage intérieur présente un orifice (72) d'entrée et la deuxième portion (77) d'extrémité de l'organe de canal de guidage intérieur présente un orifice (73) de sortie.

14. Joint selon la revendication 11, dans lequel la portion d'extrémité correspondante de la pièce rapportée est pourvue de la surface (225) intérieure et de la surface (227) extérieure, et dans lequel la portion d'extrémité correspondante de la pièce rapportée est implantée entre une surface (101) intérieure de la deuxième portion d'extrémité de l'organe de canal de guidage intérieur et la surface (102) extérieure de la deuxième portion d'extrémité de l'organe de canal de guidage intérieur.

15. Joint selon la revendication 14, dans lequel la portion d'extrémité correspondante de la pièce rapportée implantée forme une interface (103) de contact intérieure de la deuxième portion d'extrémité de l'organe de canal de guidage intérieur et une interface (104) de contact extérieure de la deuxième portion d'extrémité de l'organe de canal de guidage intérieur.

16. Joint selon la revendication 15, dans lequel l'interface (103) de contact intérieure de la deuxième portion d'extrémité de l'organe de canal de guidage intérieur est liée à la surface (225) intérieure de la portion d'extrémité correspondante de la pièce rapportée.

17. Joint selon la revendication 15, dans lequel l'interface (104) de contact extérieure de la deuxième portion d'extrémité de l'organe de canal de guidage intérieur est liée à la surface (227) extérieure de la portion d'extrémité correspondante de la pièce rapportée.

18. Joint selon la revendication 11, dans lequel la portion (220) d'extrémité correspondante de la pièce rapportée et la portion (240) d'extrémité de raccordement de la pièce rapportée délimitent une lumière (71) entre elles.

19. Joint selon la revendication 18, dans lequel la portion d'extrémité correspondante de la pièce rapportée comprend un orifice (222) d'entrée, et dans lequel la portion d'extrémité de raccordement de la pièce rapportée comprend un orifice (242) de sortie.

20. Joint selon la revendication 1, dans lequel ladite portion d'extrémité distale correspondante de la pièce rapportée a un diamètre (224) intérieur, un diamètre (226) extérieur, et une lumière (71') ;
ladite deuxième portion (77) d'extrémité de l'organe de canal de guidage intérieur a un diamètre extérieur sensiblement analogue au diamètre intérieur de la portion d'extrémité correspondante de la pièce rapportée et est disposée dans la lumière de la portion d'extrémité distale correspondante de la pièce rapportée ;
ledit joint comporte en outre un manchon (180) extérieur pourvu d'une première portion (187) d'extrémité et d'une portion (188) d'extrémité de montage et une lumière de manchon s'étend à travers les deux, la portion d'extrémité de montage du manchon extérieur ayant un diamètre intérieur sensiblement analogue au diamètre extérieur de la portion d'extrémité correspondante de la pièce rapportée et étant disposée de manière concentrique autour de la portion d'extrémité correspondante de la pièce rapportée ; et
dans lequel la portion d'extrémité correspondante de la pièce rapportée, la deuxième portion d'extrémité de l'organe de canal de guidage intérieur, et la portion d'extrémité de montage du manchon extérieur sont accouplées de manière fonctionnelle au niveau d'une jonction.

21. Joint selon la revendication 20, dans lequel la portion d'extrémité correspondante de la pièce rapportée comporte en outre des surfaces intérieure et extérieure et au moins une portion (151, 152) de fixation.

22. Joint selon la revendication 21, dans lequel la surface (102) extérieure de l'organe de canal de guidage intérieur est sensiblement alignée avec ladite au moins une portion de fixation de la portion de l'extrémité correspondante de la pièce rapportée.

23. Joint selon la revendication 21, dans lequel le manchon extérieur comporte en outre une surface (185) intérieure formée d'un matériau de liaison fondu et qui est sensiblement alignée avec au moins une fenêtre de fixation de la portion d'extrémité correspondante de la pièce rapportée.

24. Joint selon la revendication 20, dans lequel la portion d'extrémité correspondante de la pièce rapportée comprend l'orifice (222) d'entrée, et dans lequel la portion d'extrémité de raccordement de la pièce rapportée comprend l'orifice (242) de sortie.

25. Système de mise en place de dispositifs médicaux comportant un joint interne selon la revendication 1, le système de mise en place comportant en outre :
un dispositif (14) de mise en place d'une section moyenne souple longitudinale allongée s'étendant entre une portion (12) proximale du système et une portion (13) distale du système, le dispositif de mise en place de la section moyenne étant pourvu d'une gaine (50) extérieure, contenant un passage, ledit organe (41) de compression allongé s'étendant dans le passage de la gaine extérieure ;
ledit organe (70) de canal de guidage intérieur étant disposé au niveau de la portion distale du système, la première portion (78) d'extrémité de l'organe de canal de guidage intérieur comprenant un orifice (72) d'entrée de fil de guidage et la deuxième portion d'extrémité comprenant un orifice (73) de sortie de fil de guidage, les orifices définissant un canal de fil de guidage entre elles (71) ;
un organe (80) de canal de guidage extérieur, mobile axialement par rapport à l'organe de canal de guidage intérieur au niveau de la portion distale du système, l'organe de canal de guidage extérieur comprenant une première portion (88) d'extrémité distale présentant une ouverture (89) et une deuxième portion (87) d'extrémité proximale présentant un orifice (83) de sortie, la première ouverture d'extrémité et le deuxième orifice de sortie d'extrémité délimitant un organe de canal de guidage, et configuré pour avoir un profil gradué comportant un premier diamètre (84) extérieur entre les première et deuxième portions d'extrémité de l'organe de canal de guidage extérieur et un deuxième diamètre (85) extérieur plus petit situé au niveau ou près de la deuxième portion d'extrémité de l'organe de canal de guidage extérieur ;
une région (90) de montage d'un dispositif de déploiement autodilatable disposée au niveau de la portion distale du système dans l'organe de canal de guidage extérieur, la région de montage comprenant un dispositif de retenue (93) proximal, une plate-forme (91) d'extenseur d'organe de canal de guidage intérieur, et une surface (92) intérieure d'organe de canal de guidage extérieur ; et
une région (60) de transition disposée au niveau de la portion distale du système à l'extrémité proximale de la région de montage du dispositif de déploiement autodilatable, dans lequel l'orifice de sortie de l'organe de canal de guidage intérieur est en communication avec l'orifice de sortie de l'organe de canal de guidage extérieur, ayant une ouverture en position postérieure (65) située à l'extrémité proximale de l'orifice de sortie de l'organe de canal de guidage intérieur.

26. Système selon la revendication 25, dans lequel le corps de la pièce rapportée comporte en outre un orifice (222) d'entrée au niveau ou près de la portion (220) d'extrémité correspondante de la pièce rapportée, un orifice (242) de sortie au niveau ou près de la portion (240) d'extrémité de raccordement de la pièce rapportée, et les orifices d'entrée et de sortie du corps de la pièce rapportée définissent une lumière entre eux en communication fluidique avec l'organe (71) de guidage de l'organe de canal de guidage intérieur.

27. Système selon la revendication 25, dans lequel une jonction (190) accouple de manière fonctionnelle la portion d'extrémité correspondante de la pièce rapportée et la deuxième portion d'extrémité de l'organe de canal de guidage intérieur.
